(19) Europäisches Patentamt
European Patent Office
Office européen des brevets

(11) EP 4 342 530 A1

(12) EUROPEAN PATENT APPLICATION

(43) Date of publication:
27.03.2024 Bulletin 2024/13

(21) Application number: 22193792.3

(22) Date of filing: 02.09.2022

(51) International Patent Classification (IPC):
*A61P 9/00* (2006.01) *A61P 9/04* (2006.01)
*A61P 9/10* (2006.01) *A61P 25/00* (2006.01)
*A61P 25/16* (2006.01) *A61P 25/18* (2006.01)
*A61P 27/16* (2006.01) *A61P 35/00* (2006.01)
*C07C 229/14* (2006.01) *C07D 213/36* (2006.01)
*C07D 215/06* (2006.01) *C07D 215/227* (2006.01)
*C07D 249/08* (2006.01) *C07D 263/58* (2006.01)
*C07D 265/36* (2006.01) *C07D 307/78* (2006.01)
*C07D 307/87* (2006.01) *C07D 311/76* (2006.01)
*C07D 317/58* (2006.01) *C07D 319/18* (2006.01)

(52) Cooperative Patent Classification (CPC):
**C07D 307/78; A61P 9/00; A61P 9/04; A61P 9/10; A61P 25/00; A61P 25/16; A61P 25/18; A61P 27/16; A61P 35/00; C07C 229/14; C07C 309/04; C07D 213/36; C07D 215/06; C07D 215/227; C07D 249/08;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Insusense ApS**
**2100 København (DK)**

(72) Inventors:
- **LITTLE, Paul Brian**
  **DK-2100 Copenhagen (DK)**
- **CASES-THOMAS, Manuel Javier**
  **DK-2100 Copenhagen (DK)**
- **KJØLBY, Mads Fuglsang**
  **DK-2100 Copenhagen (DK)**
- **NYKJÆR, Anders**
  **DK-2100 Copenhagen (DK)**

(74) Representative: **Gill Jennings & Every LLP**
**The Broadgate Tower**
**20 Primrose Street**
**London EC2A 2ES (GB)**

Remarks:
The complete document including Reference Table(s) and the Sequence Listing(s) can be downloaded from the EPO website

# (54) SORTILIN MODULATORS

(57) The present invention relates to compounds of Formula (I) and (II) or a pharmaceutically acceptable salt, solvate, hydrate, tautomer, optical isomer, N-oxide, and/or prodrug thereof. The present invention also relates to pharmaceutical compositions comprising the compounds of the invention, and to their use in the treatment or prevention of medical conditions in which modulation of sortilin is beneficial.

EP 4 342 530 A1

(52) Cooperative Patent Classification (CPC): (Cont.)
**C07D 263/58; C07D 265/36; C07D 307/87;**
**C07D 311/76; C07D 317/58; C07D 319/18;**
**C07D 491/052; C07D 491/056;** C07B 2200/07;
C07C 2602/08; C07C 2602/10

## Description

### FIELD OF THE INVENTION

The invention relates to compounds of Formula (I) or (II), which have surprisingly been found to modulate the activity of sortilin. The invention also relates to pharmaceutical compositions comprising these compounds, and to their use in the treatment or prevention of medical conditions in which modulation of sortilin activity is beneficial. In particular, the compounds of the invention may cross the blood brain barrier and may therefore be particularly useful in the treatment of diseases of the central nervous system.

### BACKGROUND

Sortilin is a Type I transmembrane protein that acts as a receptor of several ligands (Petersen et al., 1997). Sortilin is abundantly expressed in neurons and microglia of the nervous system, the inner ear, and in some peripheral tissues involved in metabolic control (Tauris et al., 2020; Goettsch et al., 2017; Willnow et al., 2011; Kjolby et al., 2010). Besides acting as a receptor involved in signaling, sortilin mediates sorting of select cargo between the cell surface trans-Golgi network, and endosomal pathway (Nykjaer & Willnow, 2012; Willnow, Petersen, & Nykjaer, 2008). Sortilin harbors a large extracellular domain denoted VPS10 that defines the family of receptors named sortilins or VS10p domain receptors. The VPS10P domain in sortilin is homologous to yeast VPS10P and is made up by a 10-bladed beta-propeller structure and a cysteine-rich 10CC module (Nykjaer & Willnow, 2012; Zheng, Brady, Meng, Mao, & Hu, 2011).

Sortilin binds multiple ligands, including pro-nerve growth factor (pro-NGF), pro-BDNF, pro-neurotrophin-3, neurotensin, and ApoB (Chen et al., 2005; Kjolby et al., 2010; Mazella et al., 1998; Nykjaer et al., 2004; Quistgaard et al., 2009; Yano, Torkin, Martin, Chao, & Teng, 2009). Furthermore, Sortilin binds progranulin (PGRN), a secreted protein involved in many cellular functions including securing lysosomal processes, anti-inflammatory responses, and neurotrophic stimulation (Galimberti, Fenoglio, & Scarpini, 2018). Sortilin targets PGRN for rapid endocytosis and degradation, and it is now well established that sortilin is the most important clearance receptor for PGRN (Hu et al., 2010). Thus, sortilin negatively regulates the extracellular levels of PGRN in the periphery as well as in the brain. Indeed, lack of or blocking the receptor increases plasma PGRN levels both in mice and humans (Carrasquillo et al., 2010; Gass, Prudencio, Stetler, & Petrucelli, 2012; Hu et al., 2010; Lee et al., 2014; Miyakawa et al., 2020; Pottier et al., 2018).

Frontotemporal dementia is a highly heritable dementia and haploinsufficiency of the PGRN gene accounts for up to 25% of all cases (Gijselinck, Van Broeckhoven, & Cruts, 2008). Patients with heterozygous loss-of-function mutations in PGRN have >50% reduced extracellular levels of the protein and will invariably develop FTD, making PGRN a causal gene for the disease (Baker et al., 2006; Carecchio et al., 2011; Cruts & Van Broeckhoven, 2008; Galimberti et al., 2010). In addition, PGRN mutant alleles have been identified in Alzheimer's (AD) patients (Brouwers et al., 2008; Sheng, Su, Xu, & Chen, 2014) and high levels of extracellular PGRN are protective in models of ALS, Parkinson's disease, stroke, arthritis, and atherosclerosis (Egashira et al., 2013; Laird et al., 2010; Martens et al., 2012; Tang et al., 2011; Tao, Ji, Wang, Liu, & Zhu, 2012; Van Kampen, Baranowski, & Kay, 2014).

Sortilin is, however, not required for PGRN to elicit its functions. Hence, neurons devoid in sortilin expression are equally responsive to PGRN-induced neuronal outgrowth (De Muynck et al., 2013; Gass, Lee, et al., 2012). Further, PGRN is successfully delivered to neuronal lysosomes in sortilin-deficient cells, suggesting the existence of alternative trafficking pathways. Indeed, PGRN can bind to the lysosomal protein, prosaposin (PSAP). When PSAP binds to its cognate receptors, the cation-independent mannose-6-phosphate receptor and LRP1, it brings along PGRN to the lysosomes (Zhou et al., 2015). Finally, in a phase II clinical trial with a monoclonal anti-sortilin antibody, markers for lysosomal integrity were normal (NCT03987295).

The functional PGRN receptor remains to be identified. However, studies suggest that PGRN promotes neuronal survival, reduces inflammation and increases Aβ endocytosis by microglia (Martens et al., 2012; Pickford et al., 2011; Yin et al., 2010).

Binding of PGRN to sortilin requires the three amino acids in the C-terminal of PGRN (QLL in human, PLL in mouse), and a peptide derived from the last 24 amino acids of PGRN binds with similar affinity as the full-length protein (Zheng et al., 2011). It was proposed that this mode of binding is structurally similar to Neurotensin binding (Zheng et al., 2011), i.e. binding in the NTIS1 binding site of sortilin. There has been a successful small molecule screen that identified a blocker of Neurotensin to sortilin binding done in collaboration with Aarhus University (Andersen et al., 2014; Schroder et al., 2014).

Sortilin exists as a full-length and sorting competent receptor but is also capable of forming multimeric signalling receptor-ligand. Portions of sortilin can also be liberated from the plasma membrane to scavenge ligands (NT in pain) and control the activity of ligands. For example, sortilin is involved in synaptic plasticity by controlling the conversion rate of pro-BDNF into BDNF. This may also apply to other proneurotrophins.

Finally, the propeptide of sortilin, also named spadin, that is a ligand of the receptor has been demonstrated

to control the activity of the membrane transporter TREK-1, which is a target for major depression, among other diseases. Structurally, sortilin has an amino acid sequence according to SEQ ID NO: 1 and comprises a signal peptide, a propeptide, the Vps10p domain, a 10cc domain (10CCa + 10CCb), a transmembrane domain and a cytoplasmic tail. The luminal domain of sortilin has 6 potential *N*-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (pro-NGF), brain derived neurotrophic factor (pro-BDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models (Jansen et al., 2007; Tenk et al., 2005; Nykjaer et al., 2004).

Previous work has suggested a role for sortilin in cellular sorting and signalling associated with diseases such as diabetes and obesity (Huang et al., 2013). Sortilin facilitates translocation of GLUT4 to the plasma membrane and rescues it from degradation in the lysosomes (Pan et al., 2017). Sortilin levels have been shown to be modulated by the level of inflammation associated with these diseases. The pro-inflammatory cytokine, TNF$\alpha$, reduces both mRNA levels and protein levels of sortilin in cultured mouse and human adipocytes, as well as *in vivo* when injected into mice (Kaddai et al., 2009). Sortilin can also influence cytokine secretion: targeting sortilin in immune cells has been proposed to attenuate inflammation and reduce atherosclerosis disease progression (Mortensen et al., 2014). Additionally, US 2016/0331746 describes various scaffolds of small molecules capable of binding to the active site of sortilin. Sortilin is involved in the regulation of glucose uptake (Shi & Kandror. 2005) and the development of lipid disorder diseases (Gao et al., 2017).

Further, plasma sortilin levels have been reported to be a potential biomarker for identifying patients with either coronary heart disease or diabetes mellitus (Oh et al., 2017; Møller et al., 2021). Patients that showed increased sortilin levels within their plasma, and therefore identifiable as suffering from the above conditions, also displayed enhanced glucose levels suggesting sortilin as a therapeutic target for treating these conditions. Soluble sortilin is also proposed as a treatment for type II diabetes (WO2021116290 A1, 2021).

TAR DNA-binding protein 43 (TDP-43) has been implicated in various neurodegenerative diseases. For example, TDP-43 inclusion bodies have been found in cases of amyotrophic lateral sclerosis (ALS), frontotemporal lobar degeneration (FTLD), and Alzheimer's disease (AD) (Meneses et al., 2021).

TDP-43 regulates the splicing of several gene products, including Sortilin. In humans, the splicing involves inclusion of a cryptic exon 17b (between exon 17 and 18), which introduces a stopcodon in the stalk, potentially generating a non-membrane bound fragment (Prudencio et al., 2012). Furthermore, it has been shown that PGRN can reduce levels of insoluble TDP-43 and slow down axonal degeneration (Beel et al., 2018). Sortilin inhibition increases PGRN levels and is therefore beneficial in the treatment of neurodegenerative diseases in which TDP-43 is implicated.

Sortilin has been linked to various conditions affecting the central nervous system (CNS). Some studies suggest a role of circulating sortilin in patients with psychiatric disorders such as depression, which may relate to altered activity of neurotrophic factors (Buttenshon et al., 2015); and it has also been reported that sortilin plays a role in brain aging, Alzheimer's Disease and frontotemporal dementia (Xu et al., 2019). However, delivering therapeutic agents that are capable of crossing the blood-brain barrier to the CNS presents a major challenge.

The blood-brain barrier is a highly selective semipermeable border of endothelial cells that prevents solutes in the circulating blood from non-selectively crossing into the extracellular fluid of the CNS where neurons reside. Therapy of neurological diseases is therefore limited due to the restricted penetration of therapeutic agents across the blood-brain barrier.

Therapeutic agents for treating CNS diseases therefore must be capable of crossing the blood-brain barrier. In addition to this, they must also have a sufficient unbound drug concentration in the brain, as the free drug hypothesis states that only unbound compound is able to interact with and elicit a pharmacological effect.

To determine the unbound fraction of a test compound ($F_{ub}$), sample supernatants may be analysed by methods such as liquid chromatography with tandem mass spectrometry (LC-MS/MS). The unbound fraction may then be calculated from the peak area ratios obtained for each matrix according to the following formula:

$$F_{ub} = C_{PBS} / C_{plasma}$$

where $C_{PBS}$ and $C_{plasma}$ are the analyte concentrations in PBS (receiver) and plasma (donor), respectively.

Recovery samples may be prepared in each condition but without dialysis, and may be used for evaluation of recovery from dialysis experiments using the following formula:

$$\% \text{ Recovery} = 100\times(V_{PBS}\times C_{PBS}+V_{plasma}\times C_{plasma})/V_{plasma}\times C_{recovery}$$

where $V_{PBS}$ is the volume on the receiver side (PBS) and $V_{plasma}$ is the volume on donor side (plasma) of the dialysis device. $C_{recovery}$ is the analyte concentration measured from the recovery sample. Compounds such as propranolol or fluoxetine, may be included in experiments as controls.

The unbound fraction in brain ($F_{ub,\ brain}$) may be calculated from the measured value in brain homogenate ($F_{ub,\ meas}$), taking into account the dilution factor used in preparing the brain homogenate:

$$F_{ub,\ brain} = \frac{1/D}{\left(\frac{1}{F_{ub,\ meas}}\right) - 1 + (\frac{1}{D})}$$

where D = dilution factor.

The brain/plasma unbound partition coefficient ($K_{puu}$) may be determined as a ratio between the free compound concentrations in plasma and brain:

$$K_{puu} = \frac{C_{ub,brain}}{C_{ub,plasma}}$$

Where $C_{u,brain}$ = unbound concentration in brain ($C \times F_{ub,\ brain}$); wherein

C = concentration at steady state; and
$C_{ub,plasma}$ = unbound concentration in plasma ($C \times F_{ub}$).

Alternatively a ratio of AUCs can be used to determine $K_{puu}$. The compound of interest being dosed to a relevant species of interest at a known concentration by oral or intravenous route. Timecourses of concentration of compound in the plasma and cerebral spinal fluid (CSF) are measured. The plasma concentration is corrected to account for unbound fraction of compound. Areas under the CSF concentration curve and free fraction in plasma concentration curve are calculated by known methods and a ratio is determined to give:

$$K_{puu} = \text{AUC0-infcsf} / (\text{AUC0-infplasma} \times (\%\text{Fuplasma}/100))$$

For the treatment of CNS diseases, it is desirable for the $K_{puu}$ to have the highest possible value, above 0. A value around 1 indicating that the free fraction compound freely permeates the blood brain barrier; a value above 1 suggesting that an active influx transport mechanism at the blood brain barrier is involved; and less than 1, which indicates that the free fraction compound is either poorly permeable or is recognized by an active efflux mechanism, reducing the exposure in the CNS while passing back through the blood-brain barrier to plasma or the CSF. A $K_{puu}$ value of 0 or close to 0, indicates a poorly permeable compound or a highly active efflux mechanism that in any case will make highly improbable to reach a meaningful exposure in the CNS of the desired active species.

In view of the above, there is an unmet need for compounds that may be used in the treatment and prevention of medical conditions in which modulation of sortilin is beneficial. In particular, there is an unmet need for sortilin modulators that are capable of crossing the blood brain barrier and are therefore useful in the treatment of diseases of the central nervous system.

## DISCLOSURE OF THE INVENTION

Surprisingly, it has been found that compounds of Formula (I) and (II) modulate the activity of sortilin and are therefore useful in the treatment or prevention of conditions in which modulation of sortilin is beneficial. Furthermore, the compounds may cross the blood brain barrier and may therefore be particularly useful in the treatment of diseases of the central nervous system.

In a first aspect of the invention, there is provided a compound of Formula (I):

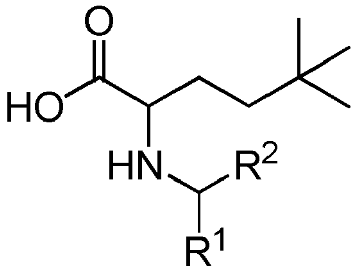

(I)

or a pharmaceutically acceptable salt, solvate, hydrate, tautomer, optical isomer, N-oxide, and/or prodrug thereof; wherein

$R^1$ is

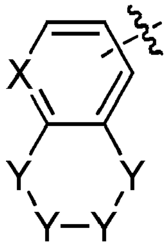 or 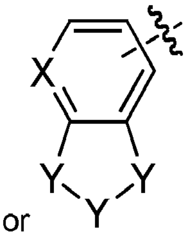 ;

X is $CR^3$ or N, wherein $R^3$ is selected from the group consisting of H, halo, and C1-C3 alkyl;

each Y is independently $CHR^4$, $NR^5$, $CR^6R^6$, or O, wherein $R^4$ is independently selected from the group consisting of H, halo, oxo, and $C_1$-$C_4$ alkyl; $R^5$ is independently selected from the group consisting of H, halo, $C_1$-$C_4$ alkyl, $C_2$-$C_4$hydroxyalkyl, -($C_2$-$C_4$ alkyl)-O-($C_1$-$C_4$ alkyl), -C(O)-($C_1$-$C_4$ alkyl), and -C(O)O-($C_1$-$C_4$ alkyl); and $R^6$ is independently selected from the group consisting of halo and $C_1$-$C_4$ alkyl;

$R^2$ is selected from the group consisting of: H, $C_1$-$C_4$ alkyl, $C_2$-$C_4$ hydroxyalkyl and C1-C3 haloalkyl;

wherein the compound is not one of the following compounds:

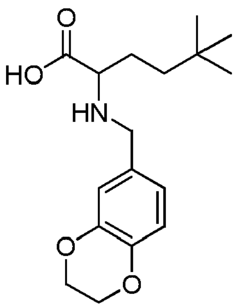 , 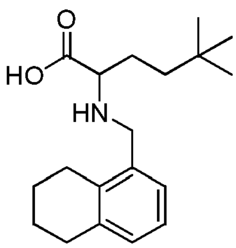 , 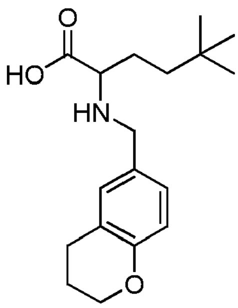 , or 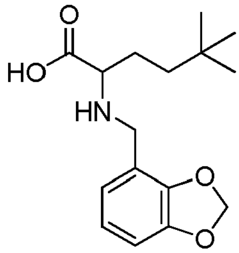 .

$R^2$ is preferably selected from the group consisting of H, $CH_3$, $CH_2F$, $CHF_2$, and $CF_3$, more preferably $R^2$ is selected from the group consisting of H, $CH_3$, $CHF_2$, and $CF_3$.

$R^3$ is preferably H or $C_1$-$C_3$ alkyl, more preferably H or methyl.

Each $R^4$ is preferably independently H or oxo.

Each $R^5$ is preferably independently H, $C_1$-$C_3$ alkyl, or -C(O)O-($C_1$-$C_4$ alkyl), more preferably H, methyl, or -C(O)O-(tert-butyl), more preferably H or methyl, most preferably H.

As defined above, $R^1$ is a partially saturated fused bicyclic ring system. The ring system may be attached to the remainder of the molecule at any suitable position on the aromatic ring. However, in a particularly preferred aspect of the invention, $R^1$ is selected from one of the following groups:

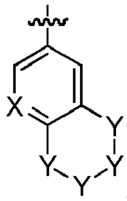, 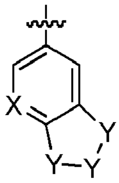, 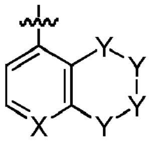, and 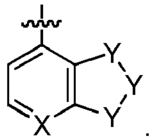.

The partially saturated ring of the $R^1$ group may comprise one or more heteroatoms. However, it is preferred that no more than two atoms in the partially saturated ring are heteroatoms. Therefore, no more than one or two Y are preferably $NR^5$ or O and the remaining Y are preferably $CHR^4$ or $CR^6R^6$.

The carbon atoms in the partially saturated ring of the $R^1$ group may be substituted with one or two substituents as defined above. However, it is preferred that any carbon atoms in the partially saturated ring are substituted with no more than 1 substituent. Therefore, if a ring atom in the partially saturated ring is carbon, it is preferably $CHR^4$.

Therefore, in a particularly preferred aspect of the invention, no more than two Y are $NR^5$ or O and the remaining Y are $CHR^4$.

Preferable examples of the $R^1$ group include:

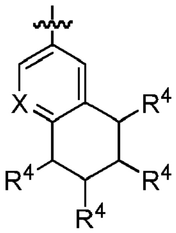, 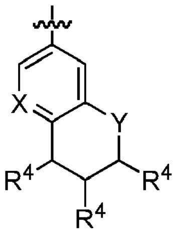, 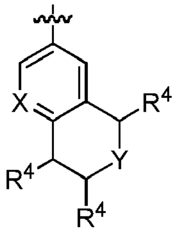, 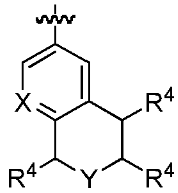, 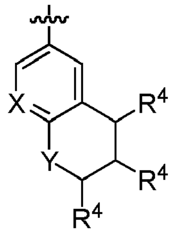, 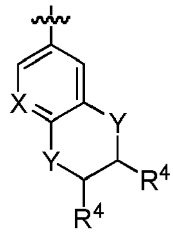,

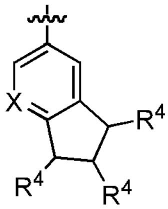, 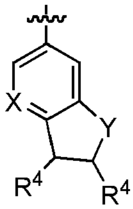, 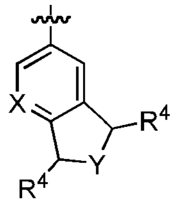, 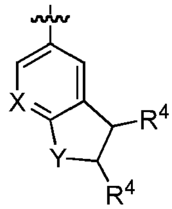, 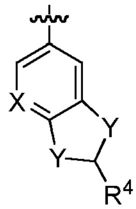, 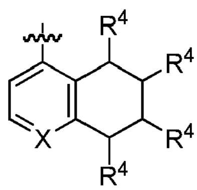,

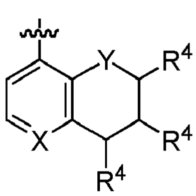, 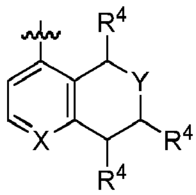, 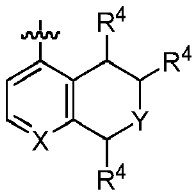, 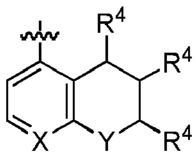, 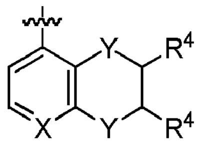,

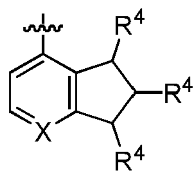, 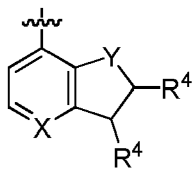, 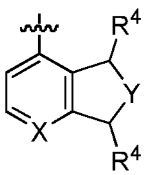, 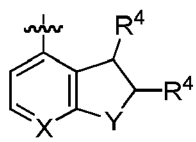, and 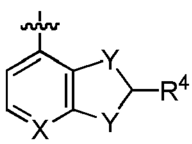.

In the preferred $R^1$ groups above, each Y is independently $NR^5$ or O. However, when the partially saturated ring comprises two Y atoms, it is preferred that one Y is O and the other Y is O or N.

It is also preferred that only one of the carbon atoms in the partially saturated ring is substituted with a substituent. Therefore, one $R^4$ group is preferably selected from the group consisting of H, halo, oxo, and $C_1$-$C_4$ alkyl and any other

$R^4$ groups are all H. More preferably, one $R^4$ group is H or oxo and any other $R^4$ groups are all H.

In the compounds of formula (I), when the $R^1$ group is attached to the remainder of the molecule at the position ortho- to the saturated ring, X may be CH and Y may be O.

In a more preferred aspect of the invention, $R^1$ is selected from one of the following groups:

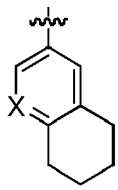, 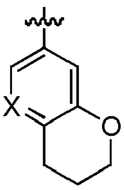, 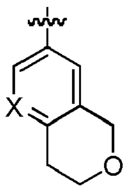, 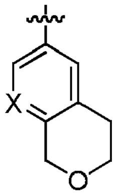, 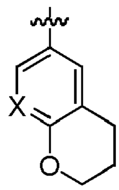, 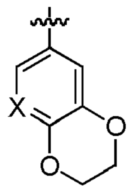, 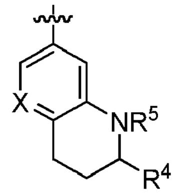,

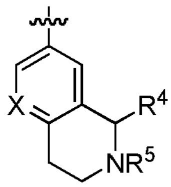, 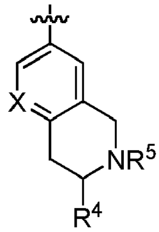, 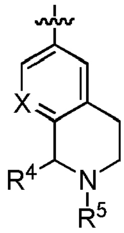, 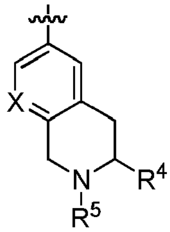, 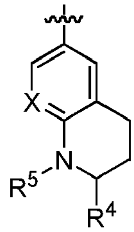, 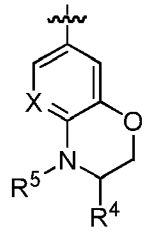,

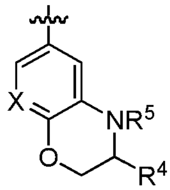, 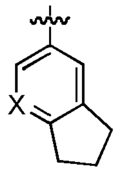, 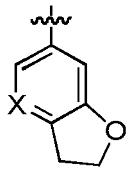, 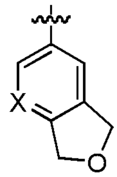, 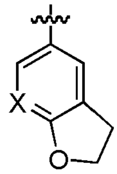, 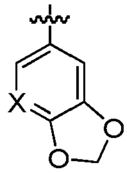, 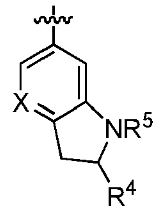,

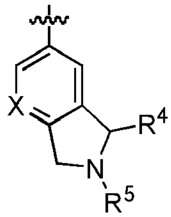, 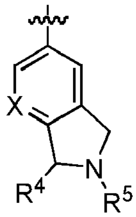, 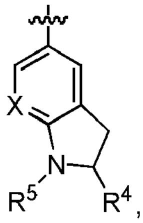, 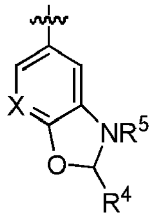, 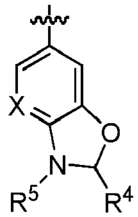, 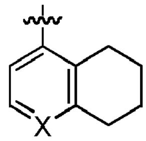, 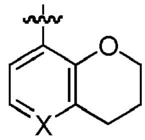,

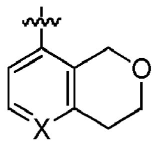, 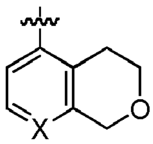, 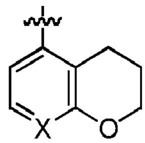, 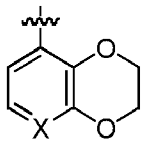, 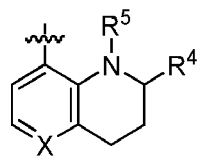, 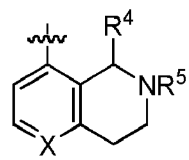,

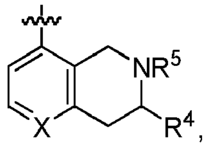, 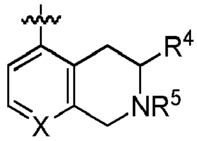, 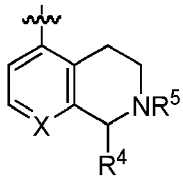, 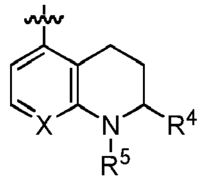, 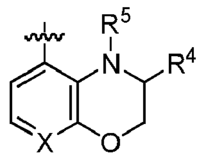,

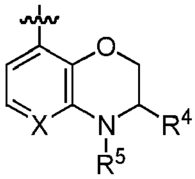, 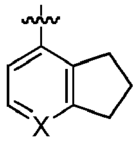, 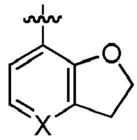, 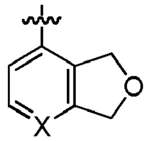, 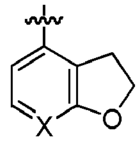, 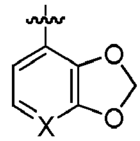,

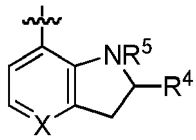, 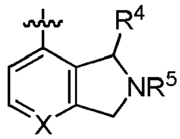, 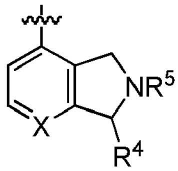, 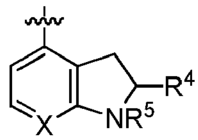, 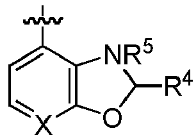,

and

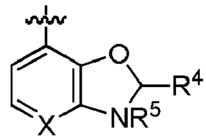.

More preferably, $R^1$ is selected from one of the following groups:

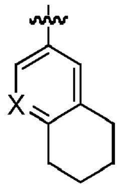, 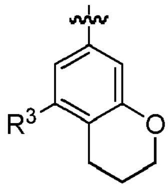, 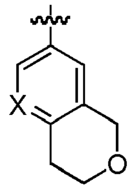, 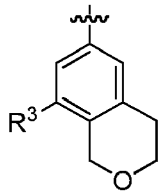, 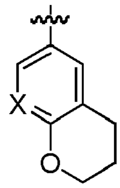, 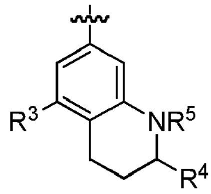,

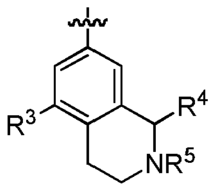, 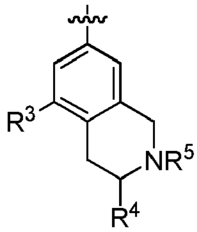, 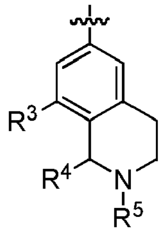, 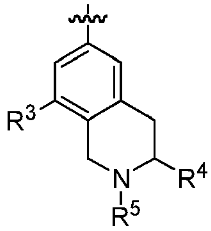, 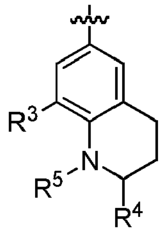, 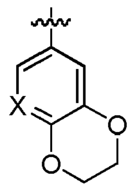

, 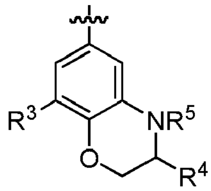, 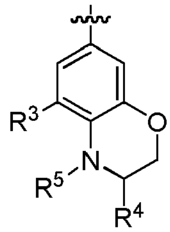, 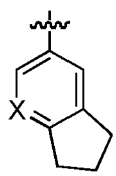, 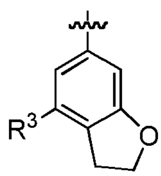, 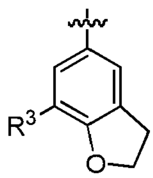, 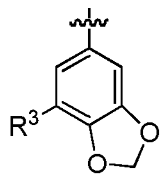,

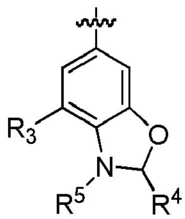, 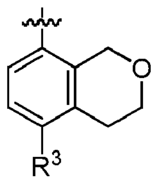, 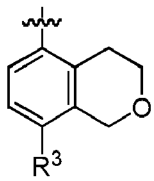, 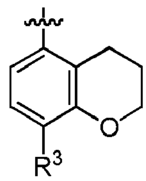, 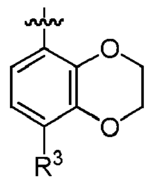, 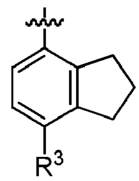,

and

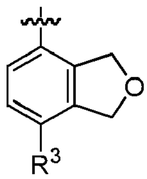 .

Most preferably, $R^1$ is selected from one of the following groups:

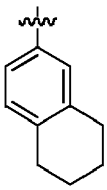, 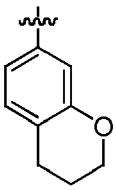, 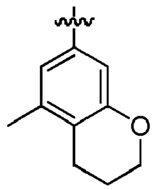, 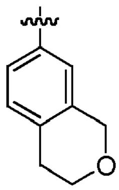, 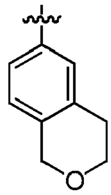, 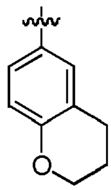, 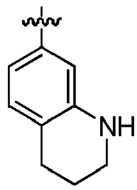, 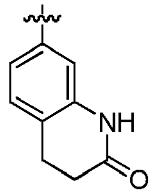,

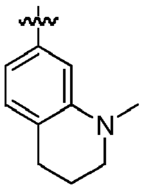, 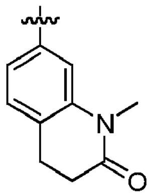, 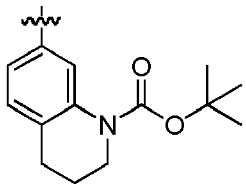, 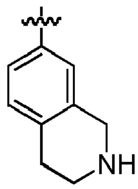, 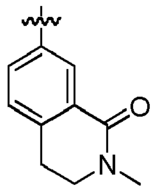, 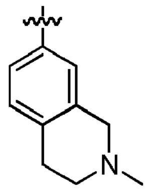,

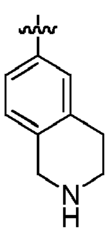, 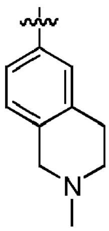, 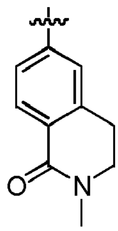, 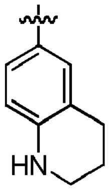, 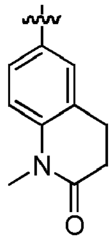, 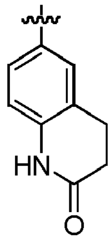, 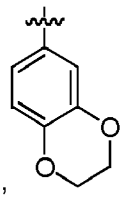, 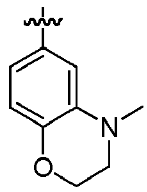,

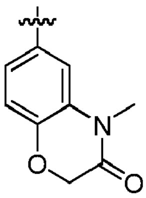, 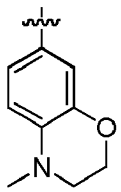, 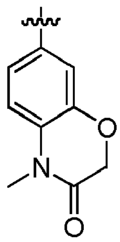, 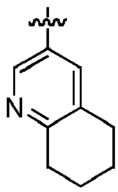, 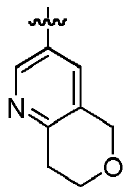, 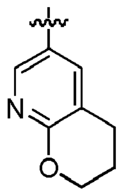, 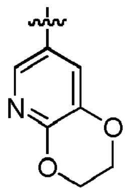, 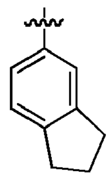,

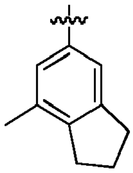, 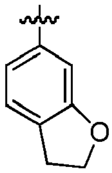, 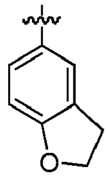, 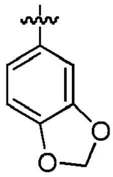, 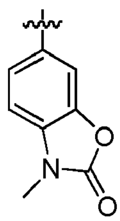, 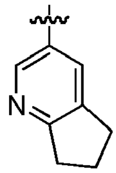, 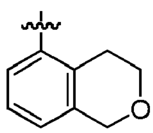, 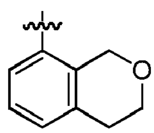,

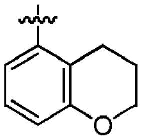, 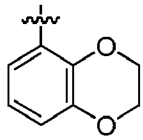, 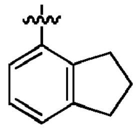, and 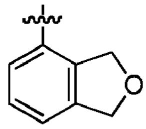.

Particular compounds of the first aspect of the invention are those listed below.

- (2S)-2-{[(1S)-2,2-difluoro-1-(5,6,7,8-tetrahydronaphthalen-2-yl)ethyl]amino}-5,5-dimethylhexanoic acid;
- (2S)-2-{[(1S)-1-(3,4-dihydro-2H-1-benzopyran-7-yl)-2,2-difluoroethyl]amino}-5,5-dimethylhexanoic acid;
- (2S)-5,5-dimethyl-2-{[(1R)-1-(5,6,7,8-tetrahydronaphthalen-2-yl)ethyl]amino}hexanoic acid;
- (2S)-5,5-dimethyl-2-{[(5,6,7,8-tetrahydronaphthalen-2-yl)methyl]amino}hexanoic acid;
- (2S)-2-[({1-[(tert-butoxy)carbonyl]-1,2,3,4-tetrahydroquinolin-7-yl}methyl)amino]-5,5-dimethylhexanoic acid;
- (2S)-2-{[(3,4-dihydro-2H-1-benzopyran-7-yl)methyl]amino}-5,5-dimethylhexanoic acid;
- (2S)-5,5-dimethyl-2-{[(1S)-2,2,2-trifluoro-1-(5,6,7,8-tetrahydroquinolin-3-yl)ethyl]amino}hexanoic acid;
- (2S)-5,5-dimethyl-2-{[(5,6,7,8-tetrahydroquinolin-3-yl)methyl]amino}hexanoic acid;
- (2S)-5,5-dimethyl-2-{[(1S)-2,2,2-trifluoro-1-(5,6,7,8-tetrahydronaphthalen-2-yl)ethyl]amino}hexanoic acid;
- (2S)-5,5-dimethyl-2-{[(1,2,3,4-tetrahydroquinolin-7-yl)methyl]amino}hexanoic acid;
- (2S)-2-{[(1R)-1-(3,4-dihydro-2H-1-benzopyran-7-yl)ethyl]amino}-5,5-dimethylhexanoic acid;
- (2S)-5,5-dimethyl-2-{[(1R)-1-(1-methyl-1,2,3,4-tetrahydroquinolin-7-yl)ethyl]amino}hexanoic acid;
- (2S)-2-{[(1S)-1-(2,3-dihydro-1H-inden-5-yl)-2,2,2-trifluoroethyl]amino}-5,5-dimethylhexanoic acid;
- (2S)-2-{[(1R)-1-(2,3-dihydro-1H-inden-5-yl)ethyl]amino}-5,5-dimethylhexanoic acid;
- (2S)-2-{[(1S)-1-(3,4-dihydro-2H-1-benzopyran-6-yl)ethyl]amino}-5,5-dimethylhexanoic acid;
- (2S)-2-[({5H,6H,7H-cyclopenta[b]pyridin-3-yl}methyl)amino]-5,5-dimethylhexanoic acid;
- (2S)-2-{[(2,3-dihydro-1H-inden-5-yl)methyl]amino}-5,5-dimethylhexanoic acid;
- (2S)-5,5-dimethyl-2-{[(4-methyl-3-oxo-3,4-dihydro-2H-1,4-benzoxazin-6-yl)methyl]amino}hexanoic acid;
- (2S)-2-{[(3,4-dihydro-1H-2-benzopyran-6-yl)methyl]amino}-5,5-dimethylhexanoic acid;
- (2S)-5,5-dimethyl-2-{[(1-methyl-2-oxo-1,2,3,4-tetrahydroquinolin-7-yl)methyl]amino}hexanoic acid;
- (2S)-5,5-dimethyl-2-[({5H,7H,8H-pyrano[4,3-b]pyridin-3-yl}methyl)amino]hexanoic acid;
- (2S)-2-{[(3,4-dihydro-1H-2-benzopyran-7-yl)methyl]amino}-5,5-dimethylhexanoic acid;
- (2S)-2-{[(1R)-1-(2,3-dihydro-1,4-benzodioxin-6-yl)ethyl]amino}-5,5-dimethylhexanoic acid;
- (2S)-2-{[(1S)-1-(2,3-dihydro-1H-inden-5-yl)ethyl]amino}-5,5-dimethylhexanoic acid;
- (2S)-2-{[(1R)-1-(3,4-dihydro-2H-1-benzopyran-6-yl)ethyl]amino}-5,5-dimethylhexanoic acid;

- (2S)-5,5-dimethyl-2-[({2H,3H,4H-pyrano[2,3-b]pyridin-6-yl}methyl)amino]hexanoic acid;
- (2S)-2-{[(1R)-1-(2H-1,3-benzodioxol-5-yl)ethyl]amino}-5,5-dimethylhexanoic acid;
- (2S)-5,5-dimethyl-2-{[(5-methyl-3,4-dihydro-2H-1-benzopyran-7-yl)methyl]amino}hexanoic acid;
- (2S)-2-{[(1R)-1-(3,4-dihydro-2H-1-benzopyran-7-yl)-2,2-difluoroethyl]amino}-5,5-dimethylhexanoic acid;
- (2S)-5,5-dimethyl-2-{[(2-oxo-1,2,3,4-tetrahydroquinolin-7-yl)methyl]amino}hexanoic acid;
- (2S)-2-{[(1S)-1-(3,4-dihydro-2H-1-benzopyran-7-yl)ethyl]amino}-5,5-dimethylhexanoic acid;
- (2S)-5,5-dimethyl-2-{[(1S)-1-(5,6,7,8-tetrahydronaphthalen-2-yl)ethyl]amino}hexanoic acid;
- (2S)-5,5-dimethyl-2-{[(1R)-1-(1,2,3,4-tetrahydroquinolin-7-yl)ethyl]amino}hexanoic acid;
- (2S)-2-{[(1S)-1-(3,4-dihydro-1H-2-benzopyran-6-yl)ethyl]amino}-5,5-dimethylhexanoic acid;
- (2S)-2-[({2H,3H-[1,4]dioxino[2,3-b]pyridin-7-yl}methyl)amino]-5,5-dimethylhexanoic acid;
- (2S)-2-{[(1S)-1-(2,3-dihydro-1,4-benzodioxin-6-yl)ethyl]amino}-5,5-dimethylhexanoic acid;
- (2S)-5,5-dimethyl-2-{[(4-methyl-3,4-dihydro-2H-1,4-benzoxazin-6-yl)methyl]amino}hexanoic acid;
- (2S)-5,5-dimethyl-2-{[(2-methyl-1-oxo-1,2,3,4-tetrahydroisoquinolin-7-yl)methyl]amino}hexanoic acid;
- (2S)-5,5-dimethyl-2-{[(2-methyl-1,2,3,4-tetrahydroisoquinolin-7-yl)methyl]amino}hexanoic acid;
- (2S)-2-{[(2,3-dihydro-1-benzofuran-6-yl)methyl]amino}-5,5-dimethylhexanoic acid;
- (2S)-5,5-dimethyl-2-{[(7-methyl-2,3-dihydro-1H-inden-5-yl)methyl]amino}hexanoic acid;
- (2S)-2-{[(3,4-dihydro-1H-2-benzopyran-5-yl)methyl]amino}-5,5-dimethylhexanoic acid;
- (2S)-2-{[(1R)-1-(3,4-dihydro-1H-2-benzopyran-7-yl)ethyl]amino}-5,5-dimethylhexanoic acid;
- (2S)-2-{[(1,3-dihydro-2-benzofuran-4-yl)methyl]amino}-5,5-dimethylhexanoic acid;
- (2S)-2-{[(2,3-dihydro-1-benzofuran-5-yl)methyl]amino}-5,5-dimethylhexanoic acid;
- (2S)-2-{[(3,4-dihydro-1H-2-benzopyran-8-yl)methyl]amino}-5,5-dimethylhexanoic acid;
- (2S)-2-{[(2H-1,3-benzodioxol-5-yl)methyl]amino}-5,5-dimethylhexanoic acid;
- (2S)-5,5-dimethyl-2-{[(3-methyl-2-oxo-2,3-dihydro-1,3-benzoxazol-6-yl)methyl]amino}hexanoic acid;
- (2S)-2-{[(1S)-1-(3,4-dihydro-1H-2-benzopyran-7-yl)ethyl]amino}-5,5-dimethylhexanoic acid;
- (2S)-5,5-dimethyl-2-{[(1,2,3,4-tetrahydroisoquinolin-7-yl)methyl]amino}hexanoic acid;
- (2S)-5,5-dimethyl-2-{[(2-methyl-1-oxo-1,2,3,4-tetrahydroisoquinolin-6-yl)methyl]amino}hexanoic acid;
- (2S)-2-{[(1S)-1-(2H-1,3-benzodioxol-5-yl)ethyl]amino}-5,5-dimethylhexanoic acid;
- (2S)-5,5-dimethyl-2-{[(1,2,3,4-tetrahydroquinolin-6-yl)methyl]amino}hexanoic acid;
- (2S)-2-{[(1R)-2,2-difluoro-1-(5,6,7,8-tetrahydronaphthalen-2-yl)ethyl]amino}-5,5-dimethylhexanoic acid;

- (2S)-2-{[(3,4-dihydro-2H-1-benzopyran-5-yl)methyl]amino}-5,5-dimethylhexanoic acid;
- (2S)-5,5-dimethyl-2-{[(2-methyl-1,2,3,4-tetrahydroisoquinolin-6-yl)methyl]amino}hexanoic acid;
- (2S)-2-{[(1S)-1-(2,3-dihydro-1,4-benzodioxin-5-yl)ethyl]amino}-5,5-dimethylhexanoic acid;
- (2S)-5,5-dimethyl-2-{[(4-methyl-3,4-dihydro-2H-1,4-benzoxazin-7-yl)methyl]amino}hexanoic acid;
- (2S)-2-{[(2,3-dihydro-1H-inden-4-yl)methyl]amino}-5,5-dimethylhexanoic acid;
- (2S)-2-{[(1R)-1-(2,3-dihydro-1,4-benzodioxin-5-yl)ethyl]amino}-5,5-dimethylhexanoic acid;
- (2S)-5,5-dimethyl-2-{[(1-methyl-2-oxo-1,2,3,4-tetrahydroquinolin-6-yl)methyl]amino}hexanoic acid;
- (2S)-5,5-dimethyl-2-{[(2-oxo-1,2,3,4-tetrahydroquinolin-6-yl)methyl]amino}hexanoic acid;
- (2S)-5,5-dimethyl-2-{[(1,2,3,4-tetrahydroisoquinolin-6-yl)methyl]amino}hexanoic acid; and
- (2S)-5,5-dimethyl-2-{[(4-methyl-3-oxo-3,4-dihydro-2H-1,4-benzoxazin-7-yl)methyl]amino}hexanoic acid.

In a second aspect of the invention, there is provided a compound of Formula (II):

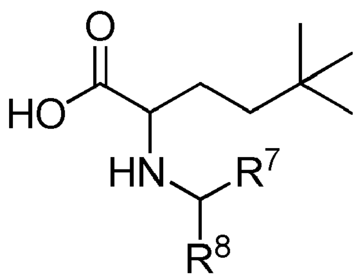

(II)

or a pharmaceutically acceptable salt, solvate, hydrate, tautomer, optical isomer, N-oxide, and/or prodrug thereof.

In one embodiment, $R^7$ is selected from the group consisting of H, $C_1$-$C_3$ alkyl, and $C_1$-$C_3$ haloalkyl, and $R^8$ is phenyl or pyridine, wherein phenyl and pyridine are independently substituted with one or more substituents selected from the group consisting of 5-membered heterocycloalkyl, triazolyl, -O-phenyl, and -$NR^9R^{10}$.

$R^9$ and $R^{10}$ are independently selected from H or $C_1$-$C_3$ alkyl, preferably H or $CH_3$, more preferably $CH_3$.

Preferably, $R^7$ is selected from the group consisting of H, $CH_3$, $CHF_2$, and $CF_3$, more preferably H and $CH_3$.

Preferably, $R^8$ is phenyl substituted with pyrrolidinyl, triazolyl, -O-phenyl, or -$NR^9R^{10}$; or $R^8$ is pyridine substituted with -O-phenyl.

More preferably, $R^8$ is selected from one of the following groups:

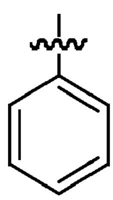
,
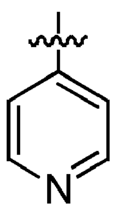
, and
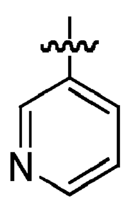
,

wherein phenyl and pyridine are substituted as defined above.

Most preferably, $R^8$ is selected from one of the following groups:

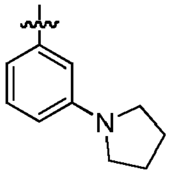, 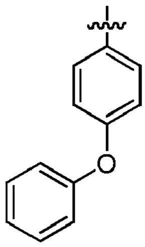, 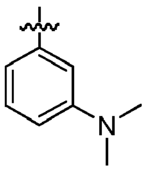, 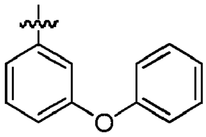, 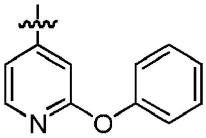, 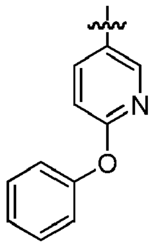,

and 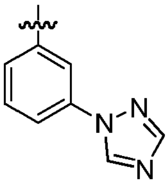.

In an alternative embodiment of the second aspect of the invention, $R^7$ is $C_1$-$C_3$ hydroxyalkyl and $R^8$ is phenyl optionally substituted with $C_1$-$C_3$ alkoxy.

Preferably, $R^7$ is $-(C_2H_4)-OH$.

Preferably, $R^8$ is phenyl optionally substituted with $-O-CH_3$.

More preferably, $R^8$ is

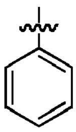 or 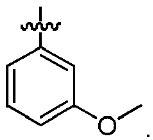.

Particular compounds of the second aspect of the invention are those listed below.

- (2S)-5,5-dimethyl-2-{[(2-phenoxypyridin-4-yl)methyl]amino}hexanoic acid;
- (2S)-5,5-dimethyl-2-{[(6-phenoxypyridin-3-yl)methyl]amino}hexanoic acid;
- (2S)-5,5-dimethyl-2-({[3-(pyrrolidin-1-yl)phenyl]methyl}amino)hexanoic acid;
- 2-{[(1S)-3-hydroxy-1-phenylpropyl]amino}-5,5-dimethylhexanoic acid;
- (2S)-5,5-dimethyl-2-{[(1R)-1-(6-phenoxypyridin-3-yl)ethyl]amino}hexanoic acid;
- (2S)-5,5-dimethyl-2-{[(1S)-1-(6-phenoxypyridin-3-yl)ethyl]amino}hexanoic acid;
- (2S)-5,5-dimethyl-2-{[(4-phenoxyphenyl)methyl]amino}hexanoic acid;
- (2S)-2-({[3-(dimethylamino)phenyl]methyl}amino)-5,5-dimethylhexanoic acid;
- (2S)-2-{[(1R)-3-hydroxy-1-(3-methoxyphenyl)propyl]amino}-5,5-dimethylhexanoic acid;
- (2S)-5,5-dimethyl-2-{[(3-phenoxyphenyl)methyl]amino}hexanoic acid;
- (2S)-2-{[(1S)-3-hydroxy-1-(3-methoxyphenyl)propyl]amino}-5,5-dimethylhexanoic acid;
- (2S)-2-{[(1R)-3-hydroxy-1-phenylpropyl]amino}-5,5-dimethylhexanoic acid; and
- (2S)-5,5-dimethyl-2-({[3-(1H-1,2,4-triazol-1-yl)phenyl]methyl}amino)hexanoic acid;

According to a third aspect of the invention, there is a pharmaceutical composition comprising a compound

according to the invention and a pharmaceutically acceptable carrier, excipient, and/or diluent.

According to a fourth aspect of the invention, there is provided a compound or pharmaceutical composition according to the invention for use in therapy.

According to a fifth aspect of the invention, there is provided a compound or pharmaceutical composition according to the invention for use in the treatment or prevention of a neurodegenerative disorder, a psychiatric disorder, an inflammatory disorder, a cancer, pain, diabetes mellitus, diabetic retinopathy, glaucoma, uveitis, cardiovascular diseases, kidney disease, psoriasis, hereditary eye conditions, hearing loss or diseases characterized by misfolded tau.

Preferably, the neurodegenerative disorder is selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy.

The neurodegenerative disorder is preferably a neurodegenerative disorder characterised by misfolded TAR DNA binding protein 43 (tdp-43). In other words, the neurodegenerative disease is characterized by truncated tdp-43 and inclusion bodies. Examples of such diseases include amyotrophic lateral sclerosis, Alzheimer's disease, Frontotemporal Lobar Degeneration, and frontotemporal dementia.

Preferably, the psychiatric disorder is selected from bipolar disorder, major depression, post-traumatic stress disorder, and anxiety disorders;

Preferably, the inflammatory disorder may be selected from inflammatory diseases and neuroinflammation.

Preferably, the cancer is selected from breast cancer, lung cancer, ovarian cancer, prostate cancer, thyroid cancer, pancreatic cancer, glioblastoma and colorectal cancer.

Preferably, the cardiovascular disease is preferably selected from atherosclerosis, cardiomyopathy, heart attack, arrhythmias, heart failure, and ischemic heart disease.

Preferably, the hearing loss is selected from noise-induced hearing loss, ototoxicity induced hearing loss, age-induced hearing loss, idiopathic hearing loss, tinnitus and sudden hearing loss.

According to a sixth aspect of the invention, there is provided the use of the compound according to the invention for the manufacture of a medicament for the treatment or prevention of a neurodegenerative disorder, a psychiatric disorder, an inflammatory disorder, a cancer, pain, diabetes mellitus, diabetic retinopathy, glaucoma, uveitis, cardiovascular diseases, kidney disease, psoriasis, hereditary eye conditions, hearing loss or diseases characterized by misfolded tau.

According to a seventh aspect of the invention, there is provided a method for the treatment or prevention of a disease or condition responsive to sortilin modulation comprising administering a therapeutically effective amount of a compound or pharmaceutical composition according to the invention.

The compounds of the invention may include isotopically-labelled and/or isotopically-enriched forms of the compounds. The compounds of the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as $^{2}H$, $^{3}H$, $^{11}C$, $^{13}C$, $^{14}C$, $^{13}N$, $^{15}O$, $^{17}O$, $^{32}P$, $^{35}S$, $^{18}F$, $^{36}Cl$.

The compounds of the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereogenic centres). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis-and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

Preferably, the compounds of Formula (I) and (II) are compounds of Formula (Ia) and (IIa) respectively:

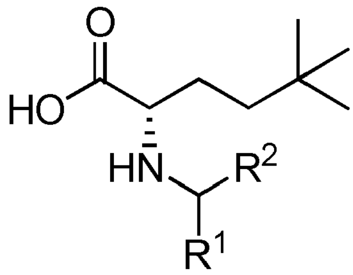

(Ia)

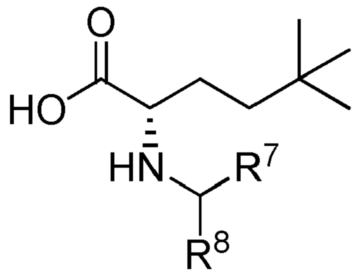

(IIa)

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or in vivo, to the parent compound of the invention. Examples of prodrugs include, but are not limited to, acetate, formate and succinate derivatives of hydroxy functional groups or phenyl carbamate derivatives of amino functional groups.

The compounds of the invention may be sortilin inhibitors, binders, modulators or antagonists. As used herein, the term "sortilin antagonist", "sortilin inhibitor", "sortilin binder" or "sortilin modulator" (used interchangeably) refers to a substance that interferes with, blocks, or otherwise attenuates the effect of, a sortilin protein binding to progranulin, or neurotensin or another extracellular ligand, or a proneurotrophin (e.g., pro-NGF, proNT3, pro-BDNF) or preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not pro-NGF) and p75NTR can simultaneously bind the NGF domain of pro-NGF. However, the resulting trimeric complex may be of lower affinity for its receptor and as a result have significantly reduced capacity to stimulate apoptosis via the mechanism described above. Skeldal et al. (2012) demonstrated that the apoptotic function of the trimeric complex is abolished when sortilin is devoid in its intracellular domain. The term "sortilin antagonist" also includes a substance or agent that

interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal *et al* showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules. "Sortilin antagonists" may act as ligand cellular uptake inhibitors wherein the ligands may be progranulin, neurotensin, BDNF etc.

The compounds of the invention may cross the blood brain barrier and therefore may be particularly useful in the treatment or prevention of diseases of the central nervous system, including neurodegenerative disorders selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke; a psychiatric disorder selected from bipolar disorder, major depression, post-traumatic stress disorder, and anxiety disorders; hearing loss selected from noise-induced hearing loss, ototoxicity induced hearing loss, age-induced hearing loss, idiopathic hearing loss, tinnitus and sudden hearing loss; brain tumours, retinopathies, glaucoma, neuroinflammation, chronic pain and diseases characterized by misfolded tau.

The compounds of the invention may have a $K_{puu}$ of more than 0.1, such as between 0.1 and 10, between 0.1 and 5, between 0.1 and 3, between 0.1 and 2, between 0.1 and 1, between 0.1 and 0.8, between 0.1 and 0.6, between 0.1 and 0.5, between 0.1 and 0.4, between 0.1 and 0.3, or between 0.1 and 0.2.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabelling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by

mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow-release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

## DEFINITIONS

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" are used interchangeably herein. It is understood that sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons (Jansen et al., 2007; Nykjaer et al., 2004; Santos et al., 2012; Skeldal et al., 2012).

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include pro-NGF, pro-BDNF, proNT3 and proNT4. Proneurotrophins may also control synaptic plasticity. Whereas mature neurotrophins induce synaptic strength, in their proforms they may weaken synapses.

"Optional" or "optionally" means that the subsequently described event or circumstance may, but need not, occur, and that the description includes instances where the event or circumstance occurs and instances in which it does not.

The term "heteroatom" means O, N, or S.

The term "$(C_1-C_n)$alkyl" denotes a straight, branched, cyclic, or partially cyclic alkyl group having from 1 to n carbon atoms, i.e. 1, 2, 3... or n carbon atoms. For the "$(C_1-C_n)$alkyl" group to comprise a cyclic portion it should be formed of at least three carbon atoms. For parts of the range "$(C_1-C_n)$alkyl" all subgroups thereof are contemplated. For example, in the range $(C_1-C_6)$alkyl, all subgroups such as $(C_1-C_5)$alkyl, $(C_1-C_4)$alkyl, $(C_1-C_3)$alkyl, $(C_1-C_2)$alkyl, $(C_1)$alkyl, $(C_2-C_6)$alkyl, $(C_2$-C5)alkyl, $(C_2-C_4)$alkyl, $(C_2-C_3)$alkyl, $(C_2)$alkyl, $(C_3-C_6)$alkyl, $(C_3-C_5)$alkyl, (C3-$C_4$)alkyl, $(C_3)$alkyl, $(C_4-C_6)$alkyl, $(C_4-C_5)$alkyl, $(C_4)$alkyl, $(C_5-C_6)$alkyl, $(C_6)$alkyl. Examples of "$C_1-C_6$ alkyl" include methyl, ethyl, n-propyl, isopropyl, cyclopropyl, n-butyl, isobutyl, sec-butyl, t-butyl, cyclobutyl, cyclopropylmethyl, branched or cyclic or partially cyclic pentyl and hexyl etc.

The term "$(C_1-C_n)$ haloalkyl" denotes a $C_1-C_n$ alkyl as described above substituted with at least one halogen atom, which is preferably, F, Cl, Br and I, more preferably F and Cl, and most preferably F.

The term "$(C_1-C_n)$ hydroxyalkyl" denotes a $C_1-C_n$ alkyl as described above substituted with at least one -OH group.

The term "$(C_1-C_n)$alkoxy" denotes -O-($(C_1-C_n)$alkyl) in which a $(C_1-C_n)$alkyl group is as defined above and is attached to the remainder of the compound through an oxygen atom.

When a term denotes a range, for instance "1 to 6 carbon atoms" in the definition of $(C_1-C_6)$alkyl, each integer is considered to be disclosed, i.e. 1, 2, 3, 4, 5 and 6.

The term "halo" means a halogen atom, and is preferably, F, Cl, Br and I, more preferably F and Cl, and most preferably F.

The term "5-membered heterocycloalkyl" denotes a non-aromatic ring having 5 ring atoms, in which at least one ring atom is a heteroatom. Preferably each heteroatom is independently selected from N, S, or O, more preferably N. Preferably, no more than 2 ring atoms are a heteroatom. More preferably, only one ring atom is a heteroatom.

"An effective amount" refers to an amount of a compound of the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

**PREPARATION OF COMPOUNDS OF THE INVENTION**

The compounds of the invention can be prepared according to the following General Synthetic Procedures scheme by methods well known and appreciated in the art. Suitable reaction conditions are well known in the art and appropriate substitutions of solvents and co-reagents are within the common general knowledge of the person skilled in the art. Likewise, it will be appreciated by those skilled in the art that synthetic intermediates may be isolated and/or purified by various well-known techniques as needed or desired, and that frequently, it will be possible to use various intermediates directly in subsequent synthetic steps with little or no purification. Furthermore, the skilled person will appreciate that in some circumstances, the orders in which moieties are introduced is not critical. The particular order of steps required to produce the compounds of formula (I) or (II) is dependent upon the particular compound being synthesized, the starting compound, and the relative liability of the substituted moieties as is well appreciated by those of ordinary skill in the art. All substituents, unless otherwise indicated, are as previously defined, and all reagents are well known and appreciated in the art.

Suitable starting materials, either optically active as single enantiomers or as racemic mixtures and protected amino acids of general formula AA-1 are either commercially available or may be prepared by a variety of methods. For example, as illustrated in the General Synthetic Procedure, **Scheme 1,** the carboxylic acid functionality of appropriately substituted amino acids of general formula AA-1, can be used as the free acid, PG = H, or protected as a suitable derivative, for example as a methyl ester. Insertion of the substituent on the primary amine present in AA-1 can be done by a variety of methods, and for the purpose of exemplification, by a reductive amination step involving a suitably substituted carbonyl compound Int-1, aldehyde or ketone and a reductive reagent, as for example but not limited to, sodium triacetoxy borohydride in a suitable solvent mixture like acetic acid and dichloromethane. An alternative method to introduce the substituent on the primary amine present in AA-1, as represented in the general Synthetic Procedures scheme, uses an alkylation step between the suitable protected AA-1 and a reagent of type Int-2. In the later, LG represents a reactive leaving group, as for example, a bromine atom, that can be selectively displaced by the free amine in AA-1, in presence of a suitable base, like, for example, potassium carbonate, in an appropriate solvent like acetonitrile. Alternatively, **Scheme 2** exemplifies complementary general synthetic strategies that can be advantageous when, for example, the intermediates of type Int-3 or Int-4 are sterically hindered. Advantageously, intermediates of type AA-2 or AA-3 can be obtained by procedures known, to those skilled in the art, from intermediates of type AA-1, purchased from commercial sources or synthesised.

## GENERAL SYNTHETIC PROCEDURES

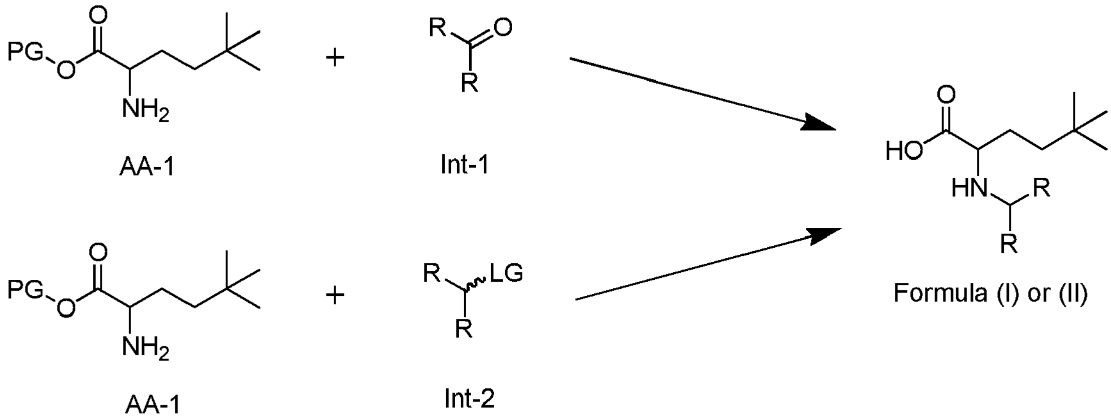

AA-1 Int-1

AA-1 Int-2

Formula (I) or (II)

**Scheme 1**

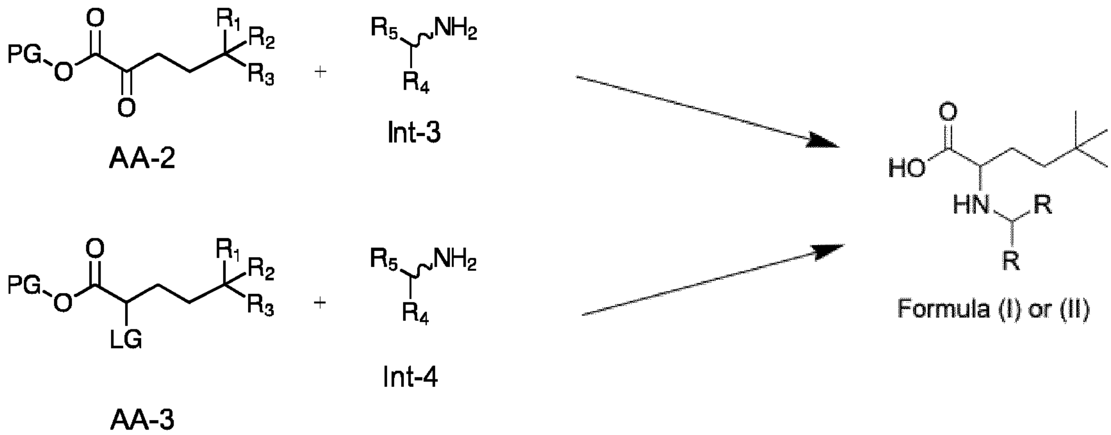

AA-2 Int-3

AA-3 Int-4

Formula (I) or (II)

**Scheme 2**

The compounds of general formula (I) or (II) may be prepared by a variety of procedures, some of which are described below. The products of each step can then be recovered by conventional methods including extraction, evaporation, precipitation, chromatography, filtration, trituration, crystallisation and the like.

Compounds of general formula (I) or (II) may contain one or more stereogenic centres. Those can be introduced from available single enantiomers, optically active, starting materials of the type AA-1. The integrity of the existing stereogenic centre can be confirmed by analytical techniques well known to those skilled in the art like for example chiral support high pressure chromatography. Alternatively, when racemic starting materials are used, it will be appreciated that if desired, single isomer products can be obtained as single enantiomers or as single diastereoisomers, by known techniques like preparative chiral support high pressure chromatography.

The skilled artisan will also appreciate that not all of the substituents in the compounds of formula (I) or (II) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006).

## EXAMPLES

### Abbreviations

approx: approximately; aq: aqueous; br: broad; *ca.*: *circa;* CDI: 1,1'-Carbonyldiimidazole; d: doublet; DCM: dichloromethane; DIC: *N,N'*-Di*i*sopropylcarbodiimide; dioxane: 1,4-dioxane; DIPEA: diisopropylethylamine; DMF: dimethylformamide; eq.: equivalent; $Et_3N$: triethylamine; EtOAc: ethyl acetate; EtOH: ethanol; Fmoc: fluorenylmethoxycarbonyl; Boc: *tert*-butoxycarbonyl; h: hours; min: minutes: HATU: 2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (ca. 20 °C); $R_T$: retention time; s: singlet, solid; SPPS: solid phase peptide synthesis. t: triplet; TBAF: tetrabutylammonium fluoride; TBME: tert-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

### General Experimental Conditions

All starting materials and solvents were obtained either from commercial sources or prepared according to the literature citation. Reaction mixtures were magnetically stirred, and reactions performed at room temperature (ca. 20 °C) unless otherwise indicated.

Column chromatography was performed on an automated flash chromatography system, such as a CombiFlash Rf system, using pre-packed silica (40 μm) cartridges, unless otherwise indicated.

$^1H$-NMR spectra were recorded at 400 MHz on a Bruker Avance AV-I-400 or on a BrukerAvance AV-II-400 instrument. Chemical shift values are expressed in ppm-values relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

### Analytical Methods

$^1H$-NMR spectra were recorded at 400 MHz on a Bruker Avance AV-I-400 instrument or on a Bruker Avance AV-II-400 instrument. Chemical shift values are expressed in ppm-values relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

**Method 1:** UPLC_AN_BASE, Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gaspressure 40 psi, drift tube temp: 50°C; column: Waters XSelect CSH C18, 50x2.1mm, 2.5μm, Temp: 25°C, Flow: 0.6 mL/min, Gradient: t0 = 5% B, t2.0min = 98% B, t2.7min = 98% B, Posttime: 0.3 min, Eluent A: 10mM ammonium bicarbonate in water (pH=9.5), Eluent B: acetonitrile.

**Method 2:** PREP_ACID-AS4A, Apparatus: Agilent Technologies G6130B Quadrupole; HPLC instrument type: Agilent Technologies 1290 preparative LC; Column: Waters XSelect CSH (C18, 100x30mm, 10μ); Flow: 55 mL/min; Column temp: RT; Eluent A: 0.1% formid acid in water; Eluent B: 100% acetonitrile lin. gradient: t=0 min 20% B, t=2 min 20% B, t=8.5 min 60% B, t=10 min 100% B, t=13 min 100% B; Detection: DAD (220-320 nm); Detection: MSD (ESI pos/neg) mass range: 100 - 1000; fraction collection based on MS and DAD.

**Method 3:** UPLC_AN_ACID, Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gaspressure 40 psi, drift tube temp: 50°C; column: Waters XSelect CSH C18, 50×2.1mm, 2.5μm, Temp: 40°C, Flow: 0.6 mL/min, Gradient: t0 = 5% B, t2.0min = 98% B, t2.7min = 98% B, Posttime: 0.3 min, Eluent A: 0.1% formic acid in water, Eluent B: 0.1% formic acid in acetonitrile.

**Method 4:** Apparatus: Agilent Technologies G6130B Quadrupole; HPLC instrument type: Agilent Technologies 1290 preparative LC; Column: Waters XSelect CSH (C18, 100x30mm, 10μ); Flow: 55 ml/min; Column temp: RT; Eluent A: 0.1% formid acid in water; Eluent B: 100% acetonitrile; Detection: DAD (220-320 nm); Detection: MSD (ESI pos/neg) mass range: 100 - 1000; fraction collection based on MS and DAD.

**Method 5:** Apparatus: ACQ-SQD2; HPLC instrument type: Waters Modular Preparative HPLC System; column: Waters XSelect (C18, 100x30mm, 10μm); flow: 55 ml/min prep pump; column temp: RT; eluent A: 10mM ammonium bicarbonate in water pH=9.5, eluent B: 100% acetonitrile; detection: DAD (220-320 nm); detection: MSD (ESI pos/neg) mass range: 100 - 800; fraction collection based on MS and DAD.

**Method 6:** Apparatus: Waters IClass; Bin. Pump: UPIBSM, SM: UPISMFTN with SO; UPCMA, PDA: UPPDATC, 210-320 nm, SQD: MS: QDA ESI, pos/neg 100-800; column: Waters XSelect CSH C18, 50×2.1mm, 2.5μm, Temp: 25°C, Flow: 0.6 mL/min, Gradient: t0 = 5% B, t1.3min = 98% B, t1.7min = 98% B, Posttime: 0.3 min, Eluent A: 10mM

ammonium bicarbonate in water (pH=9.5), Eluent B: acetonitrile. MS parameters: Source: ESI; Capillary: 2500 V; Cone: 20 V; Extractor: 3.0 V; RF: 2.5 V; Source Temp.: 150°C; Desolvation Temp.: 600°C; Cone Gas Flow: 80 L/Hr; Desolvation Gas Flow: 1000 L/Hr; Full MS scan: MS range 100-800 (positive and negative mode); scan: 0.4 sec.

**Method 7:** UPLC_AN_BASE, Instrument: Waters I-Class UPLC, Binary Solvent Manager (BSM), Sample Manager-FTN (SM-FTN) and Sample Organizer (SO), Column Manager (CM-A), PDA 210-320nm, QDa ESI 100-800 (pos) 100-800 (neg), Column: XSelect CSH C18 XP (50×2.1mm 2.5μm) Flow: 0.6 ml/min; Column temp: 25°C, Eluent A: 10mM ammonium bicarbonate in water (pH 9.5), Eluent B: acetonitrile, Gradient: t=0 min 5% B, t=2 min 98% B, t=2.7 min 98% B, Postrun: 0.3 min.

**Method 8:** MS instrument type: Agilent Technologies G6120AA Quadrupole; HPLC instrument type: Agilent Technologies 1200 preparative LC; Column: Atlantis T3 (C18, 150x19mm, 10μ); Flow: 25 ml/min; Column temp: RT; Eluent A: 0.1% formic acid in water; Eluent B: 100% acetonitrile; copy lin. Gradient from gradient method info here; Detection: DAD (220-320 nm); Detection: MSD (ESI pos/neg) mass range: 100 - 1000; Fraction collection based on MS and DAD.

**Method #acid3min** - **UPLC Acidic Method**

Apparatus: Waters HClass; Binary Solvent Pump, SM-FTN, CMA, PDA, QDa
Column: Waters ACQUITY UPLC® CSH C18, 1.7 μm, 2.1 × 30 mm at 40 °C
Detection: UV at 210-400 nm unless otherwise indicated, MS by electrospray ionisation
Solvents: A: 0.1% Formic in water, B: MeCN
Gradient:

| Time | %A | %B | Flow rate (ml/min) |
|---|---|---|---|
| 0.00 | 98 | 2 | 0.77 |
| 2.50 | 0 | 100 | 0.77 |
| 3.00 | 0 | 100 | 0.77 |

**Method #basic3min** - **UPLC Basic Method**

Apparatus: Waters HClass; Binary Solvent Pump, SM-FTN, CMA, PDA, QDa
Column: Waters ACQUITY UPLC® BEH C18, 1.7 μm, 2.1 × 30 mm at 40 °C
Detection: UV at 210-400 nm unless otherwise indicated, MS by electrospray ionisation
Solvents: A: 0.2% Ammonia in water, B: MeCN
Gradient:

| Time | %A | %B | Flow rate (ml/min) |
|---|---|---|---|
| 0.00 | 98 | 2 | 0.77 |
| 2.50 | 0 | 100 | 0.77 |
| 3.00 | 0 | 100 | 0.77 |

**Method #acid3minb**

Apparatus: Agilent 1260; Quaternery Pump, HiP Sampler, Column Compartment, DAD:, G6150 MSD
Column: Waters Cortecs C18, 30 × 2.1 mm, 2.7μm, at 40 °C
Detection: UV at 260nm +/- 90nm unless otherwise indicated, MS by electrospray ionisation
Solvents: A: 0.1% formic acid in water, B: MeCN
Gradient:

| Time | %A | %B | Flow rate (ml/min) |
|---|---|---|---|
| 0.00 | 98 | 2 | 1.35 |
| 2.50 | 0 | 100 | 1.35 |
| 3.00 | 0 | 100 | 1.35 |

**Method #basic3minb**

Apparatus: Agilent 1260; Quaternery Pump, HiP Sampler, Column Compartment, DAD:, G6150 MSD
Column: Phenomenex Evo C18, 30 × 2.1 mm, 2.6μm, at 40 °C
Detection: UV at 260nm +/- 90nm unless otherwise indicated, MS by electrospray ionisation
Solvents: A: 0.2% Ammonia in water, B: MeCN
Gradient:

| Time | %A | %B | Flow rate (ml/min) |
|---|---|---|---|
| 0.00 | 98 | 2 | 1.35 |
| 2.50 | 0 | 100 | 1.35 |
| 3.00 | 0 | 100 | 1.35 |

**Intermediates**

## Intermediate 1

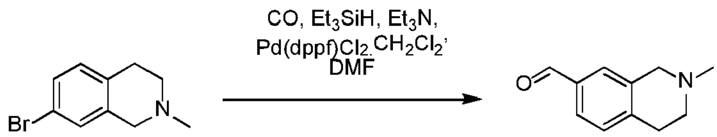


**Synthesis of 2-methyl-1,2,3,4-tetrahydroisoquinoline-7-carbaldehyde**

A solution of 7-bromo-2-methyl-1,2,3,4-tetrahydroisoquinoline (250 mg, 1 Eq, 1.11 mmol), $PdCl_2$(dppf).$CH_2Cl_2$ (80 mg, 0.089 Eq, 98 μmol), $Et_3N$ (336 mg, 0.46 mL, 3 Eq, 3.32 mmol) and triethylsilane (386 mg, 0.53 mL, 3 Eq, 3.32 mmol) in DMF (5 mL) was degassed for 5 min under a stream of $N_2$ before being sealed. This was then purged with $N_2$ (x3) before charging with CO (1.5 bar) then the reaction mixture was heated to 90 °C for 2.5 h. The reaction mixture was taken up in EtOAc (40 mL) then washed with sat. aq. $NH_4Cl$ (40 mL), water:brine (2 × 1:1, 40 mL) and brine (40 mL). The organic phase was dried over $MgSO_4$, filtered and concentrated on to silica (~1 g). The crude product was purified by chromatography on silica gel (12 g cartridge, 0-5% (1% $Et_3N$/MeOH)/DCM)) to afford 2-methyl-1,2,3,4-tetrahydroisoquinoline-7-carbaldehyde (219 mg, 1.0 mmol, 90 %, 80% Purity) as a brown oil. LCMS (Method #basic3minb, 1.25 min; M+H = 176.2. 1H NMR (500 MHz, DMSO) δ 9.93 (s, 1H), 7.66 (dd, J = 7.9, 1.7 Hz, 1H), 7.60 (d, J = 1.8 Hz, 1H), 7.34 (d, J = 7.8 Hz, 1H), 3.58 (s, 2H), 2.91 (t, J = 5.9 Hz, 2H), 2.63 (t, J = 5.9 Hz, 2H), 2.37 (s, 3H).

The following intermediates were prepared in an analogous manner to intermediate 1, starting from their corresponding aryl bromide.

| Int. | Structure | Yield | LCMS | NMR |
|---|---|---|---|---|
| 2 | 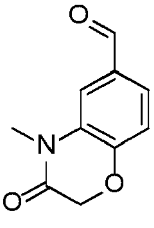 | 168 mg, (83% yield, 98% Purity) | Method #acid3minb, 1.24 min, M+H = 192.2 | 1H NMR (500 MHz, DMSO) δ 9.93 (s, 1H), 7.63 (d, J = 7.9 Hz, 2H), 7.20 (d, J = 8.0 Hz, 1H), 4.80 (s, 2H), 3.34 (s, 3H). |

(continued)

| Int. | Structure | Yield | LCMS | NMR |
|---|---|---|---|---|
| **3** | 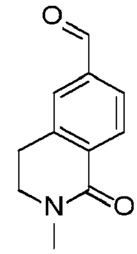 | 88 mg, (37% yield, 98% Purity) | Method #acid3minb, 1.19 min, M+H = 190.2 | 1H NMR (500 MHz, DMSO) δ 10.05 (s, 1H), 8.05 (d, J = 7.9 Hz, 1H), 7.86 (dd, J = 7.8, 1.6 Hz, 1H), 7.83 (d, J = 1.5 Hz, 1H), 3.59 (t, J = 6.7 Hz, 2H), 3.08 (t, J = 6.7 Hz, 2H), 3.05 (s, 3H). |
| **4** | 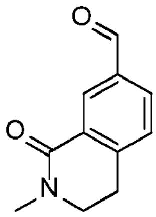 | 69 mg, (29% yield, 98% Purity) | Method #acid3minb, 1.18 min, M+H = 190.2 | 1H NMR (500 MHz, DMSO) δ 10.04 (s, 1H), 8.38 (d, J = 1.8 Hz, 1H), 7.97 (dd, J = 7.8, 1.8 Hz, 1H), 7.53 (d, J = 7.8 Hz, 1H), 3.59 (t, J = 6.7 Hz, 2H), 3.09 (t, J = 6.7 Hz, 2H), 3.05 (s, 3H). |
| **5** | 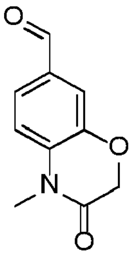 | 127 mg, (63% yield, 98% Purity) | Method #acid3minb, 1.23 min, M+H = 192.2 | 1H NMR (500 MHz, DMSO) δ 9.89 (s, 1H), 7.66 (dd, J = 8.3, 1.8 Hz, 1H), 7.46 (d, J = 1.8 Hz, 1H), 7.37 (d, J = 8.3 Hz, 1H), 4.75 (s, 2H), 3.32 (s, 3H). |
| **6** | 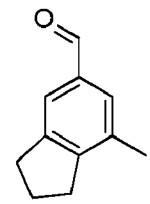 | 33 mg, (19% yield, 98% Purity) | Method #acid3minb, 1.95 min, M+H = 161.2 | 1H NMR (400 MHz, DMSO) δ 9.91 (s, 1H), 7.57 (s, 1H), 7.51 (s, 1H), 2.94 (t, J = 7.6 Hz, 2H), 2.86 (t, J = 7.5 Hz, 2H), 2.30 (s, 3H), 2.06 (app. p, J = 7.6 Hz, 2H). |
| **7** | 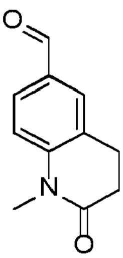 | 111 mg, (69% yield, 98% Purity) | Method #acid3minb, 1.27 min, M+H = 190.2 | 1H NMR (500 MHz, DMSO) δ 9.90 (s, 1H), 7.83 (dd, J = 8.3, 2.0 Hz, 1H), 7.77 - 7.73 (m, 1H), 7.29 (d, J = 8.4 Hz, 1H), 3.30 (s, 3H), 3.00 - 2.93 (m, 2H), 2.64 - 2.57 (m, 2H). |
| **8** | 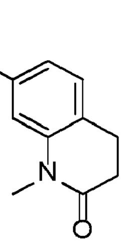 | 73 mg, (43% yield, 98% Purity) | Method #acid3minb, 1.25 min, M+H = 190.2 | 1H NMR (500 MHz, DMSO) δ 9.99 (s, 1H), 7.59 (dd, J = 7.5, 1.5 Hz, 1H), 7.56 (d, J = 1.5 Hz, 1H), 7.47 (d, J = 7.5 Hz, 1H), 3.31 (s, 3H), 2.97 (dd, J = 8.5, 6.3 Hz, 2H), 2.62 - 2.55 (m, 2H). |

(continued)

| Int. | Structure | Yield | LCMS | NMR |
|---|---|---|---|---|
| **9** | 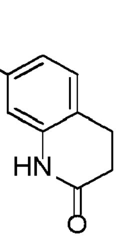 | 36 mg, (23% yield, 98% Purity) | Method #acid3minb, 1.08 min, M+H = 176.0 | 1H NMR (500 MHz, DMSO) δ 10.34 (s, 1H), 9.90 (s, 1H), 7.49 (dd, J = 7.6, 1.6 Hz, 1H), 7.42 (d, J = 7.6 Hz, 1H), 7.33 (d, J = 1.6 Hz, 1H), 2.97 (t, J = 7.6 Hz, 2H). |
| **10** | 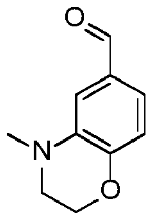 | 123 mg, (78% yield, 98% Purity) | Method #acid3minb, 1.49 min, M+H = 178.2 | 1H NMR (500 MHz, DMSO) δ 9.77 (s, 1H), 7.19 (dd, J = 8.0, 1.9 Hz, 1H), 7.14 (d, J = 1.9 Hz, 1H), 6.86 (d, J = 8.1 Hz, 1H), 4.35 - 4.30 (m, 2H), 3.31 - 3.26 (m, 2H), 2.89 (s, 3H). |
| **11** | 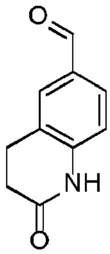 | 63 mg, (42% yield, 98% Purity) | Method #acid3minb, 1.03 min, M+H = 176.2 | 1H NMR (500 MHz, DMSO) δ 10.52 (s, 1H), 9.83 (s, 1H), 7.74 - 7.68 (m, 2H), 7.01 (d, J = 8.0 Hz, 1H), 2.97 (t, J = 7.6 Hz, 2H), 2.55 - 2.51 (m, 2H). |
| **12** | 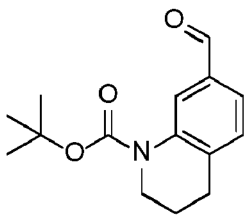 | 280 mg, (66% yield, 98% Purity) | Method #acid3minb, 1.03 min, M-tBu+2H = 206.2 | 1H NMR (500 MHz, DMSO) δ 9.93 (s, 1H), 8.17 (d, J = 1.5 Hz, 1H), 7.51 (dd, J = 7.7, 1.6 Hz, 1H), 7.34 (d, J = 7.8 Hz, 1H), 3.70 - 3.64 (m, 2H), 2.81 (t, J = 6.5 Hz, 2H), 1.85 (app. p, J = 6.4 Hz, |
| | | | | 2H), 1.48 (s, 9H). |
| **13** | 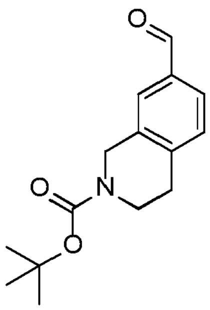 | 13 mg, (4.3% yield, 98% Purity) | Method #acid3minb, 1.91 min, M-tBu+2H = 206.2 | 1H NMR (500 MHz, MeOD) δ 9.95 (s, 1H), 7.77 - 7.70 (m, 2H), 7.39 (d, J = 7.8 Hz, 1H), 4.69 - 4.66 (m, 2H), 3.73 - 3.67 (m, 2H), 2.98 - 2.92 (m, 2H), 1.53 (s, 9H). |
| **14** | 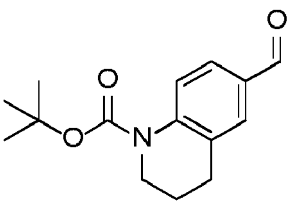 | 288 mg, (99% yield, 90% Purity) | Method #acid3minb, 2.03 min, M+H = 262.2 | 1H NMR (500 MHz, DMSO) δ 9.87 (s, 1H), 7.87 - 7.82 (m, 1H), 7.67 - 7.63 (m, 2H), 3.71 - 3.65 (m, 2H), 2.80 (t, J = 6.4 Hz, 2H), 1.90 - 1.81 (m, 2H), 1.48 (s, 9H). |

(continued)

| Int. | Structure | Yield | LCMS | NMR |
|---|---|---|---|---|
| **15** | 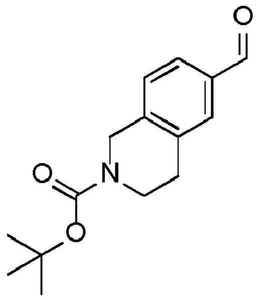 | 181 mg, (51% yield, 90% Purity) | Method #acid3minb, 1.94 min, M-tBu+2H = 206.2 | 1H NMR (500 MHz, DMSO) δ 9.96 (s, 1H), 7.74 - 7.68 (m, 2H), 7.41 (d, J = 8.3 Hz, 1H), 4.59 (s, 2H), 3.58 (t, J = 5.9 Hz, 2H), 2.87 (t, J = 5.9 Hz, 2H), 1.43 (s, 9H). |

## EXAMPLES

### Example 1

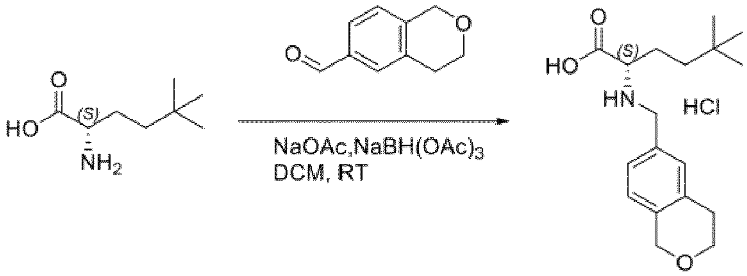


***Synthesis of (S)-2-((isochroman-6-ylmethyl)amino)-5,5-dimethylhexanoic acid hydrochloride***

A suspension of isochromane-6-carbaldehyde (102 mg, 0.628 mmol; 1.0 equivalent), (S)-2-amino-5,5-dimethylhexanoic acid (100 mg, 0.63 mmol) and sodium acetate (77 mg, 0.942 mmol; 1.5 equiv.) in dichloromethane (2 mL) was stirred at room temperature for 2 hours before addition of sodium triacetoxyborohydride (266 mg, 1.26 mmol, 2 equiv.). The remaining suspension was stirred at room temperature for 16 hours. The reaction mixture was concentrated *in vacuo* and purified by acidic preparative HPLC (method 2). The product containing fractions were concentrated, reformatted using 1M aqueous HCl and concentrated to afford (S)-2-((isochroman-6-ylmethyl)amino)-5,5-dimethylhexanoic acid hydrochloride (123.7 mg, 0.362 mmol, 57.6 % yield) as a white solid.

LCMS (Method 1, 0.906 min; M+H = 306.5; calcd. 306.2). $^1$H-NMR (400 MHz, DMSO) $\delta$ 14.02 (br, 1H), 9.37 (s, 2H), 7.31 - 7.27 (m, 2H), 7.10 (d, *J* = 7.9 Hz, 1H), 4.69 (s, 2H), 4.10 (s, 2H), 3.98 - 3.78 (m, 3H), 2.79 (t, *J* = 5.7 Hz, 2H), 1.99 -1.70 (m, 2H), 1.33 (td, *J* = 13.1, 4.8 Hz, 1H), 1.11 (td, *J* = 12.9, 4.4 Hz, 1H), 0.86 (s, 9H).

The following example was prepared in an analogous manner to example 1, starting from their corresponding aldehyde.

| Example | Structure | Yield | LCMS | NMR |
|---|---|---|---|---|
| 2 | 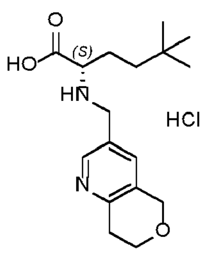 | 59.4 mg (55% yield, 94.49% purity) | Method 1, 0.777 min; M+H-HCl= 307.6; calcd. 307.2 | $^1$H-NMR (400 MHz, DMSO) δ 10.27 (s, 1H), 9.68 (s, 1H), 8.83 (d, J = 2.0 Hz, 1H), 8.25 (s, 1H), 4.82 (s, 2H), 4.31 (q, J = 13.2 Hz, 2H), 4.03 (t, J = 5.7 Hz, 3H), 3.13 (d, J = 11.4 Hz, 2H), 2.03 - 1.84 (m, 2H), 1.41 - 1.30 (m, 1H), 1.18 - 1.06 (m, 1H), 0.87 (s, 9H). |

**Example 3**

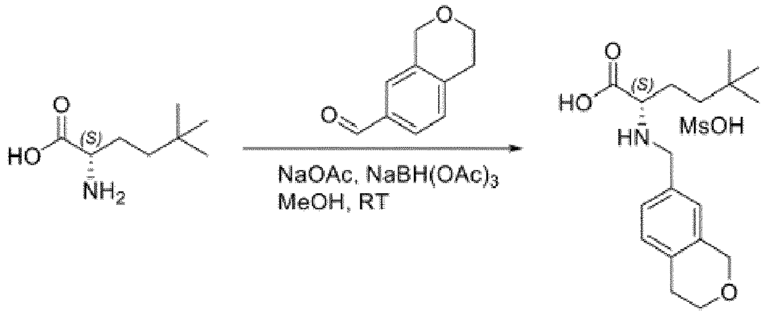


***Synthesis of (S)-2-((isochroman-7-ylmethyl)amino)-5,5-dimethylhexanoic acid mesylate salt***

Isochromane-7-carbaldehyde isochromane-7-carbaldehyde (2.26 g, 13.93 mmol; 1.0 equivalent) was added to a mixture of (S)-2-amino-5,5-dimethylhexanoic acid (2.219 g, 13.93 mmol) and sodium acetate sodium acetate (1.715 g, 20.90 mmol; 1.5 equiv.) in Methanol (20 mL). The mixture was stirred at room temperature for 2 hours before addition of sodium triacetoxyborohydride (6.61 g, 31.2 mmol; 2.0 equivalents). The mixture was stirred at room temperature for 16 hours. The product precipitated and was collected by filtration. The residue was washed with Methanol (5 mL) to afford (S)-2-((isochroman-7-ylmethyl)amino)-5,5-dimethylhexanoic acid (1.42 g, 4.65 mmol, 33 % yield). The mother liquor was concentrated in vacuo. The residue was taken up in EtOAc and washed with water (2x) and brine. The product crashed out of the organic layer and was collected by filtration. The residue was washed with a small amount of EtOAc and dried in vacuo to afford (S)-2-((isochroman-7-ylmethyl)amino)-5,5-dimethylhexanoic acid (1.04 g, 3.41 mmol, 24 % yield). (S)-2-((isochroman-7-ylmethyl)amino)-5,5-dimethylhexanoic acid (1.37 g, 4.48 mmol) was taken up in methanesulfonic acid (0.1M in MeCN) (44.8 ml, 4.48 mmol; 1 equiv.). Water 50 mL was added and the mixture was stirred at 40°C until the product was completely solubilized. The solution was lyophilized to afford (S)-2-((isochroman-7-ylmethyl)amino)-5,5-dimethylhexanoic acid compound 7 with methanesulfonic acid (1.638 g, 4.08 mmol, 29 % yield).

LCMS (Method 1, 0.902 min; M+H = 306.1; calcd. 306.2). 1H-NMR (400 MHz, DMSO) $\delta$ 10.03 (br, 2h), 7.25 (d, J = 7.8 Hz, 1H), 7.19 (d, J = 7.8 Hz, 1H), 7.13 (s, 1H), 4.68 (s, 2H), 4.11 - 3.99 (m, 2H), 3.88 (t, J = 5.7 Hz, 2H), 3.75 - 3.67 (m, 1H), 2.79 (t, J = 5.8 Hz, 2H), 2.30 (s, 3H), 1.86 - 1.67 (m, 2H), 1.30 (td, J = 12.9, 4.9 Hz, 1H), 1.13 (td, J = 12.7, 4.7 Hz, 1H), 0.85 (s, 9H).

The following examples were prepared in an analogous manner to Example 3, starting from their corresponding aldehyde.

| Example | Structure | Yield | LCMS | NMR |
|---|---|---|---|---|
| 4 | 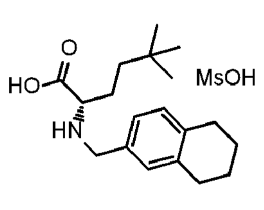  | 1.18 g (19%, 99.75% purity) | Method 1, 1.253 min; M+H = 318.6; calcd. 318.2) | $^1$H-NMR (400 MHz, DMSO) $\delta$ 14.01 (br, 1H), 9.28 - 9.00 (m, 2H), 7.21 - 7.15 (m, 2H), 7.15 - 7.06 (m, 1H), 4.08 (s, 2H), 3.89 (s, 1H), 2.72 (d, J = 5.8 Hz, 4H), 2.30 (s, 3H), 1.86 (s, 2H), 1.74 (p, *J* = 3.2 Hz, 4H), 1.32 (td, J = 13.1, 4.8 Hz, 1H), 1.12 (dt, *J* = 13.1, 6.5 Hz, 1H), 0.86 (s, 10H). |
| 5 | 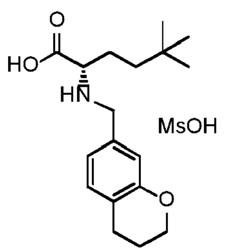  | 3.94 g (52% yield, 97.22% purity) | Method 1, 1.015 min; M+H = 306.6; calcd. 306.2) | $^1$H-NMR (400 MHz, DMSO) $\delta$ 7.08 (d, J = 7.7 Hz, 1H), 6.87 (d, J = 7.6 Hz, 1H), 6.85 (s, 1H), 4.13 (t, J = 5.1 Hz, 2H), 3.97 (q, J = 13.2 Hz, 2H), 3.58 (s, 1H), 2.73 (t, J = 6.5 Hz, 2H), 2.30 (s, 3H), 1.97 - 1.87 (m, 2H), 1.74 (d, *J* = 17.3 Hz, 2H), 1.28 (dt, J = 13.5, 7.2 Hz, 1H), 1.22 - 1.06 (m, 1H), 0.84 (s, 9H). |

(continued)

| Example | Structure | Yield | LCMS | NMR |
|---|---|---|---|---|
| 6 | 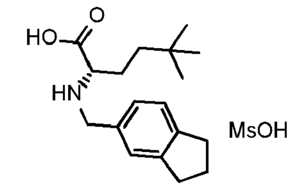 | 3.67 g (50%, 94.65 % purity) | Method 1, 1.118 min; M+H = 290.6; | $^1$H-NMR (400 MHz, DMSO) δ 10.05 (br, 2H), 7.33 (s, 1H), 7.27 (d, J = 7.6 Hz, 1H), 7.21 (d, J = 7.7 Hz, 1H), 4.13 - 4.01 (m, 2H), 3.74 (t, J = |
| | | | calcd. 290.2) | 5.9 Hz, 1H), 2.86 (t, J = 7.4 Hz, 4H), 2.31 (s, 3H), 2.03 (p, *J* = 7.5 Hz, 2H), 1.87 - 1.68 (m, 2H), 1.31 (td, J = 13.0, 4.9 Hz, 1H), 1.13 (td, J = 12.8, 4.6 Hz, 1H), 0.85 (s, 9H). |

**Example 7**

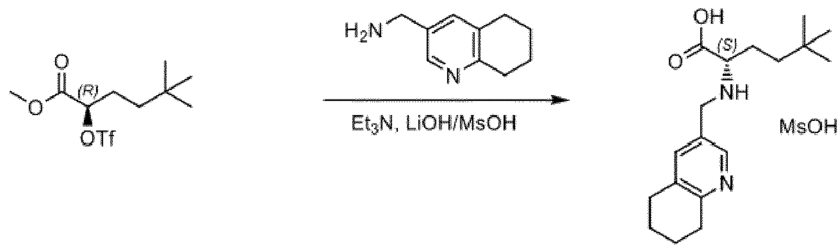


***Synthesis of (S)-5,5-dimethyl-2-(((5,6,7,8-tetrahydroquinolin-3-yl)methyl)amino)hexanoic acid-methanesulfonic acid***

To a solution of (5,6,7,8-tetrahydroquinolin-3-yl)methanamine dihydrochloride (77 mg, 0.326 mmol, 1.0 equiv.) in dichloromethane (0.9 mL) with triethylamine (148 μl, 1.061 mmol, 3.25 equiv.) was added a solution of methyl (R)-5,5-dimethyl-2-(((trifluoromethyl)sulfonyl)oxy)hexanoate (100 mg, 0.326 mmol, 1.0 equiv.) in DCM (0.9 mL). The reaction mixture was stirred at room temperature for 3 hours. The solvents were evaporated via nitrogen blow-drying. The crude was redissolved in acetonitrile: water (2 mL, 1:1). Lithium hydroxide (68.5 mg, 1.632 mmol, 5.0 equiv.) was added and the mixture was stirred for 16 hours. The mixture was purified by acidic preparative T3 HPLC (Method 8) to afford (S)-5,5-dimethyl-2-(((R)-1-(5,6,7,8-tetrahydronaphtalen-2-yl)ehtyl)amino)hexanoic acid (12.4 mg, 0.039 mmol, 17 % yield). The product was dissolved in acetonitrile (1.1 ml) and methanesulfonic acid (1.0 equiv.) was added. The mixture was lyophilized to afford (S)-5,5-dimethyl-2-(((5,6,7,8-tetrahydroquinolin-3-yl)methyl)amino)hexanoic acid compound with methanesulfonic acid (13.1 mg, 0.033 mmol, 10.02% yield). LCMS (Method 7, 0.900 min; M+H-MsO$^-$ = 305.2; calcd. 305.2). $^1$H-NMR (400 MHz, DMSO) δ 8.35 (s, 1H), 7.58 (s, 1H), 4.10 (s, 2H), 2.86 - 2.72 (m, 4H), 2.29 (s, 3H), 1.88 - 1.71 (m, 6H), 1.37 - 1.24 (m, 1H), 1.18 - 1.09 (m, 1H), 0.86 (s, 9H).

The following example can be prepared in an analogous manner to example 7, starting from the corresponding amine.

| Example | Structure | Yield | LCMS | NMR |
|---|---|---|---|---|
| 8 | 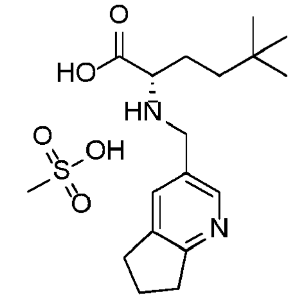 | 32 mg, (13% yield, 95% purity) | Method #basic3min, 0.77 min, M+H = 291.4 | $^1$H NMR (500 MHz, DMSO) δ 14.12 (br.s, 1H), 9.38 - 8.98 (m, 2H), 8.37 (s, 1H), 7.73 (s, 1H), 4.17 (s, 2H), 4.04 - 3.93 (m, 1H), 2.93 (app.t, J = 7.6 Hz, 4H), 2.29 (s, 3H), 2.09 (p, J = 7.6 Hz, 2H), 1.94 - 1.71 (m, 2H), 1.32 (app.td, J = 13.1, 4.7 Hz, 1H), 1.15 - 1.07 (m, 1H), 0.86 (s, 9H). |
| 9 | 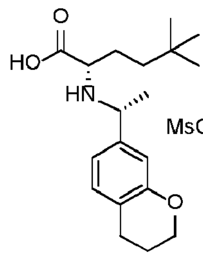 MsOH | 33.3 mg (24% yield, 94.53% purity) | LCMS (Method 7, 1.059 min; M+H-MsO-= 320.2; calcd. 320.2) | $^1$H-NMR (400 MHz, DMSO) δ 9.17 (s, 1H), 7.14 - 7.08 (m, 1H), 6.99 - 6.89 (m, 2H), 4.34 (d, *J* = 6.7 Hz, 1H), 4.18 - 4.10 (m, 2H), 3.60 - 3.51 (m, 1H), 2.74 (t, J = 6.4 Hz, 1H), 2.33 (s, 3H), 1.96 - 1.79 (m, 3H), 1.77 - 1.64 (m, 1H), 1.54 (d, *J* = 6.6 Hz, 2H), 1.33 -1.20 (m, 1H), 1.01 (td, J = 13.0, 4.1 Hz, 1H), 0.90 - 0.79 (m, 9H). |

**Example 10**

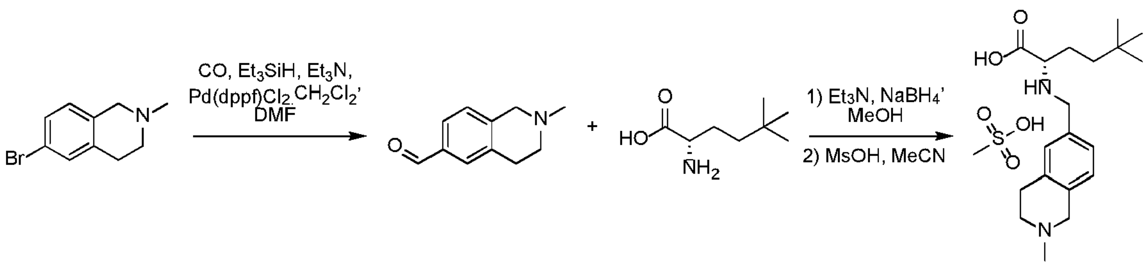


**Synthesis of (S)-5,5-dimethyl-2-(((2-methyl-1,2,3,4-tetrahydroisoquinolin-6-yl)methyl)amino)hexanoic acid, Mesylic acid**

A solution of 6-bromo-2-methyl-1,2,3,4-tetrahydroisoquinoline (129 mg, 1 Eq, 570 μmol), triethylamine (173 mg, 239 μL, 3 Eq, 1.71 mmol), triethylsilane (199 mg, 273 μL, 3 Eq, 1.71 mmol) and $PdCl_2$(dppf)-$CH_2Cl_2$ (30 mg, 0.064 Eq, 37 μmol) in DMF (3.5 mL) was degassed for 5 min under a stream of $N_2$ before being sealed. This was then purged with $N_2$ (x3) before charging with CO (1.5 bar) then the reaction mixture was heated to 90 °C for 6 hours. The reaction mixture was taken up in EtOAc (30 mL) then washed with sat. aq. $NH_4Cl$ (20 mL), water:brine (2 × 1:1, 20 mL) and brine (20 mL). The organic phase was dried over $MgSO_4$, filtered and concentrated on to silica (~1 g). The crude product was purified by chromatography on silica gel (12 g cartridge, 0-5% (1% $Et_3N$ in MeOH)/DCM) to afford 2-methyl-1,2,3,4-tetrahydroisoquinoline-6-carbaldehyde (54 mg, 0.30 mmol, 52 %, 96% Purity) as a brown gum.

A suspension of (S)-2-amino-5,5-dimethylhexanoic acid (49 mg, 1 Eq, 0.31 mmol) and 2-methyl-1,2,3,4-tetrahydroisoquinoline-6-carbaldehyde (54 mg, 1 Eq, 0.31 mmol) and triethylamine (31 mg, 43 μL, 1 Eq, 0.31 mmol) in MeOH (5.0 mL) were stirred for 2 h at 40 °C to achieve a solution. The mixture was cooled to 0 °C before sodium borohydride (12 mg, 1 Eq, 0.31 mmol) was added and stirring continued at rt for 1 hour. AcOH (0.1 mL) was added and the reaction mixture was concentrated on to celite (~1 g). The crude product was purified by chromatography on RP Flash C18 (12 g cartridge, 10-50% (0.1 % Formic acid in MeCN)/(0.1% Formic Acid in Water)). The isolated fractions were taken up in 2 M NaOH (0.5 mL) and the crude product was repurified by chromatography on RP Flash C18 (12 g cartridge, 15-50% MeCN/10 mM Ammonium Bicarbonate) to afford the freebase. The freebase was treated with MeOH (1 mL) and 0.1 M MsOH in MeCN (1 equiv) was added before concentrating to dryness to afford (S)-5,5-dimethyl-2-(((2-methyl-1,2,3,4-tetrahydroisoquinolin-6-yl)methyl)amino)hexanoic acid, Mesylic acid (19 mg, 39 μmol, 13 %, 85% Purity) as a colourless solid.

UPLC (Method #basic3min, 0.72 min; M+H = 319.3. 1H NMR (500 MHz, DMSO) δ 7.03 - 6.98 (m, 2H), 6.92 (d, J = 7.6 Hz, 1H), 3.61 - 3.55 (m, 1H), 3.44 - 3.36 (m, 3H), 3.19 - 3.13 (m, 1H), 2.77 (t, J = 6.0 Hz, 2H), 2.59 - 2.52 (m, 2H), 2.31 (s, 3H), 2.29 (s, 3H), 1.46 - 1.11 (m, 4H), 0.81 (s, 9H), 3 x exchangeable Hs not observed.

**Example 11**

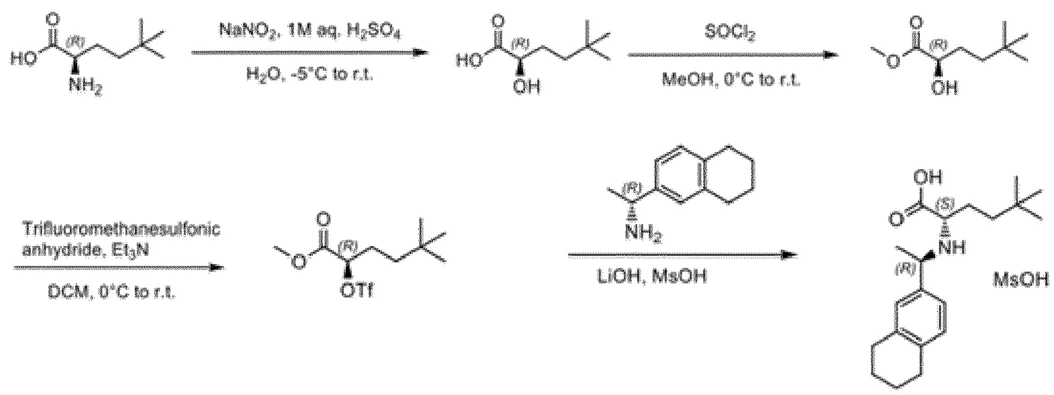


***Synthesis of (R)-2-hydroxy-5,5-dimethylhexanoic acid***

1M aq. sulfuric acid (226 mL, 226 mmol, 3.0 equiv.) was added to (R)-2-amino-5,5-dimethylhexanoic acid (12 g, 75 mmol, 1.0 equiv.) in water (220 ml). The mixture was cooled to -5°C and a solution of sodium nitrite (31.2 g, 452 mmol, 6.0 equiv.) in Water (220 ml) was added dropwise, keeping the temperature below 0°C. After addition, the mixture was allowed to warm to room temperature and stirred for 16 hours.

The mixture was extracted with $Et_2O$ (4 × 200 mL) and the combined organics were washed with brine (300 mL), dried over $Na_2SO_4$, filtered and concentrated *in vacuo* to afford (R)-2-hydroxy-5,5-dimethylhexanoic acid (8.98 g, 56.1 mmol, 74.4 % yield) as a yellow solid. 1H-NMR (400 MHz, CDCl3) δ 4.28 (dd, J = 7.2, 4.2 Hz, 1H), 1.92 - 1.80 (m, 1H), 1.75 - 1.62 (m, 1H), 1.41 - 1.27 (m, 2H), 0.90 (s, 9H).

***Synthesis of methyl (R)-2-hydroxy-5,5-dimethylhexanoate***

$SOCl_2$ (12 ml, 164 mmol, 2.93 equiv.) was added to (R)-2-hydroxy-5,5-dimethylhexanoic acid (8.98 g, 56.1 mmol, 1 equiv.) in Methanol (120 ml) at 0°C. After addition, the mixture was allowed to warm to room temperature and stirred for 16 hours. The mixture was alkalized to pH 9 by addition of sat. aq. $NaHCO_3$ and extracted with $Et_2O$ (2 × 400 mL). Combined organics were dried over $Na_2SO_4$, filtered and concentrated in vacuo to afford methyl (R)-2-hydroxy-5,5-dimethylhexanoate (10.01 g, 55.0 mmol, 98 % yield) as yellow oil. Contains 4.2% (w/w) MeOH. 1H-NMR (400 MHz, $CDCl_3$) δ 4.18 (dd, J = 7.2, 4.2 Hz, 1H), 3.80 (s, 3H), 1.84 - 1.72 (m, 1H), 1.67 - 1.52 (m, 1H), 1.37 - 1.21 (m, 2H), 0.89 (s, 9H).

***Synthesis of methyl (R)-5,5-dimethyl-2-(((trifluoromethyl)sulfonyl)oxy)hexanoate***

Trifluoromethanesulfonic anhydride (4.65 mL, 27.5 mmol, 1.10 equiv.) was added dropwise to a solution of methyl (R)-2-hydroxy-5,5-dimethylhexanoate (4.36 g, 25.02 mmol, 1.0 equiv.) and triethylamine (4.19 ml, 30.0 mmol, 1.2 equiv.) in Dichloromethane (100 mL) at 0°C. After addition, the mixture was allowed to warm to room temperature and stirred for 16 hours. Water (100 mL) was added, and the mixture was extracted with EtOAc (2 × 250 mL). The combined organics were washed with brine (250 mL), dried over $Na_2SO_4$, filtered and concentrated *in vacuo* to afford methyl (R)-5,5-dimethyl-2-(((trifluoromethyl)sulfonyl)oxy)hexanoate (7.14 g, 23.31 mmol, 49 % corrected yield) as a dark brown oil. 1H-NMR (400 MHz, CDCl3) δ 5.12 (dd, J = 6.9, 5.0 Hz, 1H), 3.85 (s, 3H), 2.05 - 1.90 (m, 2H), 1.36 - 1.24 (m, 2H), 0.90 (s, 9H).

***Synthesis of (S)-5,5-dimethyl-2-(((R)-1-(5,6,7,8-tetrahydronaphthalen-2-yl)ethyl)amino)hexanoic acid methanesulfonic acid***

Methyl (R)-5,5-dimethyl-2-(((trifluoromethyl)sulfonyl)oxy)hexanoate (70 mg, 0.229 mmol, 1.0 equivalent) in dichloromethane (1 mL) was added dropwise to a solution of (*R*)-1-(5,6,7,8-tetrahydronaphtalen-2-yl)ethan-1-amine

(40.1 mg, 0.229 mmol, 1.0 equivalent) in dichloromethane (1 mL). The dichloromethane was removed and the residue was taken up in acetonitrile : water (2 mL, 1:1). Lithium hydroxide (27.4 mg, 1.143 mmol, 5.0 equiv.) was added and the mixture was stirred for 16 hours. The mixture was submitted for acidic preparative HPLC (Method 2) to afford (*S*)-5,5-dimethyl-2-(((*R*)-1-(5,6,7,8-tetrahydronaphtalen-2-yl)ethyl)amino)hexanoic acid (12.4 mg, 0.039 mmol, 17 % yield). The product was dissolved in acetonitrile (1.1 ml) and methanesulfonic acid (0.1M in acetonitrile) (390 μl, 0.039 mmol, 0.171 equivalent) was added. The mixture was lyophilized to afford (S)-5,5-dimethyl-2-(((R)-1-(5,6,7,8-tetrahydronaphthalen-2-yl)ethyl)amino)hexanoic acid-methanesulfonic acid (20.5 mg, 0.050 mmol, 21.7% yield.

LCMS (Method 1, 1.239 min; M+H-$MsO^-$ = 318.5; calcd. 318.4). $^1$H-NMR (400 MHz, DMSO) $\delta$ 7.18 (d, J = 7.8 Hz, 2H), 7.10 (d, *J* = 7.7 Hz, 1H), 4.26 (d, J = 6.9 Hz, 1H), 3.54 - 3.40 (m, 2H), 2.71 (d, *J* = 5.7 Hz, 4H), 2.30 (s, 3H), 1.74 (h, *J* = 3.8 Hz, 4H), 1.52 (d, *J* = 6.7 Hz, 3H), 1.27 (td, J = 13.0, 4.7 Hz, 1H), 1.03 (td, J = 12.8, 3.9 Hz, 1H), 0.85 (s, 9H).

The following examples can be prepared in an analogous manner to example 11, starting from the corresponding enantiomeric secondary amine.

| Example | Structure |
|---|---|
| 12 | 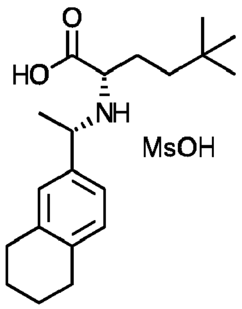 MsOH |
| 13 | 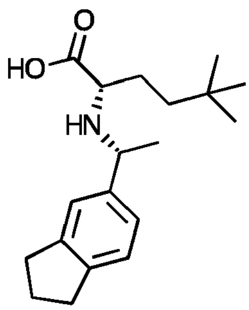 |
| 14 | 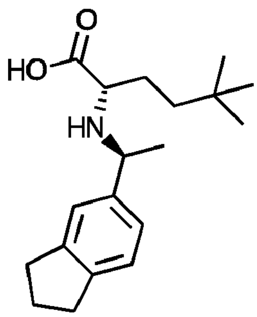 |

**Example 15**

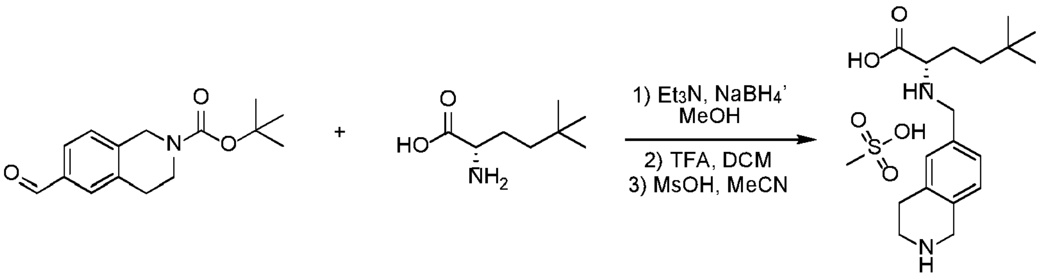


**Synthesis of (S)-2-((3,4-dimethylbenzyl)amino)-5,5-dimethylhexanoic acid, Mesylic acid**

A suspension of (S)-2-amino-5,5-dimethylhexanoic acid (61 mg, 1.0 Eq, 0.38 mmol), tert-butyl 6-formyl-3,4-dihydroisoquinoline-2(1H)-carboxylate (100 mg, 1 Eq, 383 μmol) and $Et_3N$ (40 mg, 55 μL, 1.0 Eq, 0.39 mmol) in MeOH (3 mL) was heated at 40 °C for 2 hours before being cooled with an ice bath and treated with $NaBH_4$ (15 mg, 1.0 Eq, 0.40 mmol). The mixture was allowed to warm to rt before concentrating to dryness and suspending in water (5 mL). Acetic acid (42 mg, 40 μL, 1.8 Eq, 0.70 mmol) was added before filtering. The material was suspended in water (10 mL) and acetone (2 mL) before heating at 60 °C for 30 min. After cooling to rt, the Boc protected freebase (112 mg, 0.28 mmol, 74% yield) was collected by filtration.

A sample of the freebase (61 mg, 0.15 mmol) was taken up in DCM (3 mL) and TFA (0.5 mL) was added. The mixture was stirred at rt for 1 hour before being diluted with MeOH (4 mL) and loaded on to SCX (~1 g). This was washed with MeOH and the required material was eluted with 7 M $NH_3$ in MeOH (5 column volumes) and concentrated. This was then solubilised in MeOH (2 mL) and 0.1 M MsOH in MeCN (1 equiv) was added before the material was concentrated to dryness to afford (S)-5,5-dimethyl-2-(((1,2,3,4-tetrahydroisoquinolin-6-yl)methyl)amino)hexanoic acid, Mesylic acid (67 mg, 0.16 mmol, 43 %, 98% Purity) as a colourless solid.

LCMS (Method #acid3minb, 0.13 min; M+H = 305.2. 1H NMR (500 MHz, DMSO) δ 9.01 (s, 2H), 7.32 - 7.18 (m, 2H), 4.27 (s, 2H), 3.95 - 3.89 (m, 1H), 3.87 - 3.81 (m, 1H), 3.73 - 3.65 (m, 1H), 3.42 - 3.36 (m, 2H), 3.02 - 2.96 (m, 2H), 2.80 - 2.72 (m, 1H), 2.30 (s, 3H), 1.70 - 1.59 (m, 2H), 1.29 - 1.12 (m, 2H), 0.85 (s, 9H), 1 x exchangeable Hs not observed.

The following Examples were prepared in an analogous manner to Example 15, starting from the corresponding aldehyde.

| Example | Structure | Yield | UPLC | NMR |
|---|---|---|---|---|
| 16 | 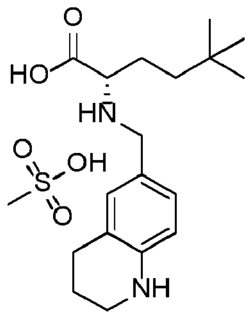 | 70 mg, (43% yield, 95% purity) | Method #acid3minb, 1.09 min, M-H = 303.2 | $^1$H NMR (500 MHz, DMSO) δ 13.91 (s, 1H), 9.08 - 8.78 (m, 2H), 6.94 - 6.89 (m, 2H), 6.43 (d, J = 8.6 Hz, 1H), 5.92 (s, 1H), 3.99 - 3.87 (m, 2H), 3.87 - 3.72 (m, 1H), 3.18 (t, J = 5.6 Hz, 2H), 2.65 (t, J = 6.4 Hz, 2H), 2.29 (s, 3H), 1.91 - 1.62 (m, 4H), 1.30 (app. td, J = 13.2, 4.7 Hz, 1H), 1.10 (app. td, J = 12.9, 4.3 Hz, 1H), 0.85 (s, 9H). |
| 17 | 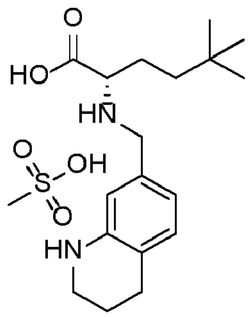 | 93 mg, (65% yield, 98% purity) | Method #acid3minb, 1.14 min, M+H = 305.2 | $^1$H NMR (500 MHz, DMSO) δ 9.02 (v. br. s, 2H), 6.86 (d, J = 7.5 Hz, 1H), 6.49 - 6.43 (m, 2H), 5.82 (br. s, 1H), 3.96 - 3.85 (m, 2H), 3.76 - 3.73 (m, 1H), 3.17 (t, J = 5.5 Hz, 2H), 2.65 (t, J = 6.2 Hz, 2H), 2.29 (s, 3H), 1.86-1.69 (m, 4H), 1.31 (app. td, J = 13.1, 4.7 Hz, 1H), 1.11 (app. td, J = 12.9, 4.5 Hz, 1H), 0.85 (s, 9H), 1 x exchangeable not observed. |

(continued)

| Example | Structure | Yield | UPLC | NMR |
|---|---|---|---|---|
| 18 | 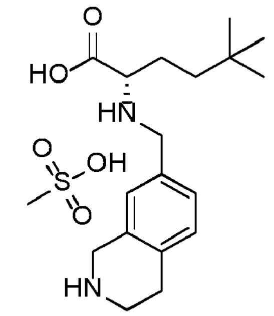 | 7.5 mg, (34% yield, 90% purity) | Method #acid3minb, 0.29 min, M+H = 305.2 | $^1$H NMR (500 MHz, DMSO) δ 9.01 (s, 2H), 7.29 (d, J = 7.9 Hz, 1H), 7.25-7.20 (m, 2H), 4.27 (s, 2H), 3.90 (d, J = 13.3 Hz, 1H), 3.80 (d, J = 13.1 Hz, 1H), 3.38 (t, J = 6.3 Hz, 2H), 3.19 - 3.14 (m, 1H), 2.98 (t, J = 6.3 Hz, 2H), 2.30 (s, 4H), 1.65-1.60 (m, 2H), 1.30 - 1.16 (m, 2H), 0.84 (s, 9H), 2 x exchangeable Hs not observed. |

**Example 19**

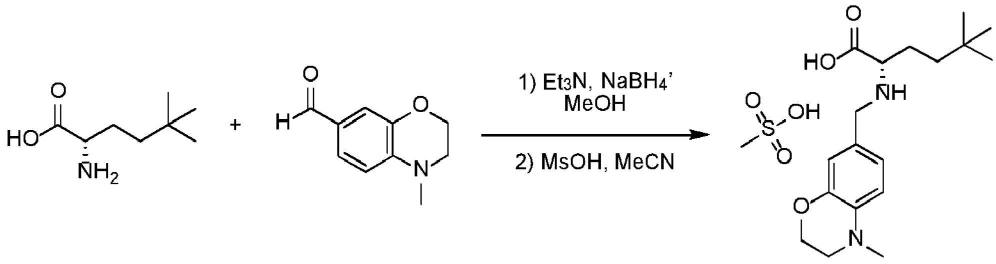


**Synthesis of (S)-5,5-dimethyl-2-(((4-methyl-3,4-dihydro-2H-benzo[b][1,4]oxazin-7-yl)methyl)amino)hexanoic acid, Mesylate**

A suspension of (S)-2-amino-5,5-dimethylhexanoic acid (112 mg, 1 Eq, 705 μmol), 4-methyl-3,4-dihydro-2H-benzo[b][1,4]oxazine-7-carbaldehyde (125 mg, 1 Eq, 705 μmol) and $Et_3N$ (71.9 mg, 99.0 μL, 1.01 Eq, 710 μmol) in MeOH (5.00 mL) was heated at 40 °C for 2.5 hours before being cooled with an ice bath and treated with $NaBH_4$ (27.0 mg, 1.01 Eq, 714 μmol). The mixture was then allowed to warm to rt and stir for 2.5 h before the reaction mixture was filtered. The solvent was removed *in vacuo* from the filtrate. The solid was triturated with water (3 mL) before being filtered and washed with MeCN (5 mL) to afford a white solid. This material was purified by chromatography on RP Flash C18 (4 g cartridge, 5-40% (0.1 % Formic acid in MeCN) / (0.1% Formic Acid in Water)) to afford (S)-5,5-dimethyl-2-(((4-methyl-3,4-dihydro-2H-benzo[b][1,4]oxazin-7-yl)methyl)amino)hexanoic acid (29.0 mg, 86 μmol, 12 %, 95% Purity) as a cream solid.

LCMS (Method #acid3minb, 1.34 min; M+H = 320.2. 1H NMR (500 MHz, MeOD) δ 0.92 (s, 9H), 1.28 - 1.43 (m, 2H), 1.73 - 1.88 (m, 2H), 2.92 (s, 3H), 3.28 - 3.31 (m, 3H), 3.44 (t, J = 6.0 Hz, 1H), 3.95 (d, J = 13.0 Hz, 1H), 4.08 (d, J = 12.9 Hz, 1H), 4.26 - 4.31 (m, 2H), 6.74 (d, J = 8.2 Hz, 1H), 6.85 (d, J = 2.1 Hz, 1H), 6.91 (dd, J = 2.1, 8.2 Hz, 1H).

(S)-5,5-dimethyl-2-(((4-methyl-3,4-dihydro-2H-benzo[b][1,4]oxazin-7-yl)methyl)amino)hexanoic acid (29.0 mg, 95% Wt, 1 Eq, 86.0 μmol) was stirred in MeOH (2.50 mL) before the addition of methanesulfonic acid (0.1 M in MeCN) (8.26 mg, 860 μL, 0.10 molar, 1 Eq, 86.0 μmol). The resultant solution was stirred at 25 °C for 30 min before being concentrated *in vacuo* to afford the product which was dried in the vacuum desiccator at 25 °C for 15 h. (S)-5,5-dimethyl-2-(((4-methyl-3,4-dihydro-2H-benzo[b][1,4]oxazin-7-yl)methyl)amino)hexanoic acid, Mesylate (27.0 mg, 64 μmol, 75 %, 99% Purity) was afforded as a green/yellow solid.

LCMS (Method #acid3minb, 0.97 min; M+H = 320.5. 1H NMR (500 MHz, DMSO) δ 14.00 (bs, 1H), 9.07 (bs, 1H), 8.99 (bs, 1H), 6.85 (dd, J = 8.2, 2.1 Hz, 1H), 6.81 (d, J = 2.0 Hz, 1H), 6.71 (d, J = 8.3 Hz, 1H), 4.25 - 4.20 (m, 2H), 4.00 - 3.97 (m, 2H), 3.81 (bs, 1H), 3.28 - 3.22 (m, 2H), 2.84 (s, 3H), 2.29 (s, 3H), 1.89 - 1.79 (m, 1H), 1.78 - 1.69 (m, 1H), 1.30 (td, J = 13.1, 4.7 Hz, 1H), 1.09 (td, J = 13.0, 4.3 Hz, 1H), 0.85 (s, 9H).

The following Examples can be prepared in an analogous manner to Example 19, starting from the corresponding aldehyde.

| Ex. | Structure | Yield | UPLC | NMR |
|---|---|---|---|---|
| 20 | 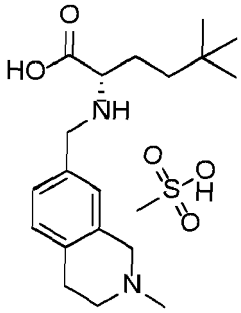 | 3.00 mg, (2.2% yield, 95% Purity) | Method #basic3minb, 1.12 min, M+H = 319.0 | 1H NMR (500 MHz, DMSO) δ 7.36 (d, J = 8.0 Hz, 1H), 7.30 (d, J = 7.9 Hz, 1H), 7.24 (s, 1H), 4.40 - 4.28 (m, 2H), 4.06 - 4.02 (m, 2H), 3.74 - 3.54 (m, 1H), 3.12 - 3.03 (m, 2H), 2.93 (s, 3H), 2.30 (s, 3H), 1.83 - 1.67 (m, 2H), 1.29 (app. td, J = 13.1, 4.9 Hz, 1H), 1.21 - 1.09 (m, 1H), 0.86 (s, 9H), CH2 obscured by residual solvent - not observed by HSQC, 3 x exchangeable Hs not observed. |
| 21 | 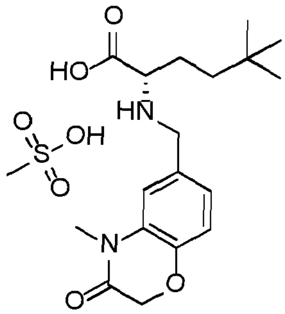 | 30.0 mg, (19% yield, 98% Purity) | Method #acid3minb, 1.23 min, M+H = 335.2 | $^{1}$H NMR (500 MHz, DMSO) δ 13.99 (s, 1H), 9.47 (s, 2H), 7.43 (s, 1H), 7.12 (dd, J = 8.1, 1.9 Hz, 1H), 7.04 (d, J = 8.1 Hz, 1H), 4.68 (s, 2H), 4.14 (s, 2H), 3.89 - 3.79 (m, 1H), 1.96 - 1.71 (m, 2H), 1.32 (app. td, J = 13.1, 4.7 Hz, 1H), 1.17 - 1.07 (m, 1H), 0.86 (s, 9H). |
| 22 | 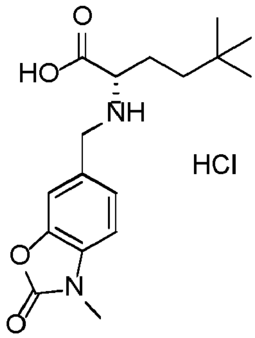 | 44 mg, (32% yield, 97% purity) | Method #basic3min, 0.62 min, M+H = 321.3 | $^{1}$H NMR (500 MHz, DMSO) δ 14.08 (br.s, 1H), 9.35 (br.s, 2H), 7.53 - 7.48 (m, 1H), 7.38-7.28 (m, 2H), 4.19 (s, 2H), 3.89 - 3.81 (m, 1H), 3.36 (s, 3H), 1.93 - 1.83 (m, 1H), 1.84-1.73 (m, 1H), 1.32 (app.td, *J* = 13.2, 4.7 Hz, 1H), 1.11 (app.td, *J* = 13.0, 4.3 Hz, 1H), 0.86 (s, 9H). |
| 23 | 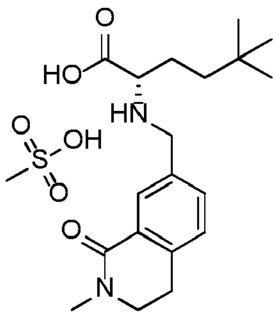 | 23.0 mg, (19% yield, 95% Purity) | Method #acid3minb, 1.28 min, M+H = 333.2 | $^{1}$H NMR (500 MHz, DMSO) δ 8.01 (s, 1H), 7.54 - 7.48 (m, 1H), 7.34 (d, J = 7.8 Hz, 1H), 4.12 - 4.08 (m, 2H), 3.74 - 3.62 (m, 1H), 3.55 (t, J = 6.7 Hz, 2H), 3.04 (s, 3H), 2.99 (t, J = 6.7 Hz, 2H), 2.29 (s, 3H), 1.81 - 1.66 (m, 2H), 1.29 (app. td, J = 12.8, 5.1 Hz, 1H), 1.18 - 1.07 (m, 1H), 0.85 (s, 9H), 3 x exchangeable Hs not observed. |

(continued)

| Ex. | Structure | Yield | UPLC | NMR |
|---|---|---|---|---|
| 24 | 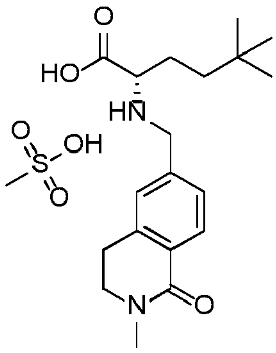 | 70.0 mg, (59% yield, 95% Purity) | Method #acid3min b, 1.27 min, M+H = 333.2 | $^1$H NMR (500 MHz, DMSO) δ 9.25 (v. br. s, 2H), 7.91 (d, J = 7.9 Hz, 1H), 7.44 (d, J = 8.1 Hz, 1H), 7.39 (s, 1H), 4.16 (s, 2H), 3.93 - 3.89 (m, 1H), 3.56 (t, J = 6.7 Hz, 2H), 3.03 (s, 3H), 3.00 (t, J = 6.6 Hz, 2H), 2.29 (s, 3H), 1.87 - 1.73 (m, 2H), 1.32 (app. td, J = 13.1, 4.7 Hz, 1H), 1.18-1.09 (m, 1H), 0.86 (s, 9H), 1 x exchangeable H not observed |
| 25 | 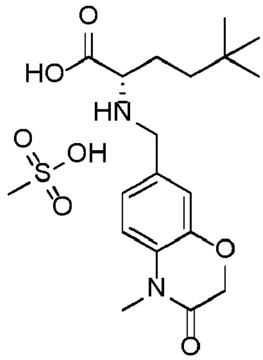 | 68.0 mg, (48% yield, 95% Purity) | Method #acid3min b, 1.25 min, M+H = 335.2 | $^1$H NMR (500 MHz, DMSO) δ 7.24 - 7.18 (m, 1H), 7.17-7.13 (m, 2H), 7.11 (s, 1H), 7.01 (s, 1H), 4.68 (s, 2H), 4.12 - 4.03 (m, 2H), 3.79 - 3.75 (m, 1H), 3.28 (s, 3H), 2.31 (s, 3H), 1.88 - 1.69 (m, 2H), 1.31 (app. td, J = 13.1, 4.7 Hz, 1H), 1.12 (app. td, J = 12.9, 4.4 Hz, 1H), 0.86 (s, 9H), 1 x exchangeable H not observed |
| 26 | 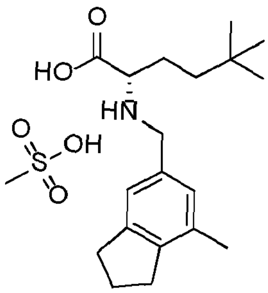 | 37.0 mg, (40% yield, 90% Purity) | Method #acid3min b, 1.60 min, M+H = 304.2 | $^1$H NMR (500 MHz, DMSO) δ 14.00 (s, 1H), 9.18 (s, 1H), 9.09 (s, 1H), 7.15 (s, 1H), 7.05 (s, 1H), 4.10 - 4.05 (m, 2H), 3.93 - 3.81 (m, 1H), 2.88 (t, J = 7.5 Hz, 2H), 2.80 (t, J = 7.4 Hz, 2H), 2.34 (s, 3H), 2.23 (s, 3H), 2.03 (app. p, J = 7.5 Hz, 2H), 1.91 - 1.72 (m, 2H), 1.32 (app. td, J = 13.1, 4.7 Hz, 1H), 1.15-1.05 (m, 1H), 0.85 (s, 9H). |
| 27 | 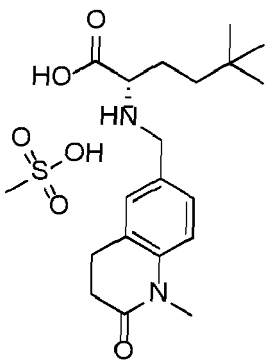 | 15 mg, (11% yield, 95% Purity) | Method #acid3min b, 1.25 min, M-H = 331.2 | $^1$H NMR (500 MHz, DMSO) δ 7.35 (dd, J = 8.3, 2.1 Hz, 1H), 7.32 (d, J = 2.0 Hz, 1H), 7.14 (d, J = 8.3 Hz, 1H), 4.11 - 4.02 (m, 2H), 3.79 - 3.76 (m, 1H), 3.26 (s, 3H), 2.91 - 2.85 (m, 2H), 2.58 - 2.52 (m, 2H), 2.29 (s, 3H), 1.85 - 1.69 (m, 2H), 1.31 (app. td, J = 13.1, 4.8 Hz, 1H), 1.13 (app. td, J = 13.0, 4.6 Hz, 1H), 0.86 (s, 9H), 3 x exchangeable Hs not observed. |

(continued)

| Ex. | Structure | Yield | UPLC | NMR |
|---|---|---|---|---|
| 28 | 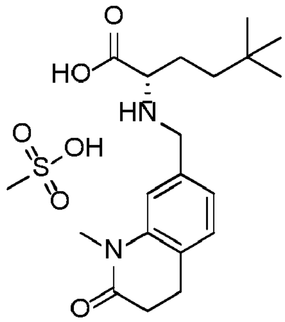 | 98 mg, (59% yield, 98% Purity) | Method #acid3min b, 1.29 min, M+H = 333.2 | $^1$H NMR (500 MHz, DMSO) δ 9.16 (s, 2H), 7.28 (d, J = 7.6 Hz, 1H), 7.23 (d, J = 1.6 Hz, 1H), 7.09 (dd, J = 7.5, 1.6 Hz, 1H), 4.20 - 4.10 (m, 2H), 3.86 - 3.82 (m, 1H), 3.27 (s, 3H), 2.91 - 2.85 (m, 2H), 2.57 - 2.51 (m, 2H), 2.29 (s, 3H), 1.89 - 1.70 (m, 2H), 1.31 (app. td, J = 13.1, 4.6 Hz, 1H), 1.16-1.10 (m, 1H), 0.86 (s, 9H) 1 × OH not observed. |
| 29 | 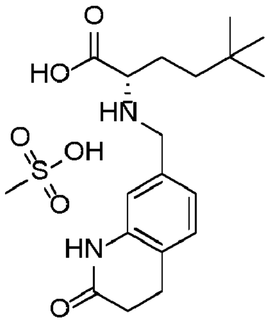 | 23 mg, (26% yield, 95% Purity) | Method #acid3min b, 1.23 min, M+H = 319.2 | 1H NMR (500 MHz, DMSO) δ 10.30 (s, 1H), 9.30 - 9.04 (m, 2H), 7.24 (d, J = 7.7 Hz, 1H), 7.05 - 6.99 (m, 1H), 6.92 (d, J = 1.7 Hz, 1H), 4.09 - 4.05 (m, 2H), 3.95 - 3.92 (m, 1H), 2.89 (t, J = 7.6 Hz, 2H), 2.47 - 2.43 (m, 2H), 2.30 (s, 3H), 1.88 - 1.73 (m, 2H), 1.32 (app. td, J = 13.2, 4.7 Hz, 1H), 1.11 (app. td, J = 12.8, 4.2 Hz, 1H), 0.86 (s, 9H), 1 × OH not observed |
| 30 | 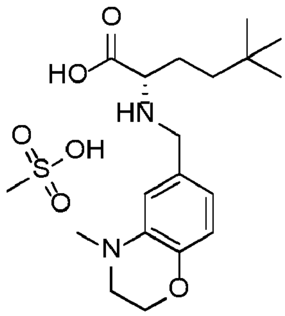 | 70 mg, (45% yield, 90% Purity) | Method #acid3min b, 1.49 min, M-H = 319.2 | $^1$H NMR (500 MHz, DMSO) δ 6.80 (d, J = 1.9 Hz, 1H), 6.68 (d, J = 8.0 Hz, 1H), 6.65 - 6.60 (m, 1H), 4.24 - 4.21 (m, 2H), 4.02 - 3.89 (m, 2H), 3.61 - 3.58 (m, 1H), 3.24 (t, J = 4.4 Hz, 2H), 2.84 (s, 3H), 2.29 (s, 3H), 1.79 - 1.68 (m, 2H), 1.29 (app. td, J = 13.1, 4.7 Hz, 1H), 1.13 (app. dt, J = 13.0, 6.8 Hz, 1H), 0.84 (s, 9H), 3 x exchangeable Hs not observed. |
| 31 | 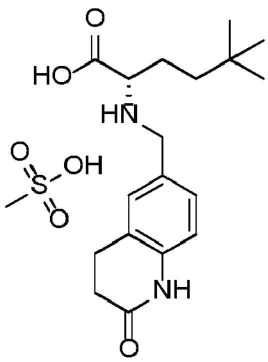 | 17 mg, (11% yield, 95% Purity) | Method #acid3min b, 1.28 min, M+H = 319.2 | $^1$H NMR (500 MHz, DMSO) δ 10.20 (s, 1H), 7.26 (s, 1H), 7.24 - 7.19 (m, 1H), 6.87 (d, J = 8.1 Hz, 1H), 4.06 - 3.94 (m, 2H), 3.77 - 3.73 (m, 1H), 2.89 (t, J = 7.5 Hz, 2H), 2.48 - 2.44 (m, 2H), 2.29 (s, 3H), 1.86 - 1.66 (m, 2H), 1.29 (app. dt, J = 13.2, 6.7 Hz, 1H), 1.16-1.08 (m, 1H), 0.85 (s, 9H), 3 x exchangeable Hs not observed |

(continued)

| Ex. | Structure | Yield | UPLC | NMR |
|---|---|---|---|---|
| 32 | 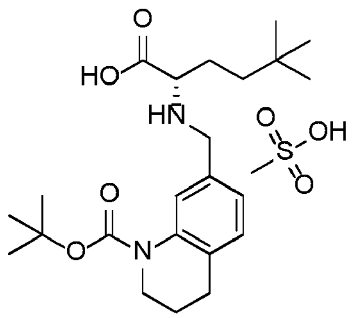 | 13 mg, (80% yield, 98% Purity) | Method #acid3min , 1.72 min, M+H = 405.2 | $^{1}$H NMR (500 MHz, DMSO) δ 9.10 (br. s, 2H), 7.76 (s, 1H), 7.17 (d, J = 7.8 Hz, 1H), 7.11 - 7.05 (m, 1H), 4.13 - 4.00 (m, 2H), 3.85 - 3.74 (m, 1H), 3.66 - 3.56 (m, 2H), 2.73 (t, J = 6.5 Hz, 2H), 2.29 (s, 3H), 1.88 - 1.68 (m, 4H), 1.48 (s, 9H), 1.37 - 1.27 (m, 1H), 1.13 (app. td, J = 12.9, 4.4 Hz, 1H), 0.85 (s, 9H), 1 x exchangeable H not observed. |

The following Examples were prepared in an analogous manner to the other examples, starting from their corresponding ketone, aldehyde, or ester.

| Example | Structure | Yield | LCMS | NMR |
|---|---|---|---|---|
| 33 | 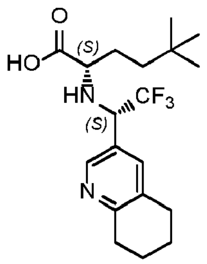 | 19.5 mg (16% yield, 98. 21% purity) | Method 7, 1.123 min; M+H = 373.2; calcd. 373.2 | $^{1}$H-NMR (400 MHz, DMSO) δ 8.31 (d, *J* = 2.3 Hz, 1H), 7.53 (s, 1H), 4.36 (q, *J* = 8.2 Hz, 1H), 3.14 (s, 2H), 2.84 - 2.70 (m, 5H), 1.85 -1.73 (m, 4H), 1.66 -1.37 (m, 2H), 1.23 (dt, *J* = 11.8, 5.8 Hz, 2H), 0.85 (s, 9H). |
| 34 | 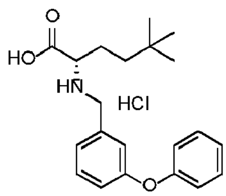 | 1.4 mg (2% yield, 97.62% purity) | Method 1, 1.179 min; M+H-HCl = 342.6; calcd. 342.2 | |
| 35 | 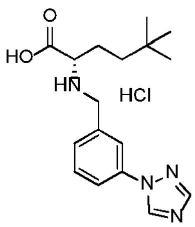 | 7.2 mg (16% yield, 98.96% purity) | Method 1, 1.083 min; M+H-HCl-= 317.2; calcd. 317.2 | |
| 36 | 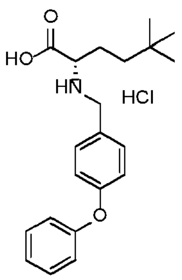 | 1.0 mg (2% yield, 98.49% purity) | Method 1, 1.262 min; M+H-HCl-= 342.3; calcd. 342.2 | |

(continued)

| Example | Structure | Yield | LCMS | NMR |
|---|---|---|---|---|
| 37 | 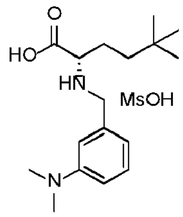 | 40.5 mg (31% yield, 97.54% purity) | Method 1, 0.990 min; M+H-MsOH = 293.2; calcd. 293.2 | $^1$H-NMR (400 MHz, DMSO) δ 9.32 - 9.09 (m, 2H), 7.28 - 7.20 (m, 1H), 6.86 (s, 1H), 6.79 (d, *J* = 8.0 Hz, 1H), 6.75 (d, *J* = 7.5 Hz, 1H), 4.14-4.06 (m, 2H), 3.90 - 3.85 (m, 1H), 2.31 (s, 3H), 1.88 - 1.71 (m, 2H), 1.32 (td, *J* = 13.1, 4.8 Hz, 1H), 1.12 (td, *J* = 12.9, 4.4 Hz, 1H), 0.86 (s, 9H). |
| 38 | 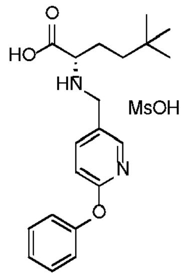 | 50.4 mg (9% yield, 100% purity) | Method 1, 0.999 min; M+H-MsOH- = 343.5; calcd. 343.2 | $^1$H-NMR (400 MHz, DMSO) δ 8.18 (d, *J* = 2.5 Hz, 1H), 7.92 (dd, *J* = 8.4, 2.4 Hz, 1H), 7.49 - 7.39 (m, 2H), 7.29 - 7.21 (m, 1H), 7.14 -7.11 (m, 1H), 7.11 -7.07 (m, 2H), 4.14 - 3.97 (m, 2H), 3.80 - 3.71 (m, 1H), 2.29 (s, 3H), 1.82 - 1.69 (m, 2H), 1.28 (td, *J* = 12.8, 5.4 Hz, 1H), 1.14 (td, *J* = 12.6, 5.0 Hz, 1H), 0.86 (s, 9H). |
| 39[1,2] | 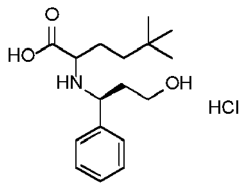 | 20.8 mg (39% yield, 98.81% purity) | Method 7, 0.896 min; M+H-HCl-= 294.2; calcd. 294.2 | $^1$H-NMR (400 MHz, DMSO) δ 10.21 - 9.33 (m, 2H), 7.62-7.35 (m, 5H), 4.38 (brs, 1H), 3.59 (brs, 1H), 3.39-3.17 (m, 2H), 3.09 - 2.99 (m, 1H), 2.36 (brs, 1H), 2.10 (brs, 1H), 1.98 - 1.65 (m, 2H), 1.34 - 1.16 (m, 1H), 1.09 -0.94 (m, 1H), 0.90 - 0.75 (m, 9H). |
| 40[1] | 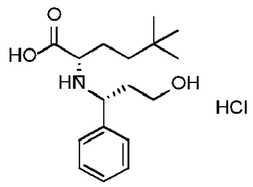 | 60.7 mg (56% yield, 98.95% purity) | Method 1, 0.871 min; M+H-HCl-= 294.5; calcd. 294.2 | $^1$H-NMR (400 MHz, DMSO) δ 9.44 (br, 2H), 7.63-7.35 (m, 5H), 4.79 (brs, 1H), 4.40-4.32 (m, 1H), 3.63-3.55 (m, 1H), 3.31 -3.27 (m, 1H), 3.10 - 3.04 (m, 1H), 2.38 - 2.29 (m, 1H), 2.11 - 2.05 (m, 1H), 1.92 - 1.85 (m, 1H), 1.78 - 1.68 (m, 1H), 1.27 (td, *J* = 13.2, 4.8 Hz, 1H), 1.01 (td, *J* = 13.1, 4.2 Hz, 1H), 0.85 (s, 9H). |
| 41 | 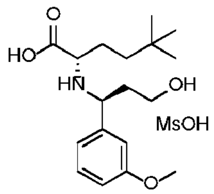 | 23.4 mg (36% yield, 96.59% purity) | Method 1, 0.907 min; M+H-MsOH- = 324.4; calcd. 324.2 | $^1$H-NMR (400 MHz, DMSO) δ 9.30 (brs, 2H), 7.39 (t, *J* = 7.9 Hz, 1H), 7.05 (t, *J* = 2.1 Hz, 1H), 7.01 (dd, *J* = 8.1, 2.5 Hz, 1H), 6.98 (d, *J* = 7.6 Hz, 1H), 5.01 -4.56 (brs, 1H), 4.39 - 4.30 (m, 1H), 3.77 (s, 3H), 3.41 - 3.38 (m, 1H), 3.09 (td, *J* = 10.3, 4.5 Hz, 1H), 2.37 - 2.25 (m, 4H), 2.06 - 1.94 (m, 1H), 1.78 - 1.57 (m, 2H), 1.20 (td, *J* = 12.9, 5.1 Hz, 1H), 1.06 (td, *J* = 12.7, 4.8 Hz, 1H), 0.80 (s, 9H). 1H missing |
| 42 | 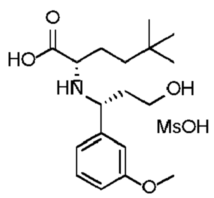 | 18.3 mg (28% yield, 99.05% purity) | Method 1, 0.898 min; M+H-MsOH- = 324.4; calcd. 324.2 | $^1$H NMR (400 MHz, DMSO) δ 9.28 (brs, 2H), 7.37 (t, *J* = 7.9 Hz, 1H), 7.10 (t, *J* = 2.1 Hz, 1H), 7.04 (d, *J* = 7.5 Hz, 1H), 7.00 (dd, *J* = 8.3, 2.5 Hz, 1H), 4.98 - 4.62 (brs, 1H), 4.43 - 4.30 (m, 1H), 3.78 (s, 3H), 3.61 - 3.54 (m, 1H), 3.16-3.06 (m, 1H), 2.31 - 2.22 (m, 4H), 2.10 - 1.98 (m, 1H), 1.92 - 1.81 (m, 1H), 1.77 - 1.63 (m, 1H), 1.32 - 1.20 (m, 1H), 1.01 (td, *J* = 12.8, 4.0 Hz, 1H), 0.86 (s, 9H). 1H missing |

(continued)

| Example | Structure | Yield | LCMS | NMR |
|---|---|---|---|---|
| 43 | 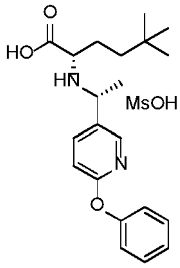 | 28.8 mg (14% yield, 100% purity) | Method 7, 1.046 min; M+H-MsOH = 357.2; calcd. 357.2 | $^1$H-NMR (400 MHz, DMSO) δ 9.42-8.72 (br, 2H), 8.21 (d, *J* = 2.5 Hz, 1H), 7.97 (dd, *J* = 8.6, 2.5 Hz, 1H), 7.44 (t, *J* = 7.9 Hz, 2H), 7.24 (t, *J* = 7.4 Hz, 1H), 7.12 (dd, *J* = 8.4, 6.7 Hz, 3H), 4.47-4.34 (m, 1H), 3.81 - 3.68 (m, 1H), 2.29 (s, 3H), 1.87 - 1.75 (m, 1H), 1.75 - 1.64 (m, 1H), 1.57 (d, *J* = 6.7 Hz, 3H), 1.26 (td, *J* = 13.2, 4.8 Hz, 1H), 1.08 (dt, *J* = 12.4, 4.0 Hz, 1H), 0.86 (s, 9H). |
| 44 | 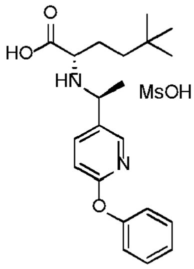 | 30.4 mg (13% yield, 100% purity) | Method 7, 1.049 min; M+Na-MsOH = 357.2; calcd. 357.2 | $^1$H-NMR (400 MHz, DMSO) δ 8.12 (s, 1H), 7.93 (d, *J* = 8.6 Hz, 1H), 7.43 (t, *J* = 7.9 Hz, 2H), 7.23 (t, *J* = 7.4 Hz, 1H), 7.13 (d, *J* = 7.6 Hz, 3H), 7.09 (d, *J* = 8.5 Hz, 1H), 4.22 (s, 1H), 2.30 (s, 3H), 1.67 - 1.56 (m, 2H), 1.50 (d, *J* = 7.4 Hz, 3H), 1.15 (t, *J* = 7.9 Hz, 2H), 0.82 (s, 9H). |

[1] was reformatted using HCl instead of 1 equiv MsOH
[2] reaction performed using racemic triflate

The following examples were synthesised in an analogous manner to the other examples.

| Example | Structure |
|---|---|
| 45 | 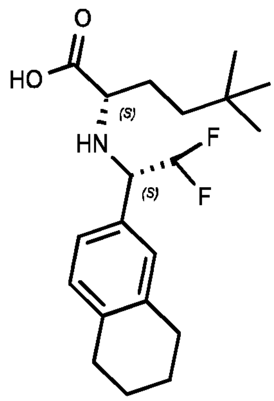 |
| 46 | 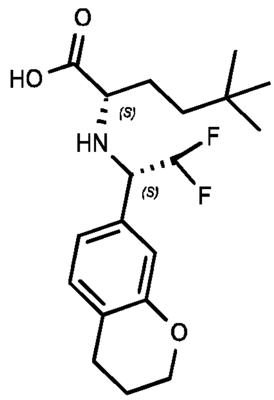 |

(continued)

| Example | Structure |
|---|---|
| 47 | 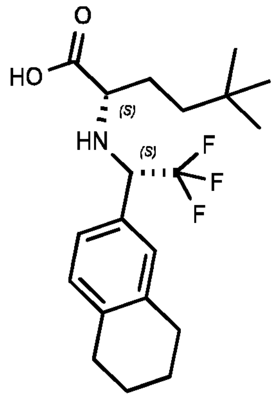 |
| 48 | 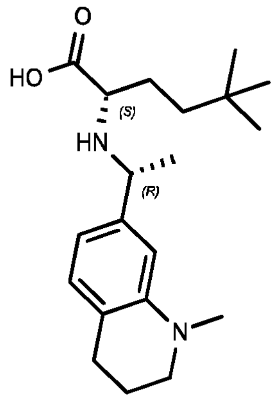 |
| 49 | 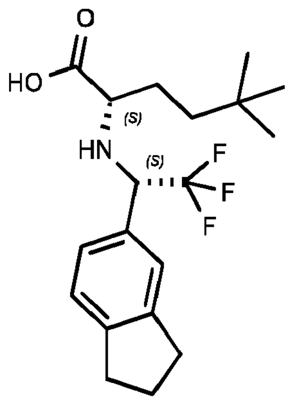 |
| 50 | 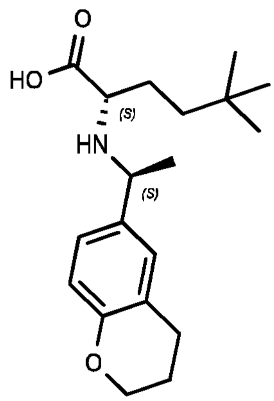 |

(continued)

| Example | Structure |
|---|---|
| 51 | 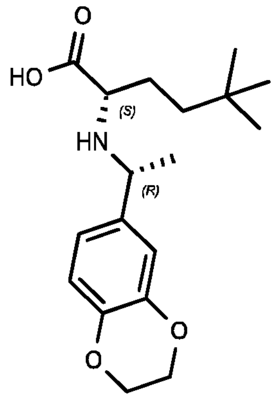 |
| 52 | 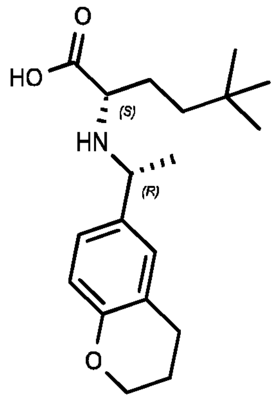 |
| 53 | 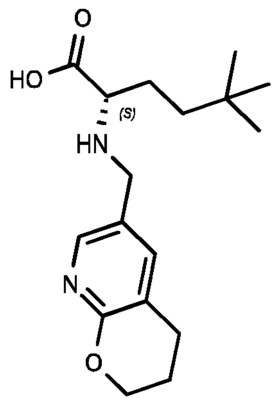 |
| 54 | 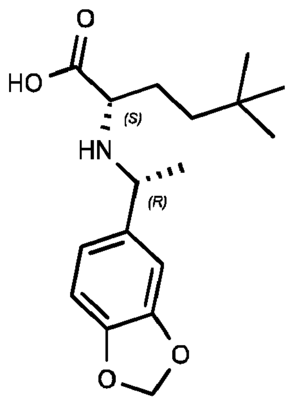 |

(continued)

| Example | Structure |
|---|---|
| 55 | 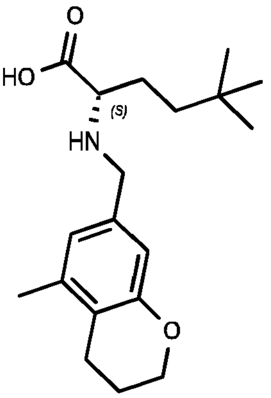 |
| 56 | 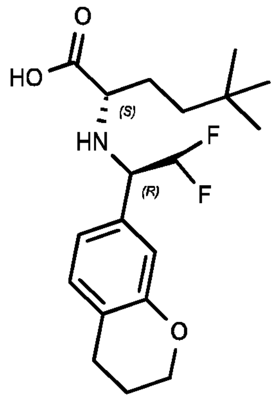 |
| 57 | 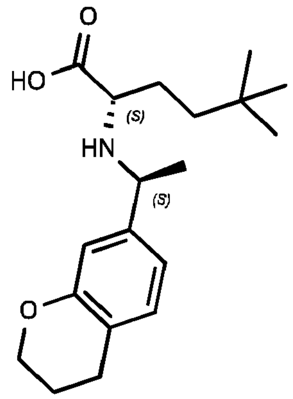 |
| 58 | 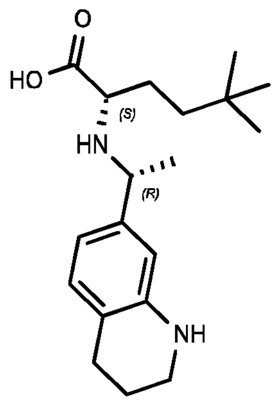 |

(continued)

| Example | Structure |
|---|---|
| 59 | 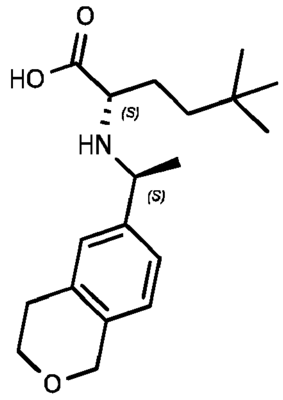 |
| 60 | 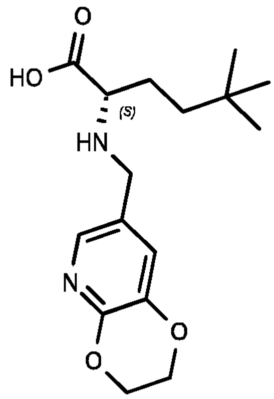 |
| 61 | 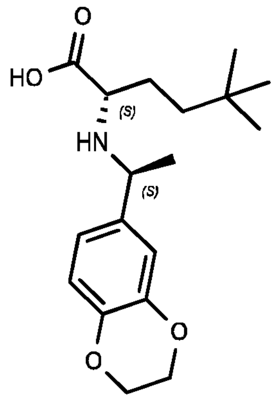 |
| 62 | 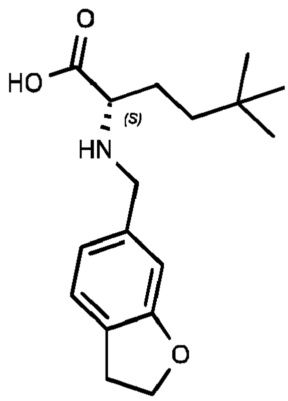 |

(continued)

| Example | Structure |
|---|---|
| 63 | 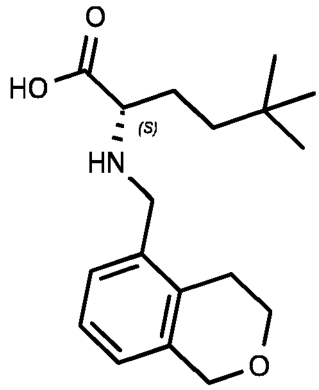 |
| 64 | 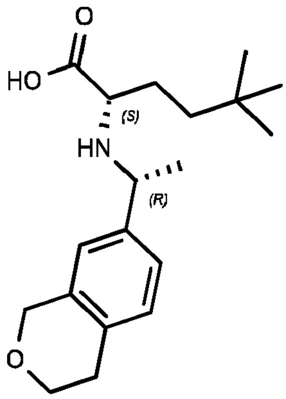 |
| 65 | 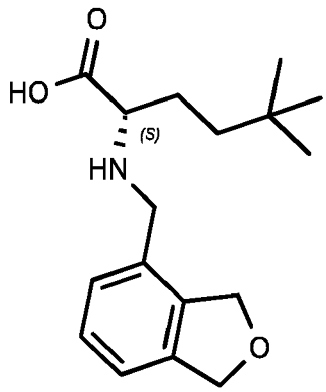 |
| 66 | 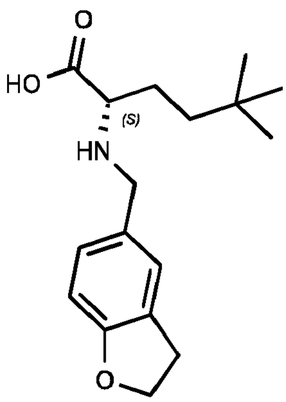 |

(continued)

| Example | Structure |
|---|---|
| 67 | 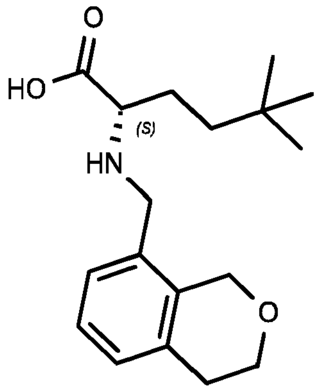 |
| 68 | 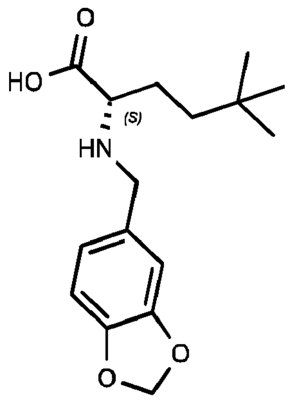 |
| 69 | 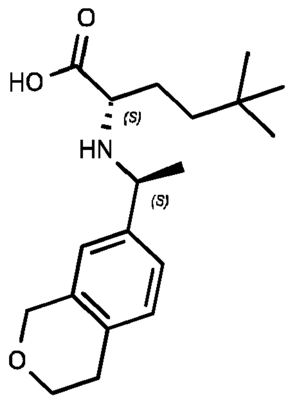 |
| 70 | 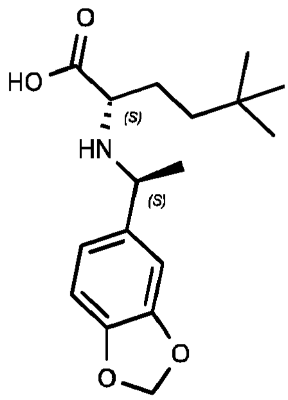 |

(continued)

| Example | Structure |
|---|---|
| 71 | 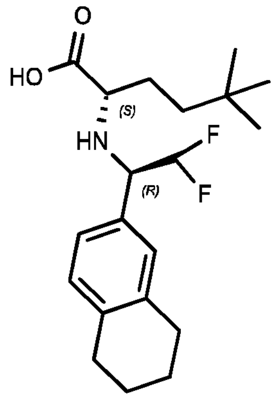 |
| 72 | 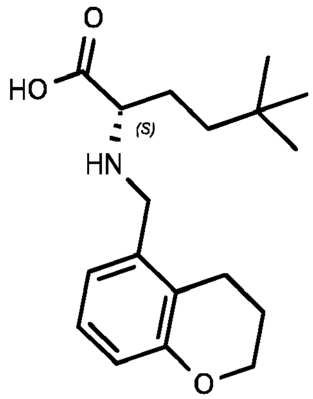 |
| 73 | 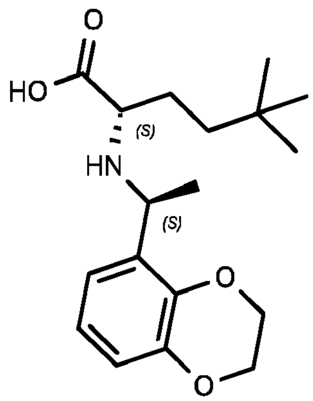 |
| 74 | 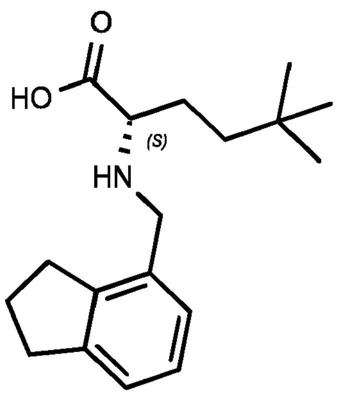 |

(continued)

| Example | Structure |
|---|---|
| 75 | 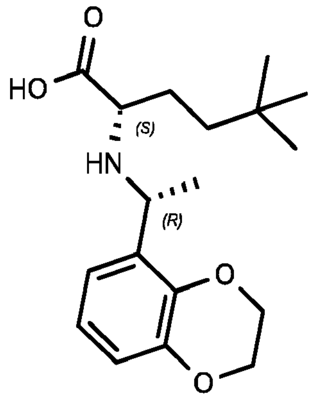 |
| 76 | 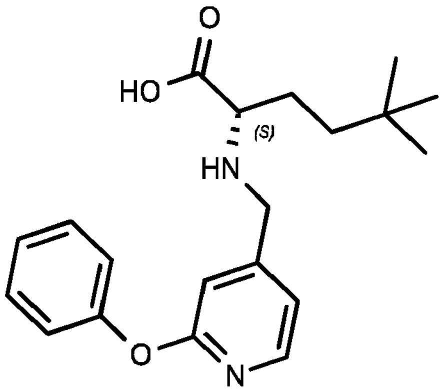 |
| 77 | 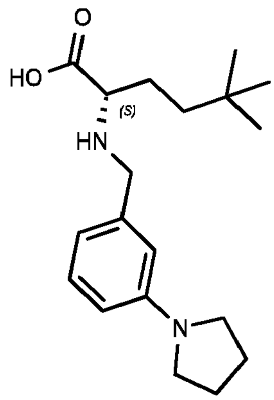 |

## BIOLOGICAL DATA

### Neurotensin scintillation proximity assay

The exemplified compounds of the invention were tested in a Neurotensin (NTS) scintillation proximity assay (SPA). The $IC_{50}$ data is shown in the table below. NTS, which is a 13 amino acid neuropeptide, is a sortilin ligand. The $IC_{50}$ is a measure of the amount of the compound required to inhibit the binding of NTS to sortilin by 50%. The skilled person will recognise that the lower the $IC_{50}$ value, the less of the compound needed to achieve the desired effect, and as a result, the chances of undesirable off-target effects are reduced.

Compound affinity was determined by measuring the displacement of [3H]-neurotensin binding to *h*-Sortilin in SPA format. Total volume of 40 µl in 50 mM HEPES pH 7.4 assay buffer containing 100 mM NaCl, 2.0 mM CaCl2, 0.1% BSA and 0.1% Tween-20. Compound pre-incubation for 30 minutes at room temperature with 150 nM of 6his-Sortilin before 5 nM [3H]-Neurotensin and Ni chelate imaging beads (Perkin Elmer) were added, after 6 hours the plate was

read on a ViewLux with 360 s exposure time. Dose-response evaluation of compounds was performed with 8 concentrations of drugs (covering 3 decades). $IC_{50}$ values were calculated by nonlinear regression using the sigmoid concentration-response (variable slope) using CDD Vault software. All values reported are average of at least 2 determinations.

The data in the table below shows that the compounds of the invention are sortilin inhibitors.

| Example | $IC_{50}$ [3H]Neurotensin SPA (μM) |
|---|---|
| 1 | 0.27 |
| 2 | 0.35 |
| 3 | 0.23 |
| 4 | 0.12 |
| 5 | 0.31 |
| 6 | 0.21 |
| 7 | 0.14 |
| 8 | 0.5 |
| 9 | 0.21 |
| 10 | 0.64 |
| 11 | 0.12 |
| 12 | 0.61 |
| 13 | 0.21 |
| 14 | 0.28 |
| 15 | 0.9 |
| 16 | 0.57 |
| 17 | 0.19 |
| 18 | 0.5 |
| 19 | 0.66 |
| 20 | 0.47 |
| 21 | 0.26 |
| 22 | 0.47 |
| 23 | 0.43 |
| 24 | 0.63 |
| 25 | 0.95 |
| 26 | 0.52 |
| 27 | 0.76 |
| 28 | 0.22 |
| 29 | 0.29 |
| 30 | 0.35 |
| 31 | 0.9 |
| 32 | 0.13 |
| 33 | 0.13 |
| 34 | 0.82 |
| 35 | 0.96 |

(continued)

| Example | $IC_{50}$ [3H]Neurotensin SPA (μM) |
|---|---|
| 36 | 0.52 |
| 37 | 0.57 |
| 38 | 0.34 |
| 39 | 0.41 |
| 40 | 6.45 |
| 41 | 0.76 |
| 42 | 0.59 |
| 43 | 0.41 |
| 44 | 0.74 |
| 45 | 0.12 |
| 46 | 0.13 |
| 47 | 0.16 |
| 49 | 0.22 |
| 50 | 0.23 |
| 51 | 0.28 |
| 52 | 0.26 |
| 53 | 0.58 |
| 54 | 0.38 |
| 55 | 0.31 |
| 56 | 0.31 |
| 57 | 0.3 |
| 58 | 0.31 |
| 59 | 0.32 |
| 60 | 0.82 |
| 61 | 0.35 |
| 62 | 0.43 |
| 63 | 0.65 |
| 64 | 0.42 |
| 65 | 0.62 |
| 66 | 0.7 |
| 67 | 0.5 |
| 68 | 0.86 |
| 69 | 0.59 |
| 70 | 0.53 |
| 71 | 0.59 |
| 72 | 1.08 |
| 73 | 0.65 |
| 74 | 0.85 |

(continued)

| Example | $IC_{50}$ [3H]Neurotensin SPA (μM) |
|---|---|
| 75 | 1.22 |
| 76 | 0.23 |
| 77 | 0.35 |

**Creoptix (GCI) method - using SEQ ID NO.4 (Murine sortillin)**

The GCI assay is based upon understood surface plasmon resonance methodologies specifically enhanced for detecting the binding of small entities to proteins. A protein, bound to a surface, is bathed in a solution containing potential ligands giving rise to binding kinetics to generate $K_{on}$ and $K_{off}$ rates, as well as a $K_d$. This methodology does not require the use of additional tracers and can be used with or without the element of competition with known ligands.

**Reagents:**

| Reagent | Part no | Supplier |
|---|---|---|
| DMSO | D8418 | Sigma |
| Borate | B BELU-50 | Xantec Bioanalytics |
| EDTA | E7889-100ML | Merck Life Science UK |
| HBS-N | BR-1006-70 | Cytiva |
| 4PCH wave chip | 4PCH | Creoptix AG |
| rhSortilin | 3154-ST-050 | Bio-Techne Ltd |
| rmSortilin | 2934-ST-050 | Bio-Techne Ltd |
| EDC | BR100050 | Cytiva |
| NHS | BR100050 | Cytiva |
| Acetate pH 5.0 | BR100351 | Cytiva |
| Trizma/ Tris | 93352-1 KG | Merck Life Science UK |

All buffers described were filtered using a 0.2μm filter (Product number: 10300461, Nalgene) and degassed for 15 minutes prior to use.

A flow cell temperature of 25 degrees Celsius was used throughout the experiment.

**Chip conditioning and immobilisation**

A 4PCH chip was conditioned across all flow cells using 0.2 X concentration running buffer (running buffer composition: 1XHBS-N, pH 7.4, 3.4 mM EDTA, 1% DMSO) using pre-set conditioning wizard (WAVE control software) injecting: 0.1 M borate, 1 M NaCl (pH 9) followed by three start-up injections of 0.2 X running buffer.

A buffer exchange was performed and 1x running buffer (1XHBS-N, pH 7.4, 3.4 mM EDTA, 1% DMSO) used during the immobilisation procedure:

An initial injection of EDC/NHS (mixed in 1:1 ratio) was performed across all 4-flow cells to activate the surface for amine coupling of ligands.

Recombinant Sortilin aliquots were thawed quickly by hand and centrifuged at 13,300 rpm for 10 minutes. A 10 μg/ml protein solution was then made in pH 5.0 acetate and injected once for 20 minutes over flow-cells 2, 3 and 4 for human, human (flow cells 2 & 3), and mouse Sortilin respectively followed by a 60 second dissociation period.

A final 7-minute passivation injection of 50 mM tris was used across all flow cells.

A flow rate of 10 μl/min was used for all conditioning and immobilisation cycles.

**Rapid Kinetics: Intermediate binders**

Compounds were screened at 1 μM using the in-built 'intermediate binders' settings: 100 ul/min, 45 s baseline, 25 s association, 300 s dissociation, with blanks every 5th sample and a DMSO correction (1.5 % DMSO at the start and end of the experiment as well as every 20 cycles. An acquisition rate of 10 Hz was used throughout the experiment.

Compounds were screened at a final assay concentration of 1 μM and a final DMSO concentration was 1 %. To achieve this, compounds were diluted in DMSO from 10 mM stocks to 100 μM (100 x final assay concentration) then diluted 1:100 in running buffer which contained no DMSO to establish a final assay concentration of compound of 1 μM and a final DMSO concentration of 1 % [DMSO].

Compounds and DMSO were mixed by plate shaking at 1000 rpm for 60 seconds using a Bioshake instrument.

A flow rate of 100 μl/min was used throughout the experiment.

**Data Evaluation:**

Data was evaluated using the RAPID kinetic analysis tool in the GCI WAVE_control software with data fitted using a standard 1:1 kinetic BioModel.

The results for compounds of the invention are shown in the table below.

| Example | Form of Compound | Species | KD (M) Sortilin |
|---|---|---|---|
| 1 | Free acid | Human | 1.07E-07 |
| 2 | Free acid | Human | 1.72E-07 |
| 3 | Free acid | Human | 2.37E-07 |
| 3 | Free acid | Mouse | 6.50E-08 |
| 3 | Mesylate salt | Human | 5.20E-08 |
| 4 | Free acid | Human | 4.09E-08 |
| 4 | HCl salt | Human | 5.17E-08 |
| 4 | Mesylate salt | Human | 4.25E-08 |
| 5 | Free acid | Human | 6.34E-08 |
| 6 | Free acid | Human | 1.17E-07 |
| 7 | Free acid | Human | 2.64E-07 |
| 8 | Free acid | Human | 1.91E-07 |
| 9 | Free acid | Human | 2.27E-07 |
| 9 | Free acid | Mouse | 6.00E-08 |
| 9 | Mesylate salt | Human | 6.49E-08 |
| 9 | Mesylate salt | Mouse | 5.95E-08 |
| 10 | Free acid | Human | 2.00E-07 |
| 10 | Free acid | Mouse | 2.41E-07 |
| 11 | Free acid | Human | 3.66E-08 |
| 11 | Mesylate salt | Human | 4.07E-08 |
| 13 | Mesylate salt | Human | 5.75E-08 |
| 14 | Mesylate salt | Human | 3.51E-08 |
| 16 | Free acid | Human | 2.55E-07 |
| 16 | Free acid | Mouse | 2.99E-07 |
| 17 | Free acid | Human | 9.73E-08 |
| 18 | Free acid | Human | 1.42E-06 |

(continued)

| Example | Form of Compound | Species | KD (M) Sortilin |
|---|---|---|---|
| 18 | Free acid | Mouse | 2.28E-06 |
| 19 | Free acid | Human | 3.72E-06 |
| 19 | Free acid | Mouse | 3.18E-06 |
| 21 | Free acid | Human | 1.64E-07 |
| 22 | Free acid | Human | 3.50E-07 |
| 25 | Free acid | Human | 5.63E-07 |
| 25 | Free acid | Human | 1.58E-07 |
| 28 | Free acid | Human | 3.17E-07 |
| 30 | Free acid | Human | 5.07E-06 |
| 30 | Free acid | Mouse | 7.60E-06 |
| 33 | Free acid | Human | 2.80E-08 |
| 34 | Free acid | Human | 2.16E-07 |
| 38 | Mesylate salt | Human | 4.91E-08 |
| 43 | Free acid | Human | 6.83E-08 |
| 44 | Free acid | Human | 1.27E-07 |
| 45 | Free acid | Human | 4.39E-08 |
| 46 | Free acid | Human | 4.34E-08 |
| 47 | Free acid | Human | 7.53E-08 |
| 48 | Free acid | Human | 4.62E-08 |
| 49 | Free acid | Human | 5.04E-07 |
| 50 | Free acid | Human | 1.20E-07 |
| 50 | Free acid | Mouse | 2.58E-07 |
| 50 | Mesylate salt | Human | 2.21E-07 |
| 50 | Mesylate salt | Mouse | 2.58E-07 |
| 51 | Free acid | Human | 1.81E-07 |
| 52 | Free acid | Human | 3.84E-07 |
| 52 | Mesylate salt | Human | 1.33E-07 |
| 52 | Mesylate salt | Mouse | 1.29E-07 |
| 54 | Free acid | Human | 1.05E-07 |
| 56 | Free acid | Human | 6.96E-07 |
| 58 | Free acid | Human | 6.70E-08 |
| 58 | Free acid | Mouse | 7.03E-08 |
| 59 | Mesylate salt | Human | 2.18E-07 |
| 59 | Mesylate salt | Mouse | 2.04E-07 |
| 61 | Free acid | Human | 1.44E-07 |
| 68 | Free acid | Human | 1.63E-07 |
| 70 | Free acid | Human | 1.33E-07 |
| 70 | Free acid | Mouse | 1.16E-07 |

(continued)

| Example | Form of Compound | Species | KD (M) Sortilin |
|---|---|---|---|
| 71 | Free acid | Human | 1.93E-07 |
| 72 | Free acid | Human | 6.13E-07 |
| 76 | Free acid | Human | 5.83E-08 |

For compounds of the invention it is advantageous to have $K_d$ lower than 1.00 E-4. The $K_d$ data generated demonstrate that examples of the invention bind directly to the sortilin protein and bind in a generally similar manner in both human and murine sortilin protein which is advantageous for the development of non-human models of pharmacokinetics and disease.

**BLOOD-BRAIN-BARRIER PERMEABILITY**

To determine whether the compounds of the invention are capable of crossing the blood brain barrier, the $K_{puu}$ was calculated for Example 3 and Comparative Example 1, which is a sortilin modulator that is not in accordance with the invention and has the following structure:

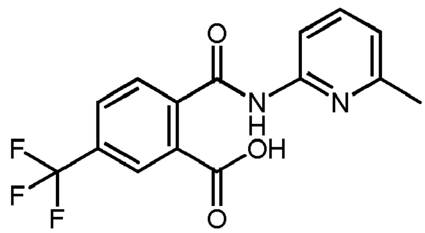

**Plasma protein binding and brain homogenate binding for study compounds in mouse, dog, and rat by rapid equilibrium dialysis**

Mice were dosed with the compound of Comparative Example 1 and rats and dogs were dosed with the compound of Example 3, and then the plasma and brain were removed at specific timepoints to be analysed for compound concentration. Separately, the fraction of compound bound to plasma protein or brain homogenate was measured to allow assessment of free fractions.

The free drug hypothesis states that only unbound compound is able to permeate through biological membranes, interact with and elicit a pharmacological effect.

Therefore, it is desirable for compounds to have a high free brain concentration. However, only the free unbound drug fraction is subject to Clearance mechanisms.

In practice, unbound fractions in plasma and brain tissue were assessed using rapid equilibrium dialysis *in vitro.* Separately a pharmacokinetic study was run *in vivo* whereby a dose of the compound of interest was given at T=0 hours and at subsequent timepoints (eg 0.5, 1 and 4 hours) plasma and separately brain samples were analysed for total concentration of the compound of interest. These total concentrations could then be adjusted with the unbound fraction to give the unbound concentrations in the plasma and brain. The unbound partition coefficient ($K_{puu}$) was then determined as a ratio between the free compound concentrations in the compartment of interest, here the brain/CNS and the plasma.

**Rapid equilibrium dialysis**

The test compounds were incubated in plasma and brain homogenate from the relevant species at 37 °C in RED device with inserts (8K MWCO, Thermo scientific) for 4 hours at 5 μM in triplicates. 350 μL of 150 mM phosphate buffered saline (PBS, pH 7.4) was used as the receiver side solutions. The samples were collected from both sides after 4 hours equilibration time and their matrices were made similar by diluting the donor side sample using blank PBS, and by diluting the receiver side sample using blank plasma/phosphate buffered saline. After the incubation, aliquots of donor side matrixes were diluted with an equal volume of the blank receiver side matrix and aliquots of receiver side matrixes were diluted with an equal volume of blank donor side matrix. All samples were protein precipitated by addition of a two-fold volume of acetonitrile containing 100 nM of repaglinide as an internal standard. After 10-minute centrifugation at 13

200 rpm, the sample supernatants were analysed with an LC-MS/MS, to obtain the unbound fraction of the test compound ($F_{ub}$). The unbound fraction was calculated from the peak area ratios obtained for each matrix:

$$F_{ub} = C_{PBS} / C_{plasma};$$

where $C_{PBS}$ and $C_{plasma}$ are the analyte concentrations in PBS (receiver) and plasma (donor), respectively.

Recovery samples were prepared in each condition but without dialysis, and were used for evaluation of recovery from the dialysis experiment using following formula:

$$\% \text{ Recovery} = 100 \times (V_{PBS} \times C_{PBS} + V_{plasma} \times C_{plasma}) / V_{plasma} \times C_{recovery}$$

where $V_{PBS}$ is the volume on the receiver side (PBS) and $V_{plasma}$ is the volume on donor side (plasma) of the dialysis device. $C_{recovery}$ is the analyte concentration measured from the recovery sample.

Propranolol (1μM) and fluoxetine (5μM) were included in the experiment as a control compounds.

The unbound fraction in brain ($F_{ub,\ brain}$) was calculated from the measured value in brain homogenate ($F_{ub,\ meas}$), taking into account the dilution factor used in preparing the brain homogenate:

$$F_{ub,\,brain} = \frac{1/D}{\left(\frac{1}{F_{ub,\,meas}}\right) - 1 + \left(\frac{1}{D}\right)}$$

where D = dilution factor (here 5).

**Analytical method**

Instrumentation: Waters Acquity UPLC + Waters Xevo TQ-XS triple quadrupole MS
Column: Waters Acquity HSS T3 (2.1x50mm, 1.8 μm) column with pre-column filter
Gradient Elution; A = 0.1% Formic acid, B = Acetonitrile

| Time (min) | Flow | A% | B% | Curve |
|---|---|---|---|---|
| 0.000 | 0.500 ml/min | 95 | 5 | - |
| 0.500 | 0.500 ml/min | 95 | 5 | 6 |
| 2.500 | 0.500 ml/min | 25 | 75 | 6 |
| 3.500 | 0.500 ml/min | 2 | 98 | 1 |
| 4.500 | 0.500 ml/min | 95 | 5 | 1 |

Temperature: 40 °C
Injection Volume: 1.5 μl
Ion Source: ESI+
Capillary voltage: 2400 V
Source temperature: 150 °C
Desolvation temperature: 650 °C
Cone gas flow: 240 L/hr
Desolvation gas flow: 1200 L/hr
Nebuliser gas flow: 7 Bar
Collision gas flow: 0.15 mL/min
Software: MassLynx 4.2

| Compound | MRM transition | Collision energy (V) | Cone (V) | Retention time |
|---|---|---|---|---|
| Example 3 | 306 > 117 | | | |

(continued)

| Compound | MRM transition | Collision energy (V) | Cone (V) | Retention time |
|---|---|---|---|---|
| | 325 > 92 | 32 | 46 | 2.11 |
| Comparative Example 1 | 325 > 109* | 16 | 46 | |
| | 325 > 217 | 26 | 64 | |
| Propranolol | 260 > 116* | 18 | 50 | 1.78 |
| | 260 > 155 | 23 | 50 | |
| | 260 > 183 | 25 | 50 | |
| Fluoxetine | 310 > 148* | 6 | 30 | 2.03 |
| * MRM trace used for quantification | | | | |

**Results of Equilibrium Dialysis**

The table below shows, for the mouse plasma, mouse brain, rat brain and dog plasma, the unbound fractions of compounds Example 3 and the Comparative Example 1 in plasma and brain homogenate.

| | Mouse Plasma | Mouse Brain | Rat Brain | Dog plasma |
|---|---|---|---|---|
| | Unbound (Fu, %) | Unbound (Fu, %) | Unbound (Fu, %) | Unbound (Fu, %) |
| Example 3 | | | 37.1 | 55.5 |
| Comparative Example 1 | 6.8 | 27.3 | | |

**Blood-brain-barrier permeability of study compounds in mice after IV administration**

The compound is administered to the animal in a suitable vehicle at T=0 hours. At 0.5 hours post administration plasma and separately brain are removed, prepared and analysed for total compound concentration.

**General sample processing procedure (Plasma):**

Protein precipitation (PPT) using 96-well plate

1). An aliquot of 5 μL unknown sample, calibration standard, quality control and dilution quality control (if have), single blank, and double blank sample was added to the 96-well plate respectively;

2). Each sample (except the double blank) was quenched with 200 μL of IS1 (6 in 1 internal standard in MeOH (Labetalol & tolbutamide & Verapamil & dexamethasone & glyburide & Celecoxib 100 ng/mL for each) respectively (double blank sample was quenched with 200 μL of MeOH), and then the mixture was vortex-mixed for 10 min at 800 rpm and centrifuged for 15 min at 3220 × g, 4 °C;

3). An aliquot of 60 μL supernatant was transferred to another clean 96-well plate and centrifuged for 5 min at 3220 × g, 4 °C, then the supernatant was directly injected for LC-MS/MS analysis.

**Dilution procedure description:**

1) Dilution factor as 10 : An aliquot of 2 μL unknown sample was added with 18 μL blank matrix;

**General sample processing procedure (Brain homogenate):**

Protein precipitation (PPT) using 96-well plate

1). An aliquot of 20 µL unknown sample, calibration standard, quality control and dilution quality control (if have), single blank, and double blank sample was added to the 96-well plate respectively;

2). Each sample (except the double blank) was quenched with 800 µL of IS1 respectively (double blank sample was quenched with 800 µL of MeOH), and then the mixture was vortex-mixed for 10 min at 800 rpm and centrifuged for 15 min at 3220 × g, 4 °C; 3). An aliquot of 60 µL supernatant was transferred to another clean 96-well plate and centrifuged for 5 min at 3220 × g, 4 °C, then the supernatant was directly injected for LC-MS/MS analysis.

**Analytical method**

Instrumentation: LC-MS/MS-CB_Triple Quad 6500 plus Column: Waters ACQUITY UPLC HSS T3 1.8 µm 2.1 × 50 mm
Gradient Elution; A = 0.1% Formic acid in water, B = 0.1% Formic acid in acetonitrile

| Time (min) | Flow | A (%) | B (%) |
|---|---|---|---|
| 0 | 0.6 mL/min | 95 | 5 |
| 1 | 0.6 mL/min | 30 | 70 |
| 1.2 | 0.6 mL/min | 0 | 100 |
| 1.5 | 0.6 mL/min | 0 | 100 |
| 1.51 | 0.6 mL/min | 95 | 5 |
| 1.6 | 0.6 mL/min | 95 | 5 |

Temperature: 45 °C
Injection Volume: 1.5 µl for brain, 4 µl for plasma
Ion Source: ESI+, SRM detection

| Compound | MRM transition | Retention time |
|---|---|---|
| Example 3 | 306 > 147 | 0,82 |
| Verapamil | 455 > 165 | 0,97 |

**Results**

Example 3 and comparative Example 1 were formulated to 1.50 mg/mL in 10% DMSO , 5% Tween80 , 40% PG , 45% saline pH=about 7 with pH strip, clear solution and administered intravenously to male CD1 (ICR) fed mice at 3 mg/kg.

The results are shown in the table below. Example 3 returned a plasma concentration of 292 ng/mL and a brain concentration of 61.2 ng/g at 0.5 hours after dosing. Meaning that brain to plasma concentration of 21% was observed. In contrast, comparative Example 1 returned a plasma concentration of 3087 ng/ml and a brain concentration of 5 ng/g at 0.5 hours. This means that the brain to plasma ratio is less than 1%.

For the treatment of CNS diseases it is advantageous to have a brain to plasma ratio value of more than 1% and preferably above 10 and 20%. Example 3 is therefore particularly useful for the treatment of CNS diseases.

| Compound | Mouse plasma concentration (0.5 h) (ng/mL) | Brain homogenate concentration (0.5h) (ng/g) | Brain to plasma ratio % |
|---|---|---|---|
| Example 3 | 292 | 61.2 | 21 |

(continued)

| Compound | Mouse plasma concentration (0.5 h) (ng/mL) | Brain homogenate concentration (0.5h) (ng/g) | Brain to plasma ratio % |
|---|---|---|---|
| Comparative example 1 | 3087 | 5 | <1 |

The unbound partition coefficient ($K_{puu}$) was determined as a ratio between the free compound concentrations in plasma and brain:

$$K_{puu} = \frac{C_{ub,brain}}{C_{ub,plasma}}$$

Where $C_{u,brain}$ = unbound concentration in brain ($C \times F_{ub}$, brain); wherein

C = concentration at steady state; and

$C_{ub,plasma}$ = unbound concentration in plasma ($C \times F_{ub}$).

The $K_{puu}$ of Comparative example 1 is thus calculated to be <0.1. This demonstrates that comparative example 1 does not effectively permeate through the blood brain barrier.

**$K_{puu}$ determination in the dog**

Example 3 was loaded as a solid, Batch No. C2210502, into enteric coated capsule (0#). Fasted male beagle dog was dosed with pentagastrin (0.25 mg/mL and 0.024 mL/kg) at 6 μg/kg by intramuscular (IM) injection at approximately 30 minutes prior to administration of test compound. Capsule containing 20 mg/kg of Example 3 were administered orally and plasma and CSF samples were taken pre-dose, 0.5, 1, 2, 4, 8 12 and 24 hours post dosing and analysed for concentration of Example 3 in analogous fashion to the study above.

**Results**

| Time (h) | Plasma concentration (ng/mL) | Time (h) | CSF concentration (ng/mL) |
|---|---|---|---|
| **0** | BQL* | **0** | BQL |
| **0,5** | 1,04 | **0,5** | BQL |
| **1** | 3,20 | **1** | BQL |
| **2** | 370 | **2** | 13,7 |
| **4** | 5350 | **4** | 745 |
| **8** | 1400 | **8** | 698 |
| **12** | 390 | **12** | 196 |
| **24** | 87,1 | **24** | 27,7 |
| *BQL: below quantification level | | | |

**Analytical data**

| | **$AUC_{0\text{-}inf}$ (ng.h/mL)** | %Fuplasma |
|---|---|---|
| Plasma | 23816 | 55 |
| CSF | 6414 | |

The unbound partition coefficient ($K_{puu}$) was determined as a ratio between the areas under the curve corrected for free compound concentrations in plasma and CSF.

$$K_{puu} = AUC0\text{-}infcsf / (AUC0\text{-}infplasma \times (\%Fuplasma/100))$$

$$K_{puu} = 6414/(23816 \times (55{,}5/100)) = 6414 / 13218 = \mathbf{0.49}$$

Example 3 therefore has a $K_{puu}$ of greater than 0.1, which indicates that a fraction of the unbound compound in the plasma permeates through the blood brain barrier. The compound is therefore particularly useful in the treatment of CNS diseases.

**REFERENCES**

Andersen, J et al., Identification of the first small-molecule ligand of the neuronal receptor sortilin and structure determination of the receptor-ligand complex. Acta Crystallogr D Biol Crystallogr (2014), 70(Pt 2), pp.451-460;

Baker, M.et al., Mutations in progranulin cause tau-negative frontotemporal dementia linked to chromosome 17. Nature (2006), 442(7105), pp. 916-919;

Brouwers, N.et al., Genetic variability in progranulin contributes to risk for clinically diagnosed Alzheimer disease. Neurology, (2008), 71(9), pp. 656-664;

Buttenshon, H.N. et al., Increased serum levels of sortilin are associated with depression and correlated with BDNF and VEGF, Nature Translational Psychiatry (2015), 5(e677), pp. 1-7;

Carecchio, M.,et al., Cerebrospinal fluid biomarkers in Progranulin mutations carriers. J Alzheimers Dis (2011), 27(4), pp. 781-790;

Carrasquillo, M. et al.,. Genome-wide screen identifies rs646776 near sortilin as a regulator of progranulin levels in human plasma. Am J Hum Genet (2010), 87(6), pp. 890-897;

Chen, Z. Y.et al., Sortilin controls intracellular sorting of brain-derived neurotrophic factor to the regulated secretory pathway. J Neurosci (2005), 25(26), pp. 6156-6166;

Cruts, M. et al., Loss of progranulin function in frontotemporal lobar degeneration. Trends Genet (2008), 24(4), pp. 186-194;

De Muynck, L. et al., The neurotrophic properties of progranulin depend on the granulin E domain but do not require sortilin binding. Neurobiol Aging (2013), 34(11), pp. 2541-2547;

Egashira, Y. et al.,The growth factor progranulin attenuates neuronal injury induced by cerebral ischemia-reperfusion through the suppression of neutrophil recruitment. J Neuroinflammation (2013), 10, pp. 105;

Galimberti, D. et al.,. GRN variability contributes to sporadic frontotemporal lobar degeneration. J Alzheimers Dis (2010), 19(1), pp. 171-177;

Galimberti, D.et al.,. Progranulin as a therapeutic target for dementia. Expert Opin Ther Targets (2018), 22(7), pp. 579-585. doi:10.1080/14728222.2018.1487951

Gao, A. et al., Implications of Sortilin in Lipid Metabolism and Lipid Disorder Diseases. DNA and Cell Biology (2017), 36(12), pp.1050-1061;

Gass, J. et al., Progranulin regulates neuronal outgrowth independent of sortilin. Mol Neurodegener (2012), 7, pp. 33;

Gass, J. et al., Progranulin: an emerging target for FTLD therapies. Brain Res (2012), 1462, pp. 118-128;

Gijselinck, I.,et al., Granulin mutations associated with frontotemporal lobar degeneration and related disorders: an update. Hum Mutat (2008), 29(12), pp. 1373-1386;

Goettsch, C., et al., Sortilin and Its Multiple Roles in Cardiovascular and Metabolic Diseases. Atherosclerosis, Thrombosis and Vascular Biology (2017), 38(1), pp. 19-25

Jansen, P., et al., Roles for the pro-neurotrophin receptor sortilin in neuronal development, aging and brain injury. Nature Neuroscience (2007), 10(11), pp.1449-1457;

Hu, F. et al., Sortilin-mediated endocytosis determines levels of the frontotemporal dementia protein, progranulin. Neuron (2010), 68(4), pp. 654-667;

Huang, G. et al., Insulin responsiveness of glucose transporter 4 in 3T3-L1 cells depends on the presence of sortilin. Mol Biol Cell (2013), 24(19), pp.3115-3122;

Kaddai, V. et al. Involvement of TNF-$\alpha$ in abnormal adipocyte and muscle sortilin expression in obese mice and humans. Diabetologia (2009) 52, pp. 932-940;

Kjolby, M.et al., Sort1, encoded by the cardiovascular risk locus 1p13.3, is a regulator of hepatic lipoprotein export.

Cell Metab (2010), 12(3), pp. 213-223;
Laird, A. S. et al., Progranulin is neurotrophic in vivo and protects against a mutant TDP-43 induced axonopathy. PLoS One (2010), 5(10), e13368;
Lee, W. et al., Targeted manipulation of the sortilin-progranulin axis rescues progranulin haploinsufficiency. Hum Mol Genet (2014), 23(6), pp. 1467-1478;
Martens, L.et al., Progranulin deficiency promotes neuroinflammation and neuron loss following toxin-induced injury. J Clin Invest (2012), 122(11), pp. 3955-3959;
Mazella, J. et al., The 100-kDa neurotensin receptor is gp95/sortilin, a non-G-protein-coupled receptor. J Biol Chem (1998), 273(41), pp. 26273-26276;
Miyakawa, S. et al, Anti-sortilin1 Antibody Up-Regulates Progranulin via Sortilin1 Down-Regulation. Front Neurosci (2020), 14, pp. 586107;
Møller et al. Sortilin as a Biomarker for Cardiovascular Disease Revisited. Frontiers in Cardiovascular Medicine (2021), 8, 652584;
Mortensen, M.B. et al., Targeting sortilin in immune cells reduces proinflammatory cytokines and atherosclerosis. J Clin Invest (2014), 124(12), pp. 5317-5322;
Nykjaer, A et al., Sortilin is essential for proNGF-induced neuronal cell death. Nature (2014), 427(6977), pp. 843-848;
Nykjaer, A., & Willnow, T. E, Sortilin: a receptor to regulate neuronal viability and function. Trends Neurosci (2012), 35(4), pp. 261-270.
Oh, T.J. et al., Circulating sortilin level as a potential biomarker for coronary atherosclerosis and diabetes mellitus. Cardiovascular Diabetology (2017), 16(92);
Pan, X. et al., Sortilin and retromer mediate retrograde transport of Glut4 in 3T3-L1 adipocytes. Mol Biol Cell (2017), 28(12), pp.1667-1675;
Petersen, C. et al., Molecular identification of a novel candidate sorting receptor purified from human brain by receptor-associated protein affinity chromatography. J Biol Chem (1997), 272(6), pp. 3599-3605;
Pickford, F.et al., Progranulin is a chemoattractant for microglia and stimulates their endocytic activity. Am J Pathol (2011), 178(1), pp. 284-295;
Pottier, C., et al., Potential genetic modifiers of disease risk and age at onset in patients with frontotemporal lobar degeneration and GRN mutations: a genome-wide association study. Lancet Neurol (2018), 17(6), pp. 548-558;
Quistgaard, E. et al., Ligands bind to Sortilin in the tunnel of a ten-bladed beta-propeller domain. Nat Struct Mol Biol (2009), 16(1), pp. 96-98;
Santos, A. M. et al., Sortilin Participates in Light-dependent Photoreceptor Degeneration in Vivo. PLoS ONE (2012), 7(4), pp. e36243-e36243.16. Kuruvilla, R. et al., A neurotrophin signaling cascade coordinates sympathetic neuron development through differential control of TrkA trafficking and retrograde signalling. Cell (2004), 118(2), pp. 243-255;
Shi, J. & Kandror, K. V., Sortilin Is Essential and Sufficient for the Formation of Glut4 Storage Vesicles in 3T3-L1 Adipocytes. Developmental Cell (2005), 9, pp. 99-108;
Schroder, T. et al., The identification of AF38469: an orally bioavailable inhibitor of the VPS10P family sorting receptor Sortilin. Bioorg Med Chem Lett (2014), 24(1), pp. 177-180;
Sheng, J. et al.,Progranulin polymorphism rs5848 is associated with increased risk of Alzheimer's disease. Gene (2014), 542(2), pp. 141-145;Skeldal, S. et al., Mapping of the Interaction Site between Sortilin and the p75 Neurotrophin Receptor Reveals a Regulatory Role for the Sortilin Intracellular Domain in p75 Neurotrophin Receptor Shedding and Apoptosis. J Biol Chem (2012), 21(287), pp. 43798-43809;
Tauris, J., et al., Proneurotrophin-3 May Induce Sortilin-Dependent Death In Inner Ear Neurons. Eur J Neuroscience (2020), 33(4), pp.622-31;
Tang, W. et al., The growth factor progranulin binds to TNF receptors and is therapeutic against inflammatory arthritis in mice. Science (2011), 332(6028), pp. 478-484;
Tao, J.et al., Neuroprotective effects of progranulin in ischemic mice. Brain Res (2012), 1436, pp. 130-136;
Tenk, H.K., et al., ProBDNF induces neuronal apoptosis via activation of a receptor complex of p75NTR and sortilin. J Neuroscience (2005), 10(11), pp.1449-1457
Van Kampen, J. M.,et al., Progranulin gene delivery protects dopaminergic neurons in a mouse model of Parkinson's disease. PLoS One (2014), 9(5), e97032;
Willnow, T. E.et al., VPS10P-domain receptors - regulators of neuronal viability and function. Nat Rev Neurosci (2008), 9(12), pp. 899-909;
Willnow, T.E., et al., Sortilins: new players in lipoprotein metabolism. Current Opinion in Lipidology (2011), 22(2), pp. 79-85.
Wuts, P.G.M. and Greene, T.W, Greene's Protective Groups in Organic Synthesis, 4th Edition, John Wiley and Sons, New York (2006);
Xu, S.H. et al., Regional and Cellular Mapping of Sortilin Immunoreactivity in Adult Human Brain, Frotiers in Neuroanatomy (2019), 13(31), pp. 1-27;

Yano, H., et al., Proneurotrophin-3 is a neuronal apoptotic ligand: evidence for retrograde-directed cell killing. J Neurosci (2009), 29(47), pp. 14790-14802;
Yin, F., et al., Exaggerated inflammation, impaired host defense, and neuropathology in progranulin-deficient mice. J Exp Med (2010), 207(1), pp. 117-128;
Zheng, Y., et al., C-terminus of progranulin interacts with the beta-propeller region of sortilin to regulate progranulin trafficking. PLoS One (2011), 6(6), e21023;
Zhou, X. et al., Prosaposin facilitates sortilin-independent lysosomal trafficking of progranulin. J Cell Biol (2015), 210(6), pp. 991-1002;
Meneses et al., TDP-43 Pathology in Alzheimer's Disease, Mol Neurodegeneration (2021), 16, 84;
Prudencio et al., Misregulation of human sortilin splicing leads to the generation of a nonfunctional progranulin receptor, Proc Natl Acad Sci U S A (2012), 109(52): 21510-21515;
Beel et al., Progranulin reduces insoluble TDP-43 levels, slows down axonal degeneration and prolongs survival in mutant TDP-43 mice, Mol Neurodegener. (2018), 13: 55.

**Sequences referenced throughout the specification and forming part of the description**

SEQ ID NO: 1 (full length sortilin- isoform 1)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCKAD LGALELWRTS DLGKSFKTIG
301 VKIYSFGLGG RFLFASVMAD KDTTRRIHVS TDQGDTWSMA QLPSVGQEQF
351 YSILAANDDM VFMHVDEPGD TGFGTIFTSD DRGIVYSKSL DRHLYTTTGG
401 ETDFTNVTSL RGVYITSVLS EDNSIQTMIT FDQGGRWTHL RKPENSECDA
451 TAKNKNECSL HIHASYSISQ KLNVPMAPLS EPNAVGIVIA HGSVGDAISV
501 MVPDVYISDD GGYSWTKMLE GPHYYTILDS GGIIVAIEHS SRPINVIKFS
551 TDEGQCWQTY TFTRDPIYFT GLASEPGARS MNISIWGFTE SFLTSQWVSY
601 TIDFKDILER NCEEKDYTIW LAHSTDPEDY EDGCILGYKE QFLRLRKSSM
651 CQNGRDYVVT KQPSICLCSL EDFLCDFGYY RPENDSKCVE QPELKGHDLE
701 FCLYGREEHL TTNGYRKIPG DKCQGGVNPV REVKDLKKKC TSNFLSPEKQ
751 NSKSNSVPII LAIVGLMLVT VVAGVLIVKK YVCGGRFLVH RYSVLQQHAE
801 ANGVDGVDAL DTASHTNKSG YHDDSDEDLL E

SEQ ID NO: 2 (full length sortilin- isoform 2)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT

601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE

SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI
361 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

SEQ ID NO: 4 (Murine Sortilin)

>sp|Q6PHU5|SORT_MOUSE Sortilin OS=Mus musculus OX=10090 GN=Sort1 PE=1 SV=1

MERPRGAADGLLRWPLGLLLLLQLLPPAAVGQDRLDAPPPPAPPLLRWAGPVGVSWGLRA

AAPGGPVPRAGRWRRGAPAEDQDCGRLPDFIAKLTNNTHQHVFDDLSGSVSLSWVGDST
G

VILVLTTFQVPLVIVSFGQSKLYRSEDYGKNFKDITNLINNTFIRTEFGMAIGPENSGKV

ILTAEVSGGSRGGRVFRSSDFAKNFVQTDLPFHPLTQMMYSPQNSDYLLALSTENGLWVS

KNFGEKWEEIHKAVCLAKWGPNNIIFFTTHVNGSCKADLGALELWRTSDLGKTFKTIGVK

IYSFGLGGRFLFASVMADKDTTRRIHVSTDQGDTWSMAQLPSVGQEQFYSILAANEDMVF

MHVDEPGDTGFGTIFTSDDRGIVYSKSLDRHLYTTTGGETDFTNVTSLRGVYITSTLSED

NSIQSMITFDQGGRWEHLRKPENSKCDATAKNKNECSLHIHASYSISQKLNVPMAPLSEP

NAVGIVIAHGSVGDAISVMVPDVYISDDGGYSWAKMLEGPHYYTILDSGGIIVAIEHSNR

PINVIKFSTDEGQCWQSYVFTQEPIYFTGLASEPGARSMNISIWGFTESFITRQWVSYTV

DFKDILERNCEEDDYTTWLAHSTDPGDYKDGCILGYKEQFLRLRKSSVCQNGRDYVVAKQ

PSVCPCSLEDFLCDFGYFRPENASECVEQPELKGHELEFCLYGKEEHLTTNGYRKIPGDK

CQGGMNPAREVKDLKKKCTSNFLNPTKQNSKSNSVPIILAIVGLMLVTVVAGVLIVKKYV

CGGRFLVHRYSVLQQHAEADGVEALDSTSHAKSGYHDDSDEDLLE

## Claims

1. A compound of formula (I)

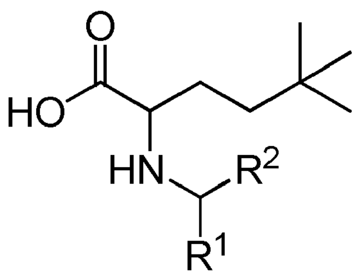

(I)

or a pharmaceutically acceptable salt, solvate, hydrate, tautomer, optical isomer, N-oxide, and/or prodrug thereof; wherein

$R^1$ is

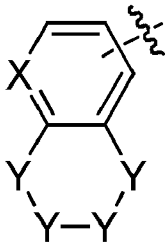 or 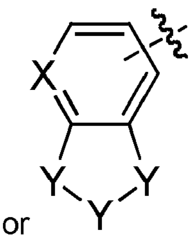 ;

X is $CR^3$ or N, wherein $R^3$ is selected from the group consisting of H, halo, and C1-C3 alkyl;
each Y is independently $CHR^4$, $NR^5$, $CR^6R^6$, or O, wherein $R^4$ is independently selected from the group consisting of H, halo, oxo, and $C_1$-$C_4$ alkyl; $R^5$ is independently selected from the group consisting of H, halo, $C_1$-$C_4$ alkyl, $C_2$-$C_4$hydroxyalkyl, -($C_2$-$C_4$ alkyl)-O-($C_1$-$C_4$ alkyl), -C(O)-($C_1$-$C_4$ alkyl), and -C(O)O-($C_1$-$C_4$ alkyl); and $R^6$ is independently selected from the group consisting of halo and $C_1$-$C_4$ alkyl;
$R^2$ is selected from the group consisting of: H, $C_1$-$C_4$ alkyl, $C_2$-$C_4$ hydroxyalkyl and $C_1$-$C_3$ haloalkyl;
wherein the compound is not one of the following compounds:

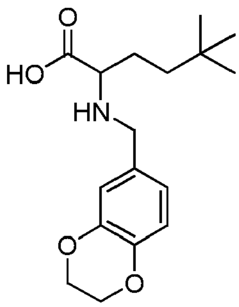 , 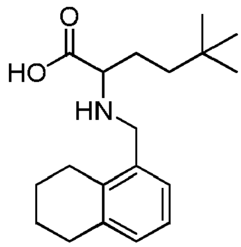 , 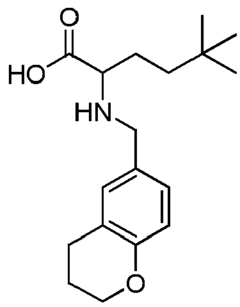 , or 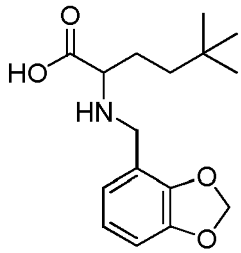 .

**2.** The compound according to claim 1, wherein $R^2$ is selected from the group consisting of H, $CH_3$, $CH_2F$, $CHF_2$, and $CF_3$, preferably wherein $R^2$ is selected from the group consisting of H, $CH_3$, $CHF_2$, and $CF_3$.

**3.** The compound according to claim 1 or 2, wherein each Y is independently $CHR^4$, $NR^5$, or O; and/or

wherein $R^3$ is H or $C_1$-$C_3$ alkyl, preferably H or methyl; and/or
wherein each $R^4$ is independently H or oxo; and/or
wherein each $R^5$ is independently H, $C_1$-$C_3$ alkyl, or -C(O)O-($C_1$-$C_4$ alkyl), preferably H, methyl, or -C(O)O-(tert-butyl), more preferably H or methyl.

**4.** The compound according to any preceding claim, wherein $R^1$ is selected from one of the following groups:

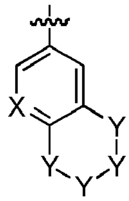 , 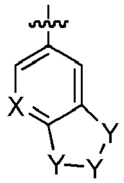 , 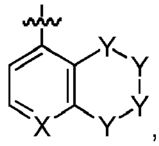 , and 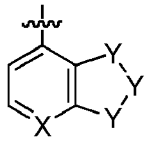 ,

preferably wherein no more than one or two Y are $NR^5$ or O and the remaining Y are $CHR^4$.

**5.** The compound according to claim 4, wherein $R^1$ is selected from one of the following groups:

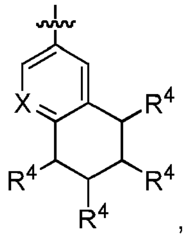 , 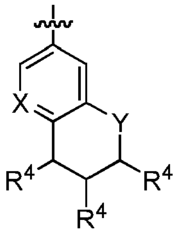 , 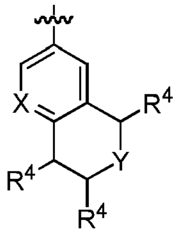 , 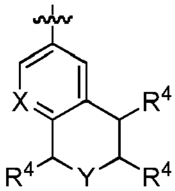 , 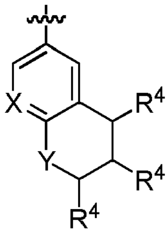 , 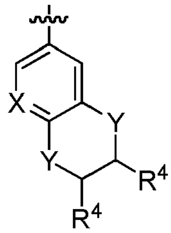 ,

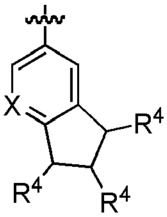 , 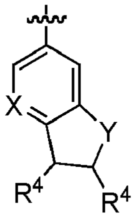 , 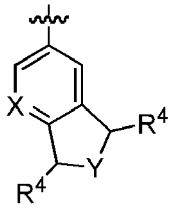 , 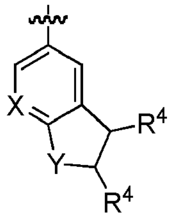 , 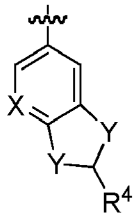 , 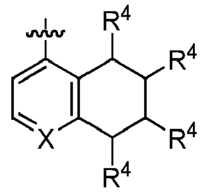 ,

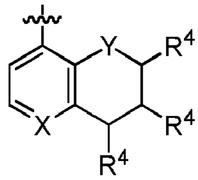 , 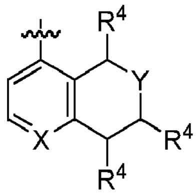 , 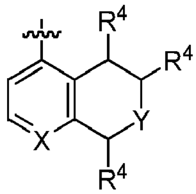 , 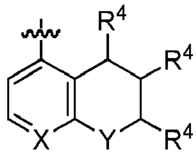 , 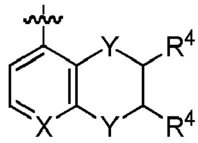 ,

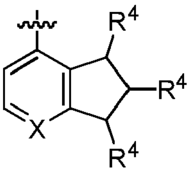 , 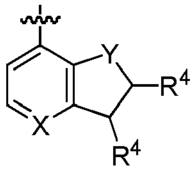 , 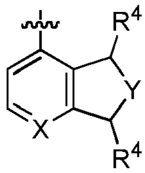 , 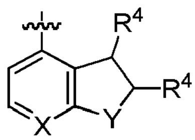 , and 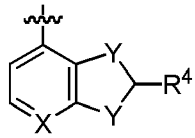 ,

wherein each Y is independently $NR^5$ or O, preferably wherein one $R^4$ group is H or oxo and the other $R^4$ groups are all H.

**6.** The compound according to claim 5, wherein $R^1$ is selected from one of the following groups:

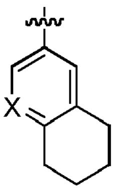 , 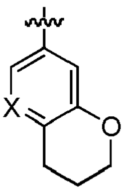 , 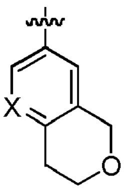 , 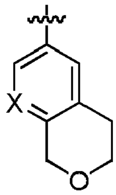 , 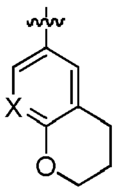 , 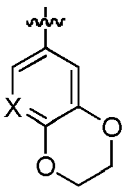 , 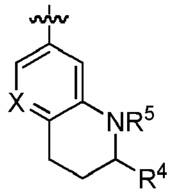 ,

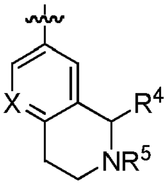 , 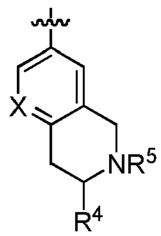 , 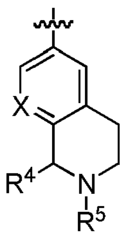 , 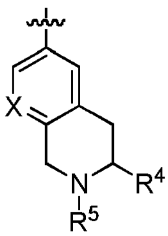 , 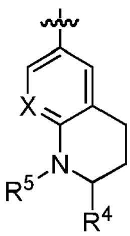 , 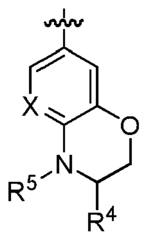 ,

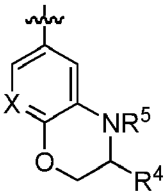, 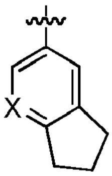, 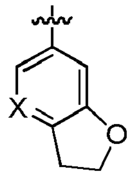, 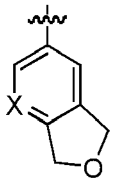, 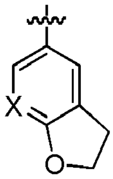, 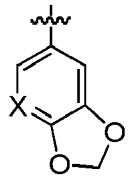, 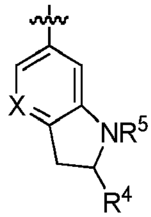,

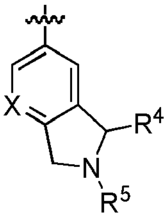, 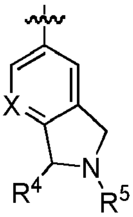, 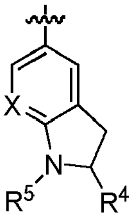, 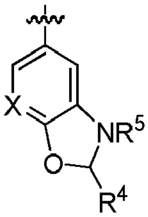, 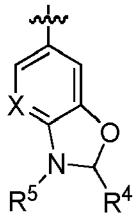, 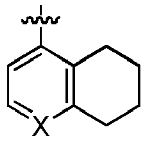, 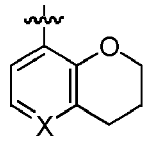,

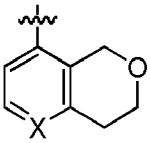, 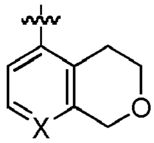, 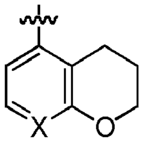, 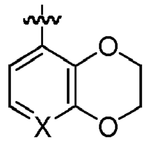, 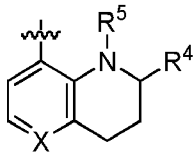, 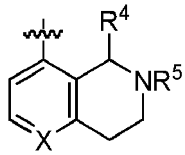,

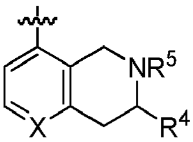, 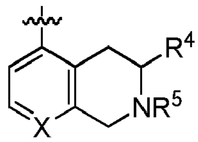, 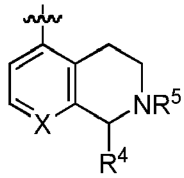, 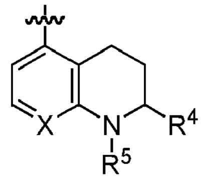, 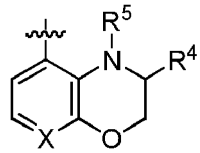,

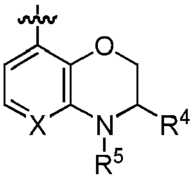, 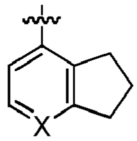, 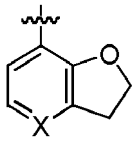, 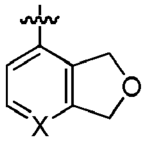, 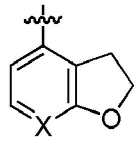, 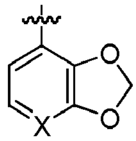,

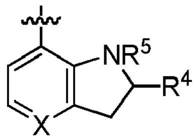, 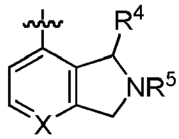, 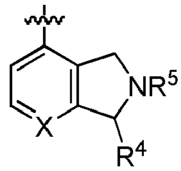, 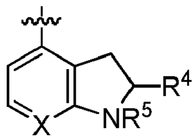, 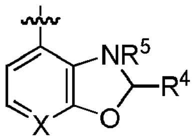,

and

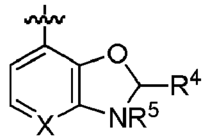.

7. The compound according to claim 6, wherein $R^1$ is selected from one of the following groups:

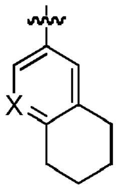, 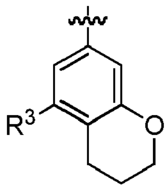, 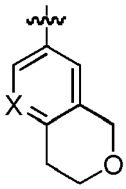, 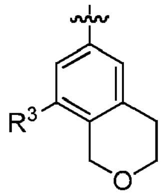, 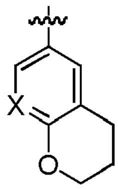, 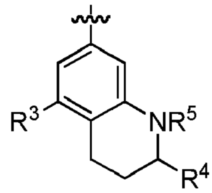,

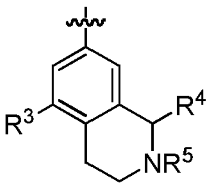, 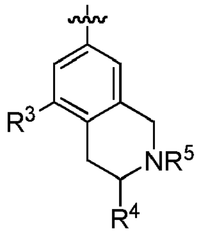, 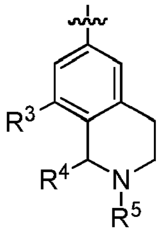, 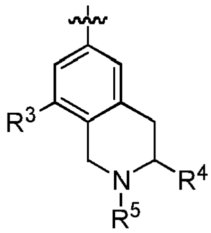, 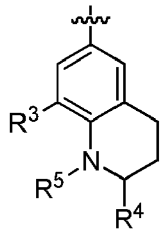, 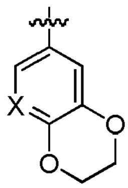

, 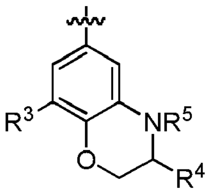, 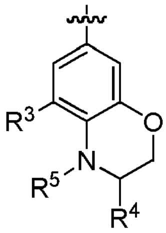, 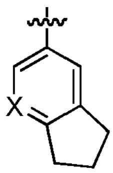, 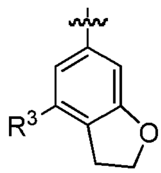, 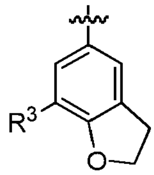, 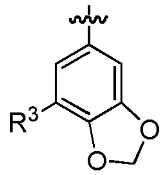,

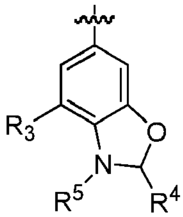, 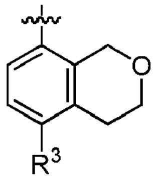, 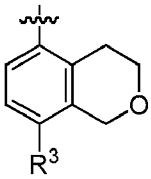, 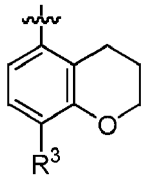, 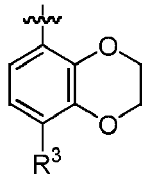, 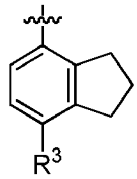,

and

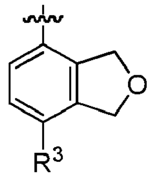.

**8.** The compound according to claim 7, wherein $R^1$ is selected from one of the following groups:

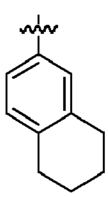, 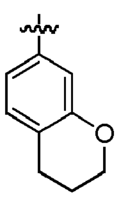, 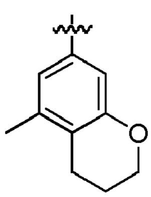, 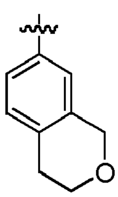, 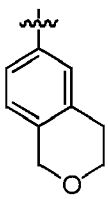, 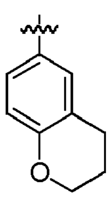, 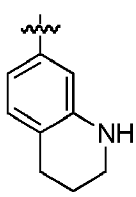, 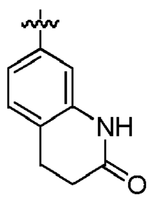,

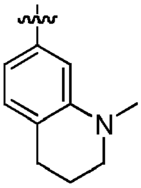, 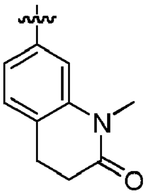, 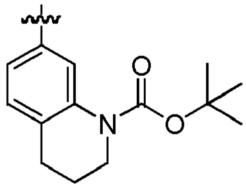, 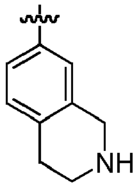, 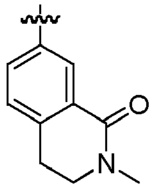, 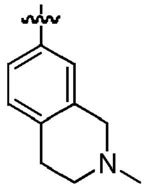,

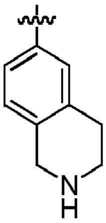, 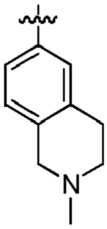, 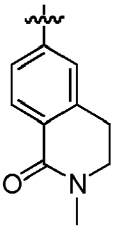, 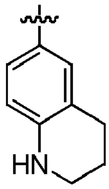, 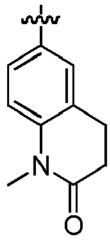, 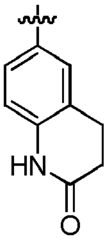, 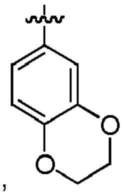, 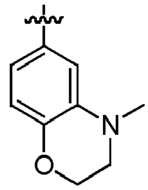,

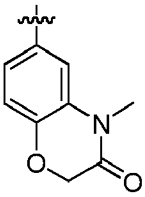, 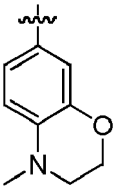, 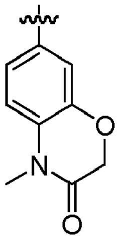, 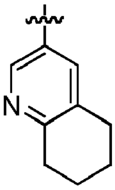, 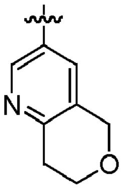, 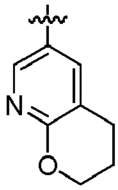, 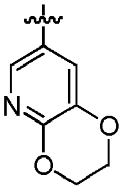, 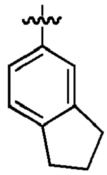,

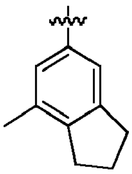, 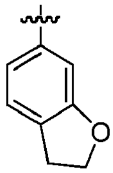, 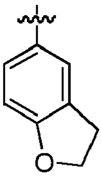, 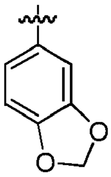, 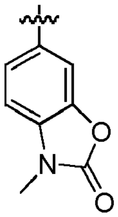, 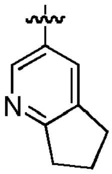, 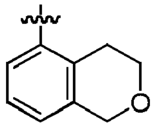, 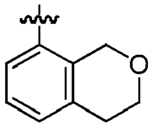,

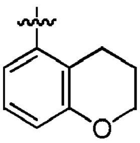, 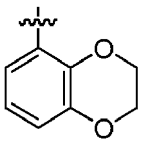, 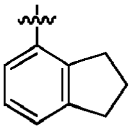, and 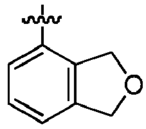.

**9.** The compound according to any preceding claim, wherein the compound is:

(2S)-2-{[(1S)-2,2-difluoro-1-(5,6,7,8-tetrahydronaphthalen-2-yl)ethyl]amino}-5,5-dimethylhexanoic acid;
(2S)-2-{[(1S)-1-(3,4-dihydro-2H-1-benzopyran-7-yl)-2,2-difluoroethyl]amino}-5,5-dimethylhexanoic acid;
(2S)-5,5-dimethyl-2-{[(1R)-1-(5,6,7,8-tetrahydronaphthalen-2-yl)ethyl]amino}hexanoic acid;
(2S)-5,5-dimethyl-2-{[(5,6,7,8-tetrahydronaphthalen-2-yl)methyl]amino}hexanoic acid;
(2S)-2-[({1-[(tert-butoxy)carbonyl]-1,2,3,4-tetrahydroquinolin-7-yl}methyl)amino]-5,5-dimethylhexanoic acid;
(2S)-2-{[(3,4-dihydro-2H-1-benzopyran-7-yl)methyl]amino}-5,5-dimethylhexanoic acid;
(2S)-5,5-dimethyl-2-{[(1S)-2,2,2-trifluoro-1-(5,6,7,8-tetrahydroquinolin-3-yl)ethyl]amino}hexanoic acid;
(2S)-5,5-dimethyl-2-{[(5,6,7,8-tetrahydroquinolin-3-yl)methyl]amino}hexanoic acid;
(2S)-5,5-dimethyl-2-{[(1S)-2,2,2-trifluoro-1-(5,6,7,8-tetrahydronaphthalen-2-yl)ethyl]amino}hexanoic acid;
(2S)-5,5-dimethyl-2-{[(1,2,3,4-tetrahydroquinolin-7-yl)methyl]amino}hexanoic acid;
(2S)-2-{[(1R)-1-(3,4-dihydro-2H-1-benzopyran-7-yl)ethyl]amino}-5,5-dimethylhexanoic acid;
(2S)-5,5-dimethyl-2-{[(1R)-1-(1-methyl-1,2,3,4-tetrahydroquinolin-7-yl)ethyl]amino}hexanoic acid;
(2S)-2-{[(1S)-1-(2,3-dihydro-1H-inden-5-yl)-2,2,2-trifluoroethyl]amino}-5,5-dimethylhexanoic acid;
(2S)-2-{[(1R)-1-(2,3-dihydro-1H-inden-5-yl)ethyl]amino}-5,5-dimethylhexanoic acid;
(2S)-2-{[(1S)-1-(3,4-dihydro-2H-1-benzopyran-6-yl)ethyl]amino}-5,5-dimethylhexanoic acid;

(2S)-2-[({5H,6H,7H-cyclopenta[b]pyridin-3-yl}methyl)amino]-5,5-dimethylhexanoic acid;
(2S)-2-{[(2,3-dihydro-1H-inden-5-yl)methyl]amino}-5,5-dimethylhexanoic acid;
(2S)-5,5-dimethyl-2-{[(4-methyl-3-oxo-3,4-dihydro-2H-1,4-benzoxazin-6-yl)methyl]amino}hexanoic acid;
(2S)-2-{[(3,4-dihydro-1H-2-benzopyran-6-yl)methyl]amino}-5,5-dimethylhexanoic acid;
(2S)-5,5-dimethyl-2-{[(1-methyl-2-oxo-1,2,3,4-tetrahydroquinolin-7-yl)methyl]amino}hexanoic acid;
(2S)-5,5-dimethyl-2-[({5H,7H,8H-pyrano[4,3-b]pyridin-3-yl}methyl)amino]hexanoic acid;
(2S)-2-{[(3,4-dihydro-1H-2-benzopyran-7-yl)methyl]amino}-5,5-dimethylhexanoic acid;
(2S)-2-{[(1R)-1-(2,3-dihydro-1,4-benzodioxin-6-yl)ethyl]amino}-5,5-dimethylhexanoic acid;
(2S)-2-{[(1S)-1-(2,3-dihydro-1H-inden-5-yl)ethyl]amino}-5,5-dimethylhexanoic acid;
(2S)-2-{[(1R)-1-(3,4-dihydro-2H-1-benzopyran-6-yl)ethyl]amino}-5,5-dimethylhexanoic acid;
(2S)-5,5-dimethyl-2-[({2H,3H,4H-pyrano[2,3-b]pyridin-6-yl}methyl)amino]hexanoic acid;
(2S)-2-{[(1R)-1-(2H-1,3-benzodioxol-5-yl)ethyl]amino}-5,5-dimethylhexanoicacid;
(2S)-5,5-dimethyl-2-{[(5-methyl-3,4-dihydro-2H-1-benzopyran-7-yl)methyl]amino}hexanoic acid;
(2S)-2-{[(1R)-1-(3,4-dihydro-2H-1-benzopyran-7-yl)-2,2-difluoroethyl]amino}-5,5-dimethylhexanoic acid;
(2S)-5,5-dimethyl-2-{[(2-oxo-1,2,3,4-tetrahydroquinolin-7-yl)methyl]amino}hexanoic acid;
(2S)-2-{[(1S)-1-(3,4-dihydro-2H-1-benzopyran-7-yl)ethyl]amino}-5,5-dimethylhexanoic acid;
(2S)-5,5-dimethyl-2-{[(1S)-1-(5,6,7,8-tetrahydronaphthalen-2-yl)ethyl]amino}hexanoic acid;
(2S)-5,5-dimethyl-2-{[(1R)-1-(1,2,3,4-tetrahydroquinolin-7-yl)ethyl]amino}hexanoic acid;
(2S)-2-{[(1S)-1-(3,4-dihydro-1H-2-benzopyran-6-yl)ethyl]amino}-5,5-dimethylhexanoic acid;
(2S)-2-[({2H,3H-[1,4]dioxino[2,3-b]pyridin-7-yl}methyl)amino]-5,5-dimethylhexanoic acid;
(2S)-2-{[(1S)-1-(2,3-dihydro-1,4-benzodioxin-6-yl)ethyl]amino}-5,5-dimethylhexanoic acid;
(2S)-5,5-dimethyl-2-{[(4-methyl-3,4-dihydro-2H-1,4-benzoxazin-6-yl)methyl]amino}hexanoic acid;
(2S)-5,5-dimethyl-2-{[(2-methyl-1-oxo-1,2,3,4-tetrahydroisoquinolin-7-yl)methyl]amino}hexanoic acid;
(2S)-5,5-dimethyl-2-{[(2-methyl-1,2,3,4-tetrahydroisoquinolin-7-yl)methyl]amino}hexanoic acid;
(2S)-2-{[(2,3-dihydro-1-benzofuran-6-yl)methyl]amino}-5,5-dimethylhexanoic acid;
(2S)-5,5-dimethyl-2-{[(7-methyl-2,3-dihydro-1H-inden-5-yl)methyl]amino}hexanoic acid;
(2S)-2-{[(3,4-dihydro-1H-2-benzopyran-5-yl)methyl]amino}-5,5-dimethylhexanoic acid;
(2S)-2-{[(1 R)-1-(3,4-dihydro-1H-2-benzopyran-7-yl)ethyl]amino}-5,5-dimethylhexanoic acid;
(2S)-2-{[(1,3-dihydro-2-benzofuran-4-yl)methyl]amino}-5,5-dimethylhexanoic acid;
(2S)-2-{[(2,3-dihydro-1-benzofuran-5-yl)methyl]amino}-5,5-dimethylhexanoic acid;
(2S)-2-{[(3,4-dihydro-1H-2-benzopyran-8-yl)methyl]amino}-5,5-dimethylhexanoic acid;
(2S)-2-{[(2H-1,3-benzodioxol-5-yl)methyl]amino}-5,5-dimethylhexanoic acid;
(2S)-5,5-dimethyl-2-{[(3-methyl-2-oxo-2,3-dihydro-1,3-benzoxazol-6-yl)methyl]amino}hexanoic acid;
(2S)-2-{[(1S)-1-(3,4-dihydro-1H-2-benzopyran-7-yl)ethyl]amino}-5,5-dimethylhexanoic acid;
(2S)-5,5-dimethyl-2-{[(1,2,3,4-tetrahydroisoquinolin-7-yl)methyl]amino}hexanoic acid;
(2S)-5,5-dimethyl-2-{[(2-methyl-1-oxo-1,2,3,4-tetrahydroisoquinolin-6-yl)methyl]amino}hexanoic acid;
(2S)-2-{[(1S)-1-(2H-1,3-benzodioxol-5-yl)ethyl]amino}-5,5-dimethylhexanoic acid;
(2S)-5,5-dimethyl-2-{[(1,2,3,4-tetrahydroquinolin-6-yl)methyl]amino}hexanoic acid;
(2S)-2-{[(1R)-2,2-difluoro-1-(5,6,7,8-tetrahydronaphthalen-2-yl)ethyl]amino}-5,5-dimethylhexanoic acid;
(2S)-2-{[(3,4-dihydro-2H-1-benzopyran-5-yl)methyl]amino}-5,5-dimethylhexanoic acid;
(2S)-5,5-dimethyl-2-{[(2-methyl-1,2,3,4-tetrahydroisoquinolin-6-yl)methyl]amino}hexanoic acid;
(2S)-2-{[(1S)-1-(2,3-dihydro-1,4-benzodioxin-5-yl)ethyl]amino}-5,5-dimethylhexanoic acid;
(2S)-5,5-dimethyl-2-{[(4-methyl-3,4-dihydro-2H-1,4-benzoxazin-7-yl)methyl]amino}hexanoic acid;
(2S)-2-{[(2,3-dihydro-1H-inden-4-yl)methyl]amino}-5,5-dimethylhexanoic acid;
(2S)-2-{[(1R)-1-(2,3-dihydro-1,4-benzodioxin-5-yl)ethyl]amino}-5,5-dimethylhexanoic acid;
(2S)-5,5-dimethyl-2-{[(1-methyl-2-oxo-1,2,3,4-tetrahydroquinolin-6-yl)methyl]amino}hexanoic acid;
(2S)-5,5-dimethyl-2-{[(2-oxo-1,2,3,4-tetrahydroquinolin-6-yl)methyl]amino}hexanoic acid;
(2S)-5,5-dimethyl-2-{[(1,2,3,4-tetrahydroisoquinolin-6-yl)methyl]amino}hexanoic acid;
(2S)-5,5-dimethyl-2-{[(4-methyl-3-oxo-3,4-dihydro-2H-1,4-benzoxazin-7-yl)methyl]amino}hexanoic acid;
or a pharmaceutically acceptable salt, solvate, hydrate, tautomer, optical isomer, N-oxide, and/or prodrug thereof.

**10.** A compound of formula (II)

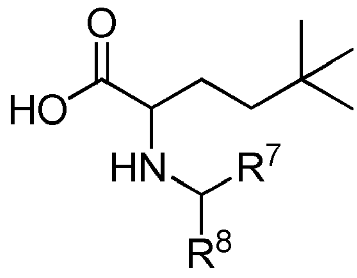

(II)

or a pharmaceutically acceptable salt, solvate, hydrate, tautomer, optical isomer, N-oxide, and/or prodrug thereof; wherein

$R^7$ is selected from the group consisting of H, $C_1$-$C_3$ alkyl, and $C_1$-$C_3$ haloalkyl, preferably H, $CH_3$, $CHF_2$, and $CF_3$, preferably H and $CH_3$; and $R^8$ is phenyl or pyridine, wherein phenyl and pyridine are independently substituted with one or more substituents selected from the group consisting of 5-membered heterocycloalkyl, triazolyl, -O-phenyl, and $-NR^9R^{10}$, and $R^9$ and $R^{10}$ are independently selected from H or $C_1$-$C_3$ alkyl, preferably H or $CH_3$, more preferably $CH_3$; or
$R^7$ is $C_1$-$C_3$ hydroxyalkyl, preferably $-(C_2H_4)$-OH; and $R^8$ is phenyl optionally substituted with $C_1$-$C_3$ alkoxy, preferably phenyl is optionally substituted with $-O-CH_3$.

**11.** The compound according to claim 10, wherein $R^7$ is selected from the group consisting of H, $C_1$-$C_3$ alkyl, and $C_1$-$C_3$ haloalkyl, and $R^8$ is phenyl substituted with pyrrolidinyl, triazolyl, -O-phenyl, or $-NR^9R^{10}$, or $R^8$ is pyridine substituted with -O-phenyl; or
wherein $R^7$ is $C_1$-$C_3$ hydroxyalkyl and $R^8$ is phenyl optionally substituted with $C_1$-$C_3$ alkoxy.

**12.** The compound according to claim 10 or 11, wherein the compound is

(2S)-5,5-dimethyl-2-{[(2-phenoxypyridin-4-yl)methyl]amino}hexanoic acid;
(2S)-5,5-dimethyl-2-{[(6-phenoxypyridin-3-yl)methyl]amino}hexanoic acid;
(2S)-5,5-dimethyl-2-({[3-(pyrrolidin-1-yl)phenyl]methyl}amino)hexanoic acid;
2-{[(1S)-3-hydroxy-1-phenylpropyl]amino}-5,5-dimethylhexanoic acid;
(2S)-5,5-dimethyl-2-{[(1R)-1-(6-phenoxypyridin-3-yl)ethyl]amino}hexanoic acid;
(2S)-5,5-dimethyl-2-{[(1S)-1-(6-phenoxypyridin-3-yl)ethyl]amino}hexanoic acid;
(2S)-5,5-dimethyl-2-{[(4-phenoxyphenyl)methyl]amino}hexanoic acid;
(2S)-2-({[3-(dimethylamino)phenyl]methyl}amino)-5,5-dimethylhexanoic acid;
(2S)-2-{[(1R)-3-hydroxy-1-(3-methoxyphenyl)propyl]amino}-5,5-dimethylhexanoic acid;
(2S)-5,5-dimethyl-2-{[(3-phenoxyphenyl)methyl]amino}hexanoic acid;
(2S)-2-{[(1S)-3-hydroxy-1-(3-methoxyphenyl)propyl]amino}-5,5-dimethylhexanoic acid;
(2S)-2-{[(1R)-3-hydroxy-1-phenylpropyl]amino}-5,5-dimethylhexanoic acid;
(2S)-5,5-dimethyl-2-({[3-(1H-1,2,4-triazol-1-yl)phenyl]methyl}amino)hexanoic acid;

or a pharmaceutically acceptable salt, solvate, hydrate, tautomer, optical isomer, N-oxide, and/or prodrug thereof.

**13.** A pharmaceutical composition comprising a compound according to any preceding claim and a pharmaceutically acceptable carrier, excipient, and/or diluent.

**14.** The compound according to any one of claims 1 to 12, or the pharmaceutical composition of claim 13, for use in therapy.

**15.** The compound according to any one of claims 1 to 12, or the pharmaceutical composition of claim 13, for use in the treatment or prevention of a neurodegenerative disorder, a psychiatric disorder, an inflammatory disorder, a cancer, pain, diabetes mellitus, diabetic retinopathy, glaucoma, uveitis, cardiovascular diseases, kidney disease, psoriasis, hereditary eye conditions, hearing loss or diseases **characterized by** misfolded tau;

wherein the neurodegenerative disorder is preferably selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy;
wherein the neurodegenerative disorder is preferably a neurodegenerative disorder **characterised by** misfolded TAR DNA-binding protein 43, such as amyotrophic lateral sclerosis, Alzheimer's disease, Frontotemporal Lobar Degeneration, or frontotemporal dementia;
wherein the psychiatric disorder is preferably selected from bipolar disorder, major depression, post-traumatic stress disorder, and anxiety disorders;
wherein the inflammatory disorder is preferably selected from inflammatory diseases and neuroinflammation;
wherein the cancer is preferably selected from breast cancer, lung cancer, ovarian cancer, prostate cancer, thyroid cancer, pancreatic cancer, glioblastoma and colorectal cancer;
wherein the cardiovascular disease is preferably selected from atherosclerosis, cardiomyopathy, heart attack, arrhythmias, heart failure, and ischemic heart disease; and
wherein the hearing loss is preferably selected from noise-induced hearing loss, ototoxicity induced hearing loss, age-induced hearing loss, idiopathic hearing loss, tinnitus and sudden hearing loss.

Europäisches Patentamt
European Patent Office
Office européen des brevets

# EUROPEAN SEARCH REPORT

Application Number
EP 22 19 3792

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | TENNA JUUL SCHRØDER ET AL: "The identification of AF38469: An orally bioavailable inhibitor of the VPS10P family sorting receptor Sortilin", BIOORGANIC & MEDICINAL CHEMISTRY LETTERS, vol. 24, no. 1, 27 November 2013 (2013-11-27), pages 177-180, XP0055379658, Amsterdam NL ISSN: 0960-894X, DOI: 10.1016/j.bmcl.2013.11.046 * the whole document * ----- | 1-15 | INV. A61P9/00 A61P9/04 A61P9/10 A61P25/00 A61P25/16 A61P25/18 A61P27/16 A61P35/00 C07C229/14 C07D213/36 C07D215/06 C07D215/227 C07D249/08 C07D263/58 C07D265/36 C07D307/78 C07D307/87 |
| X | DATABASE REGISTRY [Online] CHEMICAL ABSTRACTS SERVICE, COLUMBUS, OHIO, US; 23 March 2018 (2018-03-23), Aurora Fine Chemicals: "N-[(2,3-Dihydro-1H-inden-4-yl)methyl]-5,5-dimethylnorleucine", XP002808395, Database accession no. 2197279-42-6 * abstract * ----- | 1-8 | TECHNICAL FIELDS SEARCHED (IPC) |
| X | DATABASE REGISTRY [Online] CHEMICAL ABSTRACTS SERVICE, COLUMBUS, OHIO, US; 23 March 2018 (2018-03-23), Aurora Fine Chemicals: XP002808397, Database accession no. 2197262-75-0 * abstract * ----- -/-- | 1-8 | A61P C07C C07D |

The present search report has been drawn up for all claims

2

| Place of search | Date of completion of the search | Examiner |
|---|---|---|
| Munich | 17 January 2023 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 19 3792

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] CHEMICAL ABSTRACTS SERVICE, COLUMBUS, OHIO, US; 23 March 2018 (2018-03-23), Aurora Fine Chemicals: "5,5-Dimethyl-N-[(5,6,7,8-tetrahydro-2-naphthalenyl)methyl]norleucine", XP002808398, Database accession no. 2197214-66-5 * abstract * ----- | 1-8 | C07D311/76<br>C07D317/58<br>C07D319/18<br>C07D491/052<br>C07D491/056<br>A61K31/353<br>A61K31/47<br>A61K31/538<br>A61K31/357 |
| X | DATABASE REGISTRY [Online] CHEMICAL ABSTRACTS SERVICE, COLUMBUS, OHIO, US; Aurora Fine Chemicals: "N-[(2,3-Dihydro-1,4-benzodioxin-5-yl)methyl]-5,5-dimethylnorleucine", XP002808399, Database accession no. 2197213-62-8 * abstract * ----- | 1-8 | |
| | | | TECHNICAL FIELDS SEARCHED (IPC) |

The present search report has been drawn up for all claims

| Place of search | Date of completion of the search | Examiner |
|---|---|---|
| Munich | 17 January 2023 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160331746 A
- WO 2021116290 A1


### Non-patent literature cited in the description


- **SILVERMAN, R. B.** The Organic Chemistry of Drug Design and Drug Action. Elsevier Academic Press, 2004, 498-549
- **GREENE ; WUTS.** Protective Groups in Organic Synthesis. John Wiley and Sons, 2006
- **ANDERSEN, J et al.** Identification of the first small-molecule ligand of the neuronal receptor sortilin and structure determination of the receptor-ligand complex. *Acta Crystallogr D Biol Crystallogr,* 2014, vol. 70, 451-460
- **BAKER, M. et al.** Mutations in progranulin cause tau-negative frontotemporal dementia linked to chromosome 17. *Nature,* 2006, vol. 442 (7105), 916-919
- **BROUWERS, N. et al.** Genetic variability in progranulin contributes to risk for clinically diagnosed Alzheimer disease. *Neurology,* 2008, vol. 71 (9), 656-664
- **BUTTENSHON, H.N. et al.** Increased serum levels of sortilin are associated with depression and correlated with BDNF and VEGF. *Nature Translational Psychiatry,* 2015, vol. 5 (e677), 1-7
- **CARECCHIO, M.** Cerebrospinal fluid biomarkers in Progranulin mutations carriers. *J Alzheimers Dis,* 2011, vol. 27 (4), 781-790
- **CARRASQUILLO, M. et al.** Genome-wide screen identifies rs646776 near sortilin as a regulator of progranulin levels in human plasma. *Am J Hum Genet,* 2010, vol. 87 (6), 890-897
- **CHEN, Z. Y. et al.** Sortilin controls intracellular sorting of brain-derived neurotrophic factor to the regulated secretory pathway. *J Neurosci,* 2005, vol. 25 (26), 6156-6166
- **CRUTS, M. et al.** Loss of progranulin function in frontotemporal lobar degeneration. *Trends Genet,* 2008, vol. 24 (4), 186-194
- **DE MUYNCK, L. et al.** The neurotrophic properties of progranulin depend on the granulin E domain but do not require sortilin binding. *Neurobiol Aging,* 2013, vol. 34 (11), 2541-2547
- **EGASHIRA, Y. et al.** The growth factor progranulin attenuates neuronal injury induced by cerebral ischemia-reperfusion through the suppression of neutrophil recruitment. *J Neuroinflammation,* 2013, vol. 10, 105
- **GALIMBERTI, D. et al.** GRN variability contributes to sporadic frontotemporal lobar degeneration. *J Alzheimers Dis,* 2010, vol. 19 (1), 171-177
- **GALIMBERTI, D. et al.** Progranulin as a therapeutic target for dementia. *Expert Opin Ther Targets,* 2018, vol. 22 (7), 579-585
- **GAO, A. et al.** Implications of Sortilin in Lipid Metabolism and Lipid Disorder Diseases. *DNA and Cell Biology,* 2017, vol. 36 (12), 1050-1061
- **GASS, J. et al.** Progranulin regulates neuronal outgrowth independent of sortilin. *Mol Neurodegener,* 2012, vol. 7, 33
- **GASS, J. et al.** Progranulin: an emerging target for FTLD therapies. *Brain Res,* 2012, vol. 1462, 118-128
- **GIJSELINCK, I.** Granulin mutations associated with frontotemporal lobar degeneration and related disorders: an update. *Hum Mutat,* 2008, vol. 29 (12), 1373-1386
- **GOETTSCH, C. et al.** Sortilin and Its Multiple Roles in Cardiovascular and Metabolic Diseases. *Atherosclerosis, Thrombosis and Vascular Biology,* 2017, vol. 38 (1), 19-25
- **JANSEN, P. et al.** Roles for the pro-neurotrophin receptor sortilin in neuronal development, aging and brain injury. *Nature Neuroscience,* 2007, vol. 10 (11), 1449-1457
- **HU, F. et al.** Sortilin-mediated endocytosis determines levels of the frontotemporal dementia protein, progranulin. *Neuron,* 2010, vol. 68 (4), 654-667
- **HUANG, G. et al.** Insulin responsiveness of glucose transporter 4 in 3T3-L1 cells depends on the presence of sortilin. *Mol Biol Cell,* 2013, vol. 24 (19), 3115-3122
- **KADDAI, V. et al.** Involvement of TNF-$\alpha$ in abnormal adipocyte and muscle sortilin expression in obese mice and humans. *Diabetologia,* 2009, vol. 52, 932-940

- **KJOLBY, M. et al.** Sort1, encoded by the cardiovascular risk locus 1p13.3, is a regulator of hepatic lipoprotein export. *Cell Metab,* 2010, vol. 12 (3), 213-223
- **LAIRD, A. S. et al.** Progranulin is neurotrophic in vivo and protects against a mutant TDP-43 induced axonopathy. *PLoS One,* 2010, vol. 5 (10), e13368
- **LEE, W. et al.** Targeted manipulation of the sortilin-progranulin axis rescues progranulin haploinsufficiency. *Hum Mol Genet,* 2014, vol. 23 (6), 1467-1478
- **MARTENS, L. et al.** Progranulin deficiency promotes neuroinflammation and neuron loss following toxin-induced injury. *J Clin Invest,* 2012, vol. 122 (11), 3955-3959
- **MAZELLA, J. et al.** The 100-kDa neurotensin receptor is gp95/sortilin, a non-G-protein-coupled receptor. *J Biol Chem,* 1998, vol. 273 (41), 26273-26276
- **MIYAKAWA, S. et al.** Anti-sortilin1 Antibody Up-Regulates Progranulin via Sortilin1 Down-Regulation. *Front Neurosci,* 2020, vol. 14, 586107
- **MØLLER et al.** Sortilin as a Biomarker for Cardiovascular Disease Revisited. *Frontiers in Cardiovascular Medicine,* 2021, vol. 8, 652584
- **MORTENSEN, M.B. et al.** Targeting sortilin in immune cells reduces proinflammatory cytokines and atherosclerosis. *J Clin Invest,* 2014, vol. 124 (12), 5317-5322
- **NYKJAER, A et al.** Sortilin is essential for proNGF-induced neuronal cell death. *Nature,* 2014, vol. 427 (6977), 843-848
- **NYKJAER, A. ; WILLNOW, T. E.** Sortilin: a receptor to regulate neuronal viability and function. *Trends Neurosci,* 2012, vol. 35 (4), 261-270
- **OH, T.J. et al.** Circulating sortilin level as a potential biomarker for coronary atherosclerosis and diabetes mellitus. *Cardiovascular Diabetology,* 2017, vol. 16 (92
- **PAN, X. et al.** Sortilin and retromer mediate retrograde transport of Glut4 in 3T3-L1 adipocytes. *Mol Biol Cell,* 2017, vol. 28 (12), 1667-1675
- **PETERSEN, C. et al.** Molecular identification of a novel candidate sorting receptor purified from human brain by receptor-associated protein affinity chromatography. *J Biol Chem,* 1997, vol. 272 (6), 3599-3605
- **PICKFORD, F. et al.** Progranulin is a chemoattractant for microglia and stimulates their endocytic activity. *Am J Pathol,* 2011, vol. 178 (1), 284-295
- **POTTIER, C. et al.** Potential genetic modifiers of disease risk and age at onset in patients with frontotemporal lobar degeneration and GRN mutations: a genome-wide association study. *Lancet Neurol,* 2018, vol. 17 (6), 548-558
- **QUISTGAARD, E. et al.** Ligands bind to Sortilin in the tunnel of a ten-bladed beta-propeller domain. *Nat Struct Mol Biol,* 2009, vol. 16 (1), 96-98
- **SANTOS, A. M. et al.** Sortilin Participates in Light-dependent Photoreceptor Degeneration in Vivo. *PLoS ONE,* 2012, vol. 7 (4), 36243
- **KURUVILLA, R. et al.** A neurotrophin signaling cascade coordinates sympathetic neuron development through differential control of TrkA trafficking and retrograde signalling. *Cell,* 2004, vol. 118 (2), 243-255
- **SHI, J. ; KANDROR, K. V.** Sortilin Is Essential and Sufficient for the Formation of Glut4 Storage Vesicles in 3T3-L1 Adipocytes. *Developmental Cell,* 2005, vol. 9, 99-108
- **SCHRODER, T. et al.** The identification of AF38469: an orally bioavailable inhibitor of the VPS10P family sorting receptor Sortilin. *Bioorg Med Chem Lett,* 2014, vol. 24 (1), 177-180
- **SHENG, J. et al.** Progranulin polymorphism rs5848 is associated with increased risk of Alzheimer's disease. *Gene,* 2014, vol. 542 (2), 141-145
- **SKELDAL, S. et al.** Mapping of the Interaction Site between Sortilin and the p75 Neurotrophin Receptor Reveals a Regulatory Role for the Sortilin Intracellular Domain in p75 Neurotrophin Receptor Shedding and Apoptosis. *J Biol Chem,* 2012, vol. 21 (287), 43798-43809
- **TAURIS, J. et al.** Proneurotrophin-3 May Induce Sortilin-Dependent Death In Inner Ear Neurons. *Eur J Neuroscience,* 2020, vol. 33 (4), 622-31
- **TANG, W. et al.** The growth factor progranulin binds to TNF receptors and is therapeutic against inflammatory arthritis in mice. *Science,* 2011, vol. 332 (6028), 478-484
- **TAO, J. et al.** Neuroprotective effects of progranulin in ischemic mice. *Brain Res,* 2012, vol. 1436, 130-136
- **TENK, H.K. et al.** ProBDNF induces neuronal apoptosis via activation of a receptor complex of p75NTR and sortilin. *J Neuroscience,* 2005, vol. 10 (11), 1449-1457
- **VAN KAMPEN, J. M.** Progranulin gene delivery protects dopaminergic neurons in a mouse model of Parkinson's disease. *PLoS One,* 2014, vol. 9 (5), e97032
- **WILLNOW, T. E. et al.** VPS10P-domain receptors - regulators of neuronal viability and function. *Nat Rev Neurosci,* 2008, vol. 9 (12), 899-909
- **WILLNOW, T.E. et al.** Sortilins: new players in lipoprotein metabolism. *Current Opinion in Lipidology,* 2011, vol. 22 (2), 79-85
- **WUTS, P.G.M. ; GREENE, T.W.** Greene's Protective Groups in Organic Synthesis. John Wiley and Sons, 2006
- **XU, S.H. et al.** Regional and Cellular Mapping of Sortilin Immunoreactivity in Adult Human Brain. *Frotiers in Neuroanatomy,* 2019, vol. 13 (31), 1-27

- **YANO, H. et al.** Proneurotrophin-3 is a neuronal apoptotic ligand: evidence for retrograde-directed cell killing. *J Neurosci,* 2009, vol. 29 (47), 14790-14802
- **YIN, F. et al.** Exaggerated inflammation, impaired host defense, and neuropathology in progranulin-deficient mice. *J Exp Med,* 2010, vol. 207 (1), 117-128
- **ZHENG, Y. et al.** C-terminus of progranulin interacts with the beta-propeller region of sortilin to regulate progranulin trafficking. *PLoS One,* 2011, vol. 6 (6), e21023
- **ZHOU, X. et al.** Prosaposin facilitates sortilin-independent lysosomal trafficking of progranulin. *J Cell Biol,* 2015, vol. 210 (6), 991-1002
- **MENESES et al.** TDP-43 Pathology in Alzheimer's Disease. *Mol Neurodegeneration,* 2021, vol. 16, 84
- **PRUDENCIO et al.** Misregulation of human sortilin splicing leads to the generation of a nonfunctional progranulin receptor. *Proc Natl Acad Sci U S A,* 2012, vol. 109 (52), 21510-21515
- **BEEL et al.** Progranulin reduces insoluble TDP-43 levels, slows down axonal degeneration and prolongs survival in mutant TDP-43 mice. *Mol Neurodegener,* 2018, vol. 13, 55